(19) Europäisches Patentamt / European Patent Office / Office européen des brevets

(11) **EP 3 244 197 B1**

(12) **EUROPÄISCHE PATENTSCHRIFT**

(45) Veröffentlichungstag und Bekanntmachung des Hinweises auf die Patenterteilung:
**27.01.2021   Patentblatt 2021/04**

(51) Int Cl.:
*G01N 21/65* *(2006.01)*          *A01K 43/00* *(2006.01)*
*A01K 45/00* *(2006.01)*          *G01N 33/08* *(2006.01)*

(21) Anmeldenummer: **17170602.1**

(22) Anmeldetag: **11.05.2017**

(54) **VERFAHREN UND VORRICHTUNG ZUR EINSTELLUNG DES LASERFOKUS EINES ANREGUNGSLASERS IN BLUT DURCHFLOSSENEN GEFÄSSEN FÜR OPTISCHE MESSUNGEN ZUR BESTIMMUNG DES GESCHLECHTES VON VOGELEIERN**

METHOD AND DEVICE FOR ADJUSTING THE LASER FOCUS OF AN EXCITATION LASER FOR VESSELS THROUGH WHICH BLOOD FLOWS FOR OPTICAL MEASUREMENTS FOR DETERMINING THE SEX OF BIRD EGGS

PROCÉDÉ ET DISPOSITIF DE RÉGLAGE DE LA POSITION FOCALE D'UN LASER D'EXCITATION DANS DES VAISSEAUX SANGUINS POUR MESURES OPTIQUES DESTINÉES À DÉTERMINER LE SEXE D' OEUFS DE VOLAILLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.05.2016   DE 102016005974**

(43) Veröffentlichungstag der Anmeldung:
**15.11.2017   Patentblatt 2017/46**

(73) Patentinhaber:
• **Technische Universität Dresden**
  **01069 Dresden (DE)**
• **Hochschule für Technik und Wirtschaft Dresden**
  **01069 Dresden (DE)**

(72) Erfinder:
• **SCHART, Christoph**
  **01307 Dresden (DE)**
• **KOCH, Edmund**
  **01307 Dresden (DE)**

• **STEINER, Gerald**
  **08340 Schwarzenberg (DE)**
• **SCHNABEL, Christian**
  **01099 Dresden (DE)**
• **GALLI, Roberta**
  **01309 Dresden (DE)**
• **PREUSSE, Grit**
  **01445 Radebeul (DE)**
• **DIMTER, Tom**
  **01189 Dresden (DE)**

(74) Vertreter: **Rauschenbach, Marion**
  **Rauschenbach Patentanwälte**
  **Bienertstrasse 15**
  **01187 Dresden (DE)**

(56) Entgegenhaltungen:
WO-A1-2016/000678          WO-A1-2016/005539
DE-A1-102007 013 107       DE-B3-102010 006 161
US-B1- 7 950 349

**Beschreibung**

Die Erfindung betrifft ein Verfahren und eine Vorrichtung zur Einstellung des Laserfokus eines Anregungslasers in Blut durchflossenen Gefäßen für optische Messungen zur Bestimmung des Geschlechtes von Vogeleiern.

Eine Bewegungserkennung (engl. motion detection) durch Nutzung einer temporären Information aus einer Bildersequenz wird z.B. in der Druckschrift Martinez-Martin, del Pobil: Robust Motion Detection in Real-Life Scenarios, 2012 Springer-Verlag London, DOI10.1007/978-1-4471-4216-4 beschrieben. Für ein Bild, welches ein sich im Vordergrund bewegendes Objekt auf einem statischen Hintergrund enthält, kann jeder sich ändernde Pixel als Teil des Vordergrundes betrachtet werden. Aus diesem Grund können Verfahren angewendet werden, die auf der temporären Information aus einer schwellwertdefinierten Bilddifferenz basieren. Dabei wird festgelegt, dass ein Pixel x Teil eines sich bewegenden Objektes ist, wenn seine Intensität sich signifikant zwischen zwei aufeinanderfolgenden Bildern ändert:

$$|I_t(x) - I_{t-1}(x)| > \tau \qquad\qquad (I)$$

mit

$I_t(x)$     als Intensitätswert an der Pixelposition x zur Zeit t und
$\tau$     als Schwellwert.

In der Druckschrift US 2014/0273068 ist zur hämatologischen Untersuchung ein Autofokussystem für Blutzellen in einer Durchflusszelle beschrieben. Der Autofokus basiert auf einer Kontrastmaximierung zu einem Referenztarget, welches in unveränderlicher Distanz zur Durchflusszelle befestigt ist.

Zwei Autofokussysteme für Raman-Messungen in Blutgefäßen sind in den Druckschriften US 7 583 380 B2 und US 7 663 748 B2 beschrieben. In der Druckschrift US 7 583 380 B2 ist eine spektroskopische Analyseapparatur zur Blutanalyse von Patienten beschrieben, bei der die Autofokussierung mit einem bildgebenden System realisiert wird, welches aus zwei Kameras mit leicht unterschiedlichen Bildebenen besteht. Die konfokale Raman-Detektionsebene liegt zwischen diesen beiden Bildebenen, sodass sich bei gleicher Defokussierung der zwei Kamerabildebenen der Fokus der Laseranregung im Zentrum des Blutgefäßes befindet. Durch Erfassung der Defokussierung der Bildebenen während der Bildgebung des Blutgefäßes kann eine kontinuierliche Autofokussierung des Ramanlasers im Blutgefäß erreicht werden.

In der Druckschrift US 7 663 748 B2 wird ein Autofokusmechanismus für spektroskopische Systeme beschrieben, der auf Fluktuationsmessungen optischer Eigenschaften des Probevolumens basiert. Im Fall der Raman-Spektroskopie von fließendem Blut wird die elastisch gestreute Strahlung des Anregungsstrahles für Fluktuationen gemessen, die durch den Fluss der roten Blutzellen erzeugt werden. Eine Kontrollschleife maximiert die Amplitude und/oder die Intensität der Fluktuationen und fokussiert damit in das Zentrum des Blutgefäßes. Das System ist vorgesehen für konfokale Ramansysteme mit einem Laserfokusvolumen kleiner dem der roten Blutzellen, sodass die Fluktuationen auf der Streuung der Einzelzelle beruhen. In nicht-konfokalen Systemen mit Laserspots größer als die einzelne rote Blutzelle, werden die Fluktuationen mit wachsendem Probenvolumen gemittelt und sind nicht mehr detektierbar, wenn das Laserfokusvolumen mehrere Blutzellen enthält.

In der Druckschrift US 6 681 133 B2 wird ein Verfahren zur Ausbildung optischer Systeme für spektroskopische in-vivo Untersuchungen der Haut beschrieben. Dabei wird zwischen Ramansignalen der Hautoberfläche und Ramansignalen von tiefer gelegenem Hautgewebe unterschieden, um den Fokus des optischen Systems in der gewünschten Hautschicht zu positionieren. Der größte Nachteil dieses Verfahrens besteht in der Dauer der Ramanmessung, die für in-ovo Messungen kein Echtzeit-Feedback ermöglicht.

Weitere Raman-Spektroskopie-Systeme mit integriertem Autofokus sind in den Druckschriften US 8 159 662 B2 und US 8 306 780 B2 veröffentlicht. Beide Anwendungen sind für Fernerkundungen konzipiert, die Brennweite beträgt bis zu mehreren Metern und der Autofokus basiert auf Entfernungsmessung.

Eine in-ovo Blutflussdetektion mittels Velozimetrie (engl. Particle Image Velocimetry (PIV)) unter einem Mikroskop (micro-PIV), bei der mit einer Hochgeschwindigkeits-CMOS Kamera Bilder der fließenden roten Blutzellen aufgenommen werden, um den Blutfluss in extraembryonalen Blutgefäßen von Hühnerembryonen beurteilen zu können, wird in den Druckschiften Lee, Ji, Lee: Micro-PIV measurements of blood flow in extraembryonic blood vessels of chicken embryos, Physiol. Meas. 28 (2007) 1149-1162 und Kloosterman, Hierck, Westerweel, Poelma: Quantification of Blood Flow and Topology in Developing Vascular Networks, PlosONE (2014) 9(5): e96856 publiziert. Mit diesem Verfahren kann keine volumetrische Information erhalten werden, um eine Autofokussierung im Blutgefäß zu realisieren.

Es werden im folgend genannten Stand der Technik optische Verfahren im biomedizinischen Bereich angegeben, die entweder genutzt werden für

- eine Blutflussdetektion mittels Differenzbildanalyse, um durchblutete Gefäße und Fließeigenschaften zu erhalten, z.B. in der Retina, und
- eine 3D-Lokalisation von Blutgefäßen zur Blutentnahme.

Laser-Holographie-Verfahren werden für die Partikelfluss-Analyse genutzt. Obwohl ein holographisches Video die gesamte 4D-Information liefern kann, wie in der Druckschrift Meinecke, Sabitov, Sinzinger: Information extraction from digital holograms for particle flow analysis, Applied Optics 49(13), 2010 beschrieben ist, ist dieses Verfahren niemals genutzt worden, um ein Autofokussystem zu steuern, was hauptsächlich auf die Komplexizität der erforderlichen Hardware und die hohen Kosten zurückzuführen ist.

Alle oben beschriebenen Verfahren nutzen die temporären Differenzen zwischen Bildsequenzen, wobei die Beleuchtung mittels Laser erfolgt. Sie basieren nicht auf einer einfachen Kamerabildgebung mit Lampen- oder LED-Beleuchtung. Die Anwendung dieser Verfahren ist weder für ein Blutgefäße-Sampling noch für eine Autofokussierung in Verbindung mit spektroskopischen Verfahren jemals beschrieben worden.

Es ist erwähnenswert, dass ebenso Hochfrequenz-Ultraschall (>20 MHz) die Bildgebung von mikrovaskulärem Blutfluss ermöglicht, wie in der Druckschrift Goertz, Yu, Kerbel, Burns, Foster: High-frequency 3-D color-flow imaging of the microcirculation, Ultrasound Med Biol. 2003 Jan; 29 (1): p. 39 - 51 beschrieben ist. 3D-Flow-Bilder von Gewebevolumen mit einer Dimension von bis zu 10 mm lateral und 5 mm Tiefe werden für eine Vielzahl wissenschaftlicher und klinischer Fragestellungen genutzt. Die Geschwindigkeiten des Blutflusses, die von diesen Systemen gemessen werden können, liegen im Bereich von <1 mm/s bis 25 mm/s für Blutgefäße mit Durchmessern zwischen 30 $\mu$m -100 $\mu$m. Die Anwendung der dynamischen Speckle-Bildgebung (engl. Speckle Flow Imaging (SFI)) auf die Ultraschallbilder ermöglicht die Darstellung eines langsamen Blutflusses in der Mikrozirkulation mit hoher räumlicher Auflösung, wie in der Druckschrift Aoudi, Liebgott, Needles, Yang, Foster, Vray: Estimation methods for flow imaging with high frequency ultrasound, Ultrasonics 44 (2006), S. 135-140 beschrieben ist. Ultraschall-Bildgebung in all seinen Ausführungen ist jedoch für die in-ovo Analyse nicht anwendbar, da ein direkter Kontakt zwischen der Oberfläche und dem Transducer/Schallkopf erforderlich ist, und somit nicht vergleichbar mit der simultanen kontaktlosen Ramanspektroskopie ist.

In den Druckschriften Nakamachi: Development of Three Dimensional Blood Vessel Search System by Using on Stereo and Autofocus Hybrid Method, 33rd Annual International Conference of the IEEE EMBS, 2011 und Nakamachi, Morita, Mizuno: Development of Automatic 3D Blood Vessel Search and Automatic Blood Sampling System by Using Hybrid Stereo-Autofocus Method, International Journal of Optics Vol. 2012, ist jeweils ein 3D-System zum Auffinden von Blutgefäßen, welches in ein automatisches Blutprobensystem implementiert wird, beschrieben. Das 3D-System nutzt Nahinfrarot-Bildgebung und das Stereo- und Autofokus-Hybridverfahren, um die Lage der Blutgefäße in drei Dimensionen korrekt zu bestimmen. Der Autofokus basiert auf der Schärfe der Bilder, die in Transmission registriert werden. Die Genauigkeit der Positionsbestimmung der Blutgefäße beträgt 100 $\mu$m für Blutgefäße mit Durchmessern von 500 $\mu$m -1000 $\mu$m.

Diese Applikation ist nicht in ovo anwendbar: Die Messung erfolgt in Transmission, sodass die Blutgefäßlokalisierung auf der Basis der Bilder erfolgt, die durch die Hämoglobinabsorption im NIR-Bereich generiert werden.

In der Druckschrift WO 2016/000678 A1 sind eine Vorrichtung und ein Verfahren zur Ramanspektroskopischen in-ovo Geschlechtsbestimmung von befruchteten und bebrüteten Vogeleiern beschrieben.

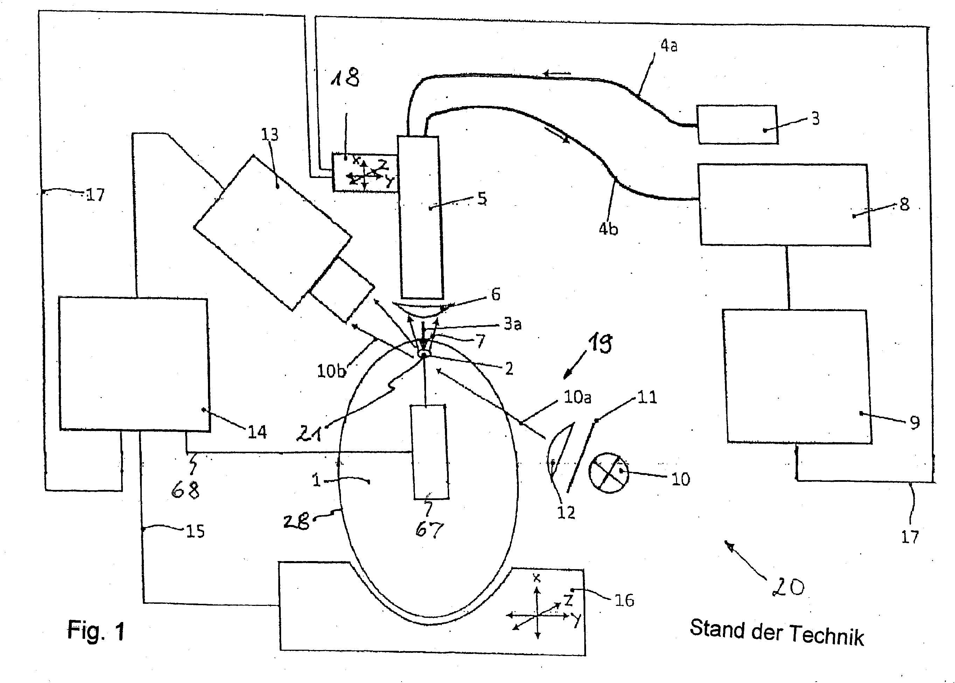

Die in einer Fig. 1 gezeigte schematische Darstellung der Vorrichtung 20 zur Bestimmung des Geschlechtes von befruchteten und bebrüteten Vogeleiern 1 umfasst zumindest

- eine Ei-Lagerungseinheit 16,
- eine Blutgefäß-Positions-Auswerteeinrichtung 14, die mit der Ei-Lagerungseinheit 16 in Verbindung steht,
- eine Strahlungseinrichtung 19 mit Licht 10a einer Lichtquelle 10 zur Erkennung mindestens eines Blutgefäßes 21, wobei die Strahlungseinrichtung 19 zumindest einen Teil des Eies 1 durchstrahlt,
- einen Detektor 13 für das Licht 10b zur Erkennung von Blutgefäßen 21, wobei der Detektor 13 mit der Blutgefäß-Positionier-Auswerteeinrichtung 14 in Verbindung steht, wobei die Strahlungseinrichtung 19 und der Detektor 13 zumindest ein Visions-System 19, 13 bilden,
- eine Locherzeugungseinheit 67 zur Schaffung eines Lochs 2 in der Kalkschale 28 im Nahbereich des erkannten Blutgefäßes 21,
- eine Vorrichtung 5 zur Einbringung von Laserlicht 3a in das geschaffene Loch 2 des Ei 1 und zur Erfassung der Ramanstreustrahlung 7, die zumindest in Verbindung steht mit

- einer das Laserlicht 3a emittierenden Laserquelle 3, wobei das Laserlicht 3a auf mindestens ein Blutgefäß 21 fokussiert gerichtet ist,
- einem Spektrometer 8 zur Aufnahme der Ramanstreustrahlung 7 des Blutes des vom Laserlicht 3a bestrahlten Blutgefäßes 21 über mindestens eine Leitung 4b und
- einer Steuereinheit 18 zur xyz-Positionierung der Vorrichtung 5 auf ein in das Ei 1 eingebrachtes Loch 2,

- eine Geschlechtsbestimmungs-Auswerteeinheit 9, die mit dem Spektrometer 8 und der Blutgefäß-Positionier-Auswerteeinrichtung 14 in Verbindung steht und aus der verarbeiteten erfassten Ramanstreustrahlung 7 des Spektrometers 8 das Geschlecht des bebrüteten Vogeleies 1 angibt.

Die Locherzeugungseinheit 67 in Form eines Lasers oder einer Einrichtung zur mechanischen Perforation steht mit der Blutgefäß-Positions-Auswerteeinrichtung 14 über eine Leitung 68 in Verbindung.

Das Visions-System 19, 13 weist eine Einrichtung zur Feststellung der Lage der Blutgefäße 21 mit einem Detektor 13 auf.

Das der Fig. 1 zugehörige Verfahren zur Ramanspektroskopischen in-ovo Geschlechtsbestimmung von befruchteten und bebrüteten Vogeleiern, wobei der Embryo einschließlich der extraembryonalen Strukturen beweglich im Ei 1 ist und noch nicht zum Zeitpunkt einer Messung der Ramanstreustrahlung 7 an der Kalkschale 28 fixiert ist, weist folgende Schritte auf:

- Überwachung des Zeitverlaufs des Bebrütens bis zur Ausbildung mindestens eines erkennbaren Blutgefäßes 21,
- Schaffung eines Lochs 2 in der Kalkschale 28 im Nahbereich des erkannten Blutgefäßes 21 mittels einer Locherzeugungseinheit 67,
- Suchen der sich im Ei 1 ausbildenden Blutgefäße 21 mittels eines Visions-Systems 19, 13 und einer koaxialen oder lateralen Beleuchtung mit Licht 10a des sichtbaren Wellenlängenbereiches,
- Positionieren zumindest eines Blutgefäßes 21 in den Laserfokus 30 einer Laserstrahlenquelle 3 entweder durch Bewegen des Eies 1 oder Bewegen eines Objektivs 6 einer Vorrichtung 5 zur Einbringung der Laserstrahlung 3a und Erfassung der Ramanstreustrahlung 7,
- Registrieren der Ramanstreustrahlung 7 des bestrahlten Blutgefäßes 21, 27 mittels der Vorrichtung 5 zur Einbringung der Laserstrahlung 3a und zur Erfassung der Ramanstreustrahlung 7, wobei während der Messung eine Bewegung des Blutgefäßes 21 aus dem Fokus 30 heraus durch Nachführung mittels des Visions-Systems 19, 13 vermieden werden kann,
- Auswertung der Ramanstreustrahlung 7 und Bestimmung des Geschlechtes des Embryos in einer Auswerteeinheit 9,
- Anzeige des Geschlechtes des Embryos im Vogelei 1.

Zum Visions-System 19, 13 gehören noch ein grüner Filter 11 und eine Linse 12. Des Weiteren sind Steuerleitungen 15 und 17 vorhanden, die zu der Blutgefäß-Positionier-Auswerteeinrichtung 14 führen.

Zur Überwachung des Zeitverlaufs der Vorgänge beim Bebrüten des Vogeleies 1 wird als Licht 10a des sichtbaren Wellenlängenbereiches weißes oder blaues und/oder grünes Licht aus der Lichtquelle 10 der Strahlungseinrichtung 19 des Visions-Systems 19, 13 eingesetzt. Insbesondere wird Licht des grünen Wellenlängenbereiches eingesetzt.

Im Allgemeinen wird das beschriebene Verfahren zur Ramanspektroskopischen in-ovo Geschlechtsbestimmung von befruchteten und bebrüteten Vogeleiern 1 ab dem dritten Bruttag durchgeführt.

Bei der bisherigen Geschlechtsbestimmung wird gemäß der Fig. 1 der Laserfokus 30 auf die Blutgefäße 21 ausgerichtet. Anschließend wird ein Blutgefäß 21 mit möglichst hohem Durchmesser manuell selektiert und in der horizontalen Ebene zentriert. Dadurch wird sichergestellt, dass der Raman-Anregungslaser 3 sich im Arbeitsbereich befindet. Nach dem Öffnen der Kalkschale 28 des Eies 1 kommt es aufgrund der charakteristischen Eigenschaften des Hühnereies 1 zu einem Absinken des Eiweißes und des Dottersacks und somit der Blutgefäße 21 im embryonalen Bereich, wodurch sich das Blutgefäß 21 aus dem Laserfokus 30 des Anregungslasers 3 heraus verschiebt. Zur Ramanspektroskopischen in-ovo Geschlechtsbestimmung von befruchteten und bebrüteten Vogeleiern 1 ist es erforderlich, dass der Laserfokus 30 des Anregungslasers 3 im embryonalen Blutgefäß 21 mit einem Durchmesser von 20 µm bis 200 µm positioniert wird. Aufgrund der Bewegungen des Blutgefäßes 21 in x-, y-, z-Richtung kann die Tatsache auftreten, dass das selektierte Blutgefäß 21 während der Dauer der spektroskopischen Messungen jedoch diese Sollposition des Laserfokus 30 verlässt. Damit wird das reflektierte, zu messende Ramansignal 7 des Blutes abgeschwächt, was zu Fehlern bei der Geschlechtsbestimmung führen kann. Das vorherige Absinken entsteht durch das Herausdiffundieren der Luft aus der Luftkammer des Hühnereies. Deshalb ist es bisher immer notwendig, den Laserfokus 30 des Anregungslasers 3 manuell kontinuierlich dem absinkenden Blutgefäß 21 nachzuführen, was relativ zeitaufwendig ist.

Außerdem wird bei der Bestimmung der Position eines Blutgefäßes 21 im Ei 1 mittels Bildgebung der Kontrast durch störende Faktoren wie beispielsweise Schalenstaub, spiegelnde Reflexion der Eiweißschichtoberfläche oder der Fließbewegung der Erythrozyten 36 beeinträchtigt.

Bekannt aus der DE 10 2010 006 161 B3 ist ein Verfahren und eine Vorrichtung zur Bestimmung des Geschlechtes von befruchteten und nicht bebrüteten Vogeleiern, wobei ein Ei eine feste Eischale, ein von der Eischale und weiteren Eihüllen umgebenes Eidotter und eine dem Eidotter zugeordnete Keimscheibe aufweist und wobei eine Sonde zur Messung eines Spektrums durch ein Loch in der Eischale hindurch in Richtung zur Keimscheibe mit Keimscheibenzellen geführt wird. Dabei werden die Schritte der Positionierung der Sonde im Bereich der Keimscheibe, spektroskopische in-ovo Charakterisierung der Keimscheibenzellen, Erkennung des Geschlechts durch eine automatische Klassifizierung der Spektren, durchgeführt.

Und auch bekannt aus der DE 10 2007 013 107 A1 ist ein Verfahren zur Bestimmung des Geschlechts von Vögeln, bei dem DNA-relevantes Zellmaterial des geschlechtlich zu bestimmenden Vogels mit Licht untersucht und die Molekülschwingungen gemessen werden, dass das durch das Licht entstehende Spektrum der Molekülschwingungen erfasst und mit vorgegebenen sowie geschlechtsspezifische DNA-Strukturen der zu untersuchenden Vogelart repräsentierenden Referenzspektren verglichen wird und dass aus diesem Spektralvergleich eine auf Grundlage des DNA-Gehalts des Zellmaterials basierende Geschlechtszuordnung des Vogels getroffen wird.

Der Erfindung liegt deshalb die Aufgabe zugrunde, ein Verfahren und eine Vorrichtung zur Einstellung des Laserfokus eines Anregungslasers in Blut durchflossenen Gefäßen für optische Messungen zur Bestimmung des Geschlechtes von Vogeleiern anzugeben, die derart geeignet ausgebildet sind, dass erstens der Fokus des Laserstrahls des Anregungslasers für die zu messende Rückstrahlung ständig und mit hoher Genauigkeit in das seine Lage verändernde Blut durchflossene Gefäß positioniert und zweitens der Fokus während der optischen Messung dem sich die Lage verändernden Blut durchflossenen Gefäß nachgeführt wird.

Die Aufgabe wird durch die Merkmale der Patentansprüche 1 und 13 gelöst. Das Verfahren zur Einstellung des Laserfokus eines Anregungslasers in einem Blut durchflossenen Gefäß für optische Messungen zur Bestimmung des Geschlechts von Vogeleiern, wobei die Blut durchflossenen Gefäße einschließlich des Embryos beweglich im Ei sind und noch nicht zum Zeitpunkt einer optischen Messung der Rückstreustrahlung an der Kalkschale fixiert ist, weist im Kennzeichenteil des Patentanspruchs 1

folgende Schritte auf:

- Selektion eines Blut durchflossenen Gefäßes in dem lochgeöffneten und bebrüteten Ei mittels eines kontrastbasierten Auswerteverfahrens unter zumindest einer Beleuchtung eines Blut durchflossenen Gefäßgeflechts mit Strahlung zumindest im grünen Wellenlängenbereich mittels einer optischen Vorrichtung zur Einbringung von Laserstrahlung, zur Erfassung von Rückstreustrahlung und Analysepositionsbildflächen $SAF_1$, $SAF_2$,
- Herstellen des Laserfokus für den Anregungslaser und eines Fokus für eine Kamera in dem selektierten Blut durchflossenen Gefäß durch das dem Gefäß zugewandten Loch hindurch mittels der optischen Vorrichtung zur Einbringung von Laserstrahlung, zur Erfassung von Rückstreustrahlung und Analysepositionsbildflächen $SAF_1$, $SAF_2$,
- Einstellung des Laserfokus innerhalb des selektierten Blut durchflossenen Gefäßes mittels der optischen Vorrichtung zur Einbringung von Laserstrahlung, zur Erfassung von Rückstreustrahlung und Analysepositionsbildflächen $SAF_1$, $SAF_2$ mittels eines erythrozytbewegungsbasierten Differenzbild-Verfahrens aufgrund der in jeweils zwei nacheinander aufgenommenen Bildern mit infolge der Bewegung der Erythrozyten innerhalb des selektierten Blut durchflossenen Gefäßes auftretenden Intensitätsänderungen $I_{t-1}(x)$, $I_t(x)$ als Intensitätswerte an der Pixelposition $x$ zur Zeit $t-1$ und $t$ und Nachführen des Laserfokus und des Fokus der Kamera bei Absenken der Fläche des Blutgefäßgeflechtes und der Eiweißschicht,
- Ausgleich von auftretenden x-,y-Horizontalbewegungen des selektierten Blut durchflossenen Gefäßes mittels der optischen Vorrichtung zur Einbringung von Laserstrahlung, zur Erfassung von Rückstreustrahlung und Analysepositionsbildflächen $SAF_1$, $SAF_2$,
- Durchführung der optischen Messungen mittels der optischen Vorrichtung zur Einbringung von Laserstrahlung, zur Erfassung von Rückstreustrahlung und Analysepositionsbildflächen $SAF_1$, $SAF_2$.

Für die Durchführung des Verfahrens kann als die zu messende, aus dem Blut durchflossenen Gefäß rückgestrahlte Rückstreustrahlung eine Ramanstreustrahlung und/oder eine rückgestreute Fluoreszenzstrahlung für die optischen Messungen ausgewählt werden.

In dem Verfahren werden folgende Schritte realisiert:

- Einbringung eines Lochs in der Kalkschale mittels einer Locherzeugungseinheit und Positionierung der Blutgefäße im Bereich des Loches,
- Suchen der sich im Ei ausbildenden Blutgefäße mittels eines zumindest aus einer Strahlungseinrichtung und aus einem Detektor bestehenden Visions-Systems und einer koaxialen oder lateralen Beleuchtung mit Licht des sichtbaren Wellenlängenbereiches,
- Positionieren zumindest des Blut durchflossenen Gefäßes in den Laserfokus des Anregungslasers entweder durch Bewegen des Eies mittels einer gesteuerten Positioniereinheit oder durch ein gesamtheitliches Bewegen einer optischen Vorrichtung zur Einbringung von Laserstrahlung, zur Erfassung von Rückstreustrahlung und Analysepositionsbildflächen $SAF_1$, $SAF_2$ oder durch einen Wechsel mindestens eines Objektivs oder durch ein Objektiv mit variabler Vergrößerung in der Vorrichtung zur Einbringung von Laserstrahlung, zur Erfassung von Rückstreustrahlung und Analysepositionsbildflächen $SAF_1$, $SAF_2$,
- Registrieren der Rückstreustrahlung des bestrahlten Blut durchflossenen Gefäßes mittels der Vorrichtung zur Einbringung von Laserstrahlung, zur Erfassung von Rückstreustrahlung und Analysepositionsbildflächen $SAF_1$, $SAF_2$, wobei während der Registrierung der Rückstreustrahlung eine Bewegung des Blutgefäßes aus dem Laserfokus und dem Fokus der Kamera heraus durch Nachführung des Eies mittels einer gesteuerten Positioniereinheit und/oder durch ein gesamtheitliches Bewegen der Vorrichtung zur Einbringung von Laserstrahlung, zur Erfassung von Rückstreustrahlung und Analysepositionsbildflächen $SAF_1$, $SAF_2$ oder nach einem Wechsel mindestens eines Objektivs oder nach einem Wechsel der Vergrößerung jeweils eines der Objektive in der optischen Vorrichtung zur Einbringung von Laserstrahlung, zur Erfassung von Rückstreustrahlung und Analysepositionsbildflächen $SAF_1$, $SAF_2$ vermieden werden kann,

- Auswertung der Rückstreustrahlung und Bestimmung des Geschlechtes des Embryos in einer Auswerteeinheit,
- Anzeige des Geschlechtes des Embryos im Vogelei,

wobei in der Auswerteeinheit mittels programmtechnischen Mitteln in einem ersten Algorithmus ein kontrastbasiertes Verfahren zur Festlegung einer gesamten Analysepositionsbildfläche $SAF_1$ mit dem Blut durchflossenen Blutgefäßgeflecht durchgeführt wird, und

in der Auswerteeinheit mittels programmtechnischer Mittel in einem zweiten Algorithmus ein erythrozytbewegungsbasiertes Differenzbild-Verfahren zur Einstellung des Laserfokus mittels der optischen Vorrichtung zur Einbringung von Laserstrahlung, zur Erfassung von Rückstreustrahlung und Analysepositionsbildflächen $SAF_1$, $SAF_2$ auf eine reduzierte Bildfläche RBF in der selektierten Analysepositionsbildfläche $SAF_2$ mit dem selektierten Blut durchflossenen Gefäß durchgeführt wird, wobei beim erythrozytbewegungsbasierten Differenzbild-Verfahren die Intensitätsinhalte $I_{t-1}(x)$, $I_t(x)$ von mindestens zwei aufeinanderfolgenden Bildern der auf das selektierte Blut durchflossene Gefäß reduzierten Bildfläche RBF - ROI - innerhalb der selektierten Analysepositionsbildfläche $SAF_2$ verglichen werden, wobei die Intensitätsinhalte $I_{t-1}(x)$, $I_t(x)$ von den sich jeweils in der Bildfläche RBF widerspiegelnden Bewegungen der Erythrozyten unterschiedlich ausgebildet werden, und wobei der Laserfokus des Anregungslasers und der zum Laserfokus konform gebundene Fokus der Kamera mittels der optischen Vorrichtung zur Einbringung von Laserstrahlung, zur Erfassung von Rückstreustrahlung und Analysepositionsbildflächen $SAF_1$, $SAF_2$ auf eine dem selektierten Blut durchflossenen Gefäß zugeordnete reduzierte Bildfläche RBF der Analysepositionsbildfläche $SAF_2$ eingestellt wird, wobei sich der Laserfokus und der Fokus im finalen Schritt immer innerhalb des selektierten Blut durchflossenen Gefäßes befinden.

Als Strahlung kann eine Strahlung des sichtbaren Wellenlängenbereiches oder des grünen Wellenlängenbereiches aus der Strahlungsquelle der Strahlungseinrichtung des Visions-Systems zur Überwachung des Zeitverlaufs der Vorgänge beim Bebrüten des Vogeleies eingesetzt werden.

Das optische Messverfahren zur in-ovo Geschlechtsbestimmung von befruchteten und bebrüteten Vogeleiern kann ab dem dritten Bruttag durchgeführt werden.

Zur Durchführung des Verfahrens können folgende Parameter für die optischen Messungen der vom Blut durchflossenen Gefäß rückgestreuten Ramanstreustrahlung eingesetzt werden:

- Anregungswellenlängen der Laserstrahlung des Anregungslasers (3): > 600 nm (z.B. HeNe Laser 633 nm, Festkörperlaser (Nd basiert z.B. Nd:YAG Laser 1064 nm, VIS NIR Diodenlaser, z.B. 785 nm),
- Einkopplung der Laserstrahlung direkt mit Spiegeln und/oder mit optischen Fasern,
- Ramanstreustrahlungsmessungen werden unter Nutzung von optischen Vorrichtungen mit großer numerischer Apertur wie Mikroskop-Objektiven oder einer Fasersonde durchgeführt,
- Direkte Auskopplung der gesammelten Ramanstreustrahlung mittels Spiegeln zu einem Spektrometer oder mittels Transports über optische Fasern,
- Einsatz eines Spektrometers in Form von dispersivem Spektrometer und Fourier Transform Spektrometer.

Des Weiteren können folgende Parameter für die optischen Messungen der vom Blut durchflossenen Gefäß rückgestreuten Fluoreszenzstrahlung eingesetzt werden:

- Anregungswellenlängen der Laserstrahlung (3a) des Anregungslasers (3): > 400 nm (z.B. HeNe Laser 633 nm, Festkörperlaser (Nd basiert z.B. Nd:YAG Laser 532 nm, VIS NIR Diodenlaser, z.B. 785 nm),
- Einkopplung der Laserstrahlung direkt mit Spiegeln und/oder mit optischen Fasern,
- Fluoreszenzstrahlungsmessungen werden unter Nutzung von optischen Vorrichtungen wie Mikroskop-Objektiven oder einer Fasersonde durchgeführt,
- Direkte Auskopplung der gesammelten Fluoreszenzstrahlung mittels mindestens eines Spiegels oder mittels Transports über optische Fasern,
- Einsatz eines Detektors.

Während des Positionierungsvorgangs des Vogeleis können permanent Messwerte von optischen Messungen aufgezeichnet und einer Auswertung zugeführt werden, wobei eine automatische Klassifizierung der Rückstreustrahlung anhand des spektralen Fingerabdruckes des Blutes erfolgt.

Zur Durchführung der optischen Messungen kann das Vogelei pollastig gestellt werden.

Dazu kann ein Loch im Bereich des nach oben gerichteten spitzen Pols bei einer Lochgröße bis zu 18 mm nach 48 Stunden mittels der Locherzeugungseinheit ausgebildet werden.

Auch kann ein Loch am nach oben gerichteten, stumpfen Pol des Eies einen Durchmesser bis zu 18 mm, vorzugsweise zwischen 8 mm und 12 mm mittels der Locherzeugungseinheit ausgebildet werden.

Das optische Messverfahren kann als Ramanspektroskopisches in-ovo MessVerfahren zur Geschlechtsbestimmung von befruchteten und bebrüteten Vogeleiern ab dem dritten Bruttag durchgeführt werden.

Die Vorrichtung zur Einstellung des Laserfokus eines Anregungslasers in einem Blut durchflossenen Gefäß dient für optische Messungen zur Bestimmung des Geschlechts von Vogeleiern, wobei die Blut durchflossenen Gefäße einschließlich des Embryos beweglich im Ei sind und noch nicht zum Zeitpunkt einer optischen Messung der Rückstreustrahlung an der Kalkschale fixiert ist, unter Nutzung des vorgenannten Verfahrens,

wobei gemäß dem Kennzeichenteil des Patentanspruchs 13

die Vorrichtung zumindest umfasst

- eine Halterungseinrichtung für das mit einem Loch versehene Ei, wobei die Eier jeweils mit einem Pol nach oben gelagert sind,
- eine Beleuchtungseinrichtung für die Beleuchtung der im Ei befindlichen Fläche mit Eiweißschicht und mit Blutgefäßgeflecht,
- eine koordinative x-,y-,z-Positioniereinheit für eine optische Vorrichtung zur Einbringung von Laserstrahlung, zur Erfassung von Rückstreustrahlung und Analysepositionsbildflächen $SAF_1$, $SAF_2$,
- die optische Vorrichtung zur Einbringung von Laserstrahlung, zur Erfassung von Rückstreustrahlung und Analysepositionsbildflächen $SAF_1$, $SAF_2$, zumindest enthaltend

  - mindestens ein Mess-Objektiv,
  - einen Strahlteiler,
  - eine Kamera mit mindestens einem Kamera-Objektiv,

  wobei die optische Vorrichtung zur Einbringung von Laserstrahlung, zur Erfassung von Rückstreustrahlung und Analysepositionsbildflächen $SAF_1$, $SAF_2$ zur Abbildungsgrößenänderung zwischen einem Bild der $SAF_1$ und einem Bild der RBF in der selektierten Analysepositionsfläche $SAF_2$ dient, wobei das Bild der Analysepositionsbildfläche $SAF_1$ die gesamte Bildfläche des Blutgefäßgeflechtes und das Bild in der Analysepositionsbildfläche $SAF_2$ die reduzierte Bildfläche des selektierten Blut durchflossenen Gefäßes betreffen,
- eine Auswerteeinrichtung für optische Spektren,
- eine Auswerteeinheit, die zumindest umfasst

  - eine Verbindungsleitung zur Kamera,
  - eine Verbindungsleitung zur Halterungseinrichtung,
  - eine Verbindungsleitung zur koordinativen x-,y-,z-Positioniereinheit,
  - eine Verbindungsleitung zur Auswerteeinrichtung für optische Messungen,

  wobei die Auswerteeinheit programmtechnische Mittel zur Durchführung eines kontrastbasierten Verfahrens, das sich auf eine gesamte Bildfläche der Analysepositionsbildfläche $SAF_1$ mit dem Blutgefäßgeflecht bezieht, und eines erythrozytbewegungsbasierten Differenzbild-Verfahrens, das sich auf die reduzierte Bildfläche RBF der selektierten Analysepositionsbildfläche $SAF_2$ mit einem selektierten Blut durchflossenen Gefäß des Blutgefäßgeflechtes bezieht.

Die optische Vorrichtung zur Einbringung von Laserstrahlung, zur Erfassung von Rückstreustrahlung und Analysepositionsbildflächen $SAF_1$, $SAF_2$ kann zumindest enthalten:

ein erstes Mess-Objektiv zur Durchführung eines kontrastbasierten Auswerteverfahrens und
ein zweites Mess-Objektiv zur Durchführung eines erythrozytbewegungsbasierten Differenzbild-Verfahrens,
wobei sich die Mess-Objektive jeweils zur Durchführung der beiden Verfahren abwechselnd in den Strahlengang der Kamera einschieben.

Anstelle der beiden Mess-Objektive kann ein Objektiv mit variabler Vergrößerung in den Strahlengang der Kamera eingebracht werden, so dass keine Verschiebung von Objektiven erforderlich ist.

Die optische Vorrichtung zur Einbringung von Laserstrahlung, zur Erfassung von Rückstreustrahlung und Ana-

lysepositionsbildflächen SAF$_1$, SAF$_2$ kann auch enthalten:

> ein erstes Kamera-Objektiv zur Durchführung eines kontrastbasierten Auswerteverfahrens und
> ein zweites Kamera-Objektiv zur Durchführung eines erythrozytbewegungsbasierten Differenzbild-Verfahrens,
> wobei sich die Kamera-Objektive jeweils zur Durchführung der beiden Verfahren abwechselnd in den Strahlengang der Kamera einschieben.

Anstelle der beiden Kamera-Objektive kann ein Objektiv mit variabler Vergrößerung in den Strahlengang der Kamera eingebracht werden, so dass keine Verschiebung von Objektiven erforderlich ist.

Die optische Vorrichtung zur Einbringung von Laserstrahlung, zur Erfassung von Rückstreustrahlung und Analysepositionsbildflächen SAF$_1$, SAF$_2$ steht mit der koordinativen x-,y-,z-Positioniereinheit in Verbindung, wobei die koordinative x-,y-,z-Positioniereinheit die optische Vorrichtung in x-,y-,z-Richtung steuernd bewegen kann.

Der Gesamtablauf der Einstellung des Laserfokus des Anregungslasers besteht aus folgenden Schritten:

Schritt 1: Positionieren eines Eies in einer Halterungseinrichtung,

Schritt 2: Einbringen eines Loches/einer Öffnung in die Kalkschale entweder direkt über dem Blutgefäßgeflecht oder in einem Bereich außerhalb des Blutgefäßgeflechtes, wobei sich der Embryo mit dem extraembryonalen Blutgefäßgeflecht aufgrund der Dichteunterschiede im Ei ab dem dritten Bruttag immer am höchsten Punkt des Ei-Inneren befindet,

Schritt 3: Positionieren des Blutgefäßgeflechtes im Bereich der Öffnung durch Bewegung/Drehung des Eies für den Fall der Locherzeugung in der Kalkschale außerhalb des Gefäßgeflechtes, bis die erzeugte Öffnung den höchsten Punkt des Eies bildet.

Schritt 4: Beleuchtung des Embryos und des Blutgefäßgeflechts mittels Strahlung im sichtbaren Wellenlängenbereich oder im grünen Wellenlängenbereich unter Verwendung eines Visions-Systems.

Schritt 5: Bestimmung der Fokusebene:

- durch Nutzung einer angepassten Objektivvergrößerung, wobei eine Adaption an den Lochdurchmesser erfolgt,
- damit die schärfste Ebene $z_{Fokus}$ innerhalb des Blutgefäßes ermittelt werden kann, wird mittels Schrittmotoren einer koordinativen x-,y-,z-Positioniereinheit/ Steuereinheit ein definierter Bereich von $z_{max} < z_{Fokus} < z_{min}$, wobei $z_{min}$ den minimaler Abstand und $z_{max}$ den maximalen Abstand zwischen Objektiv und Blutgefäß sind, abgefahren,
- Ermittlung des Maximums mittels eines kontrastbasierten Schärfeindikators mit Hilfe eines kontrastbasierten Verfahrens, z.B. mittels einer Varianz-Darstellung "Variance" mit
Schärfewert der Varianz:

$$S_{VAR} = \frac{1}{H\ddot{o}he \cdot Breite} \sum_{(x,y)\in I} \sum_{(x,y)\in I} (I(x,y) - \mu)^2$$

wobei, wenn notwendig, eine Glättung des Verlaufes des Schärfeindikators zur Minimierung der durch Rauschen bedingten Störeinflüsse, z.B. mit einem Savitzky-Golay-Filter, und
eine Einschränkung des definierten Bereiches für das lokale Maximum $z_{Fokus}$ durch Bildung der ersten Ableitung der Funktion des Schärfewertes der Varianz erzeugt wird:
- Positionierung der z-Achse über eine absolute Bewegung zum ermittelten Punkt $z_{Fokus}$ und
- Bildaufnahme des gesamten Gefäßgeflechtes des definierten Bereiches mittels einer Kamera mit einem vorgegebenen Objektiv.

Schritt 6: Blutgefäßselektion durch Auswertung der Bildmatrix:

- Homogenisierung / Entfernung des Hintergrundes (alle Bestandteile des Eies, die nicht zum extraembryonalen Gefäßgeflecht gehören, z.B. Dottersack, Embryo mit Herz) mittels mathematischer Algorithmen, z.B. mittels eines Rolling-Ball-Algorithmus,
- Maximierung des Kontrastes, z.B. durch lineare Transformation der Grauwertfunktion,

- Segmentierung der Blutgefäße, z.B. bestehend aus den Schritten

  - Schwellwertbestimmung,
  - Partikelentfernung mittels Erosion und
  - morphologischem Schließen,

- Extraktion des Blutgefäßes und Bestimmung der selektierten Analysepositionsbildfläche $SAF_2$ für die anschließende optische Messung:
  Ermittlung der selektierten Analysepositionsbildfläche $SAF_2$ durch Bildung des geometrischen Schwerpunktes aus dem Objekt mit der größten Fläche A, welches nach mehrfacher Erosion bis zum Iterationsschritt i-1 erhalten wird (wenn $A_{max}$= 0 bei Laufvariable i), wobei unter Erosion die morphologische Operation in der Bildverarbeitung, bei dem Pixel durch ein Strukturelement abgetragen werden, verstanden wird.

Schritt 7: Adaption der Objektivvergrößerung an die Autofokussierung und das Tracking des Laserfokus in der selektierten Analysepositionsbildfläche $SAF_2$, beispielsweise durch einen Objektivwechsel.

Schritt 8: Autofokussierung und Tracking der selektierten Analysepositionsbildfläche $SAF_2$ im Blutgefäß:

Unterschritt 8.1: Erneute Bestimmung der Fokusebene entsprechend Schritt 5 nur dann, wenn durch ein starkes Absinken der Blutgefäße während des Objektivwechsels der Fokus des Objektivs des Anregungslasers für die optische Messung nicht mit dem Fokus des Objektivs für die Blutgefäßselektion übereinstimmt, d.h., wenn Laserfokus und Fokus der Kamera nicht konform sind.

Unterschritt 8.2: Exakte Positionierung der z-Achse im Blut durchflossenen Gefäß durch Anwendung des erythrozytbewegungsbasierten Differenzbild-Verfahrens auf der Basis der Änderung der Pixelintensität durch Änderung des Bildes aufgrund des geänderten Blut-Durchflusses - im Folgenden als erythrozytbewegungsbasiertes Diffenrenzbild-Verfahren genannt - , wobei das erythrozytbewegungsbasierte Differenzbild-Verfahren als Differenz der Pixelintensitäten der beiden nacheinander registrierten Bilder eines Blut durchflossenen Gefäßes definiert wird.

Dabei erfolgt eine

- Auswahl eines reduzierten Bildausschnitts RBA bzw. ROI (engl. region of interest) in einem Blut durchflossenen Gefäß aus der selektierten Analysepositionsbildfläche $SAF_2$.

Eine exakte Positionierung des reduzierten Bildausschnitts RBA in das Bild des gesamten Blutgefäßes mittels des Differenzbild-Verfahrens ist notwendig, da ein kontrastbasiertes Verfahren das Maximum des Schärfewertes für das gesamte Bild mit der Analysepositionsbildfläche $SAF_1$ erstellt, jedoch nicht den Laserfokus in das Blut durchflossene Gefäß hinein selbst.

Die Ermittlung des exakten Fokus-Punktes $z_{Fokus\_exakt}$ funktioniert folgendermaßen:

- der abgefahrene Bereich der reduzierten Bildfläche RBA wird aus dem bekannten Punkt $z_{Fokus}$ und den erwarteten Änderungen $\Delta z_{max}$ und $\Delta z_{min}$ ermittelt, die durch das Absinken des Embryos innerhalb des Eies und der unpräzisen kontrastbasierten Fokussierung auf das Blutgefäß entstehen:

$$z_{max\_exakt} = z_{Fokus} + \Delta z_{max} \text{ und } z_{min\_exakt} = z_{Fokus} + \Delta z_{min},$$

- Glättung des Schärfewertes vom Differenzbild bevorzugt durch einen kausalen gleitenden Mittelwert, um die Pulsation der Erythrozyten in den Blutgefäßen auszugleichen,
- Ermittlung des Maximums des Schärfewertes mittels Differenzbild,
- exakte Positionierung der z-Achse in das Blutgefäß.

Zusätzlich wird vom ermittelten Maximum $z_{Fokus\_exakt}$ ein Versatz benötigt, damit gewährleistet ist, dass der Zweipunktregler der Positioniereinheit immer kontinuierlich hinterher eilt: $z_{Versatz} = z_{Fokus\_exakt} - \Delta z_{Versatz}$
Differenzbild-Verfahren: Zeitpunkt t = 0 → Kurve DifR
Mittels einer Absolutbewegung wird der Ausgangspunkt $z_{Versatz}$ für die Regelung angefahren.

Aufgrund des Embryo-Absinkens im Ei verschiebt sich die Kurve DifR-t mit größer werdender Zeit t,

- Halten der Position im Blutgefäß mittels einer Regelung, z.B. einer Zweipunktregelung der Positioniereinheit.

Unterschritt 8.3: Ausgleich der Bewegungen in der x,y Ebene, z.B. durch einen Templatevergleich.

Dabei wird die Bildgebung der fließenden Erythrozyten eines Blutgefäßes innerhalb der reduzierten Bildfläche RBA - Roi ("region of interest") - und deren Auswertung über das erythrozytbewegungsbasierte Differenzbild-Verfahren zweier aufeinanderfolgender, durch den Blutfluss, d.h. die Bewegung der Erythrozyten, in der Intensität der Pixel veränderten Bilder genutzt, wobei sich die Intensität der einzelnen Pixel unterschiedlich ändert, um den Laserfokus des Anregungslasers und den Fokus der Kamera nachgeführt in dem sich seine Lage verändernden Blutgefäß zu halten. Damit ist die Differenz der beiden in der Auswertung berücksichtigten nachfolgenden Bilder durch die Bewegung der Erythrozyten selbst Teil des Schärfeindikators für das lageveränderte Blutgefäß, weil sich die Bewegung der Erythrozyten im Blutgefäß in veränderten Intensitäten der Pixel der berücksichtigten, zu verarbeitenden Bilder niederschlägt.

Der Schärfeindikator auf der Basis des Differenzbild-Verfahrens der Bewegung der Erythrozyten unterstützt das Positionieren des Laserfokus des Anregungslasers in das Blutgefäß eines embryonalen Vogeleies hinein mit hoher Genauigkeit und wesentlich geringerer Standardabweichung im Vergleich zu kontrastbasierten Auswertungsalgorithmen.

Das Verfahren zeichnet sich weiterhin dadurch aus, dass nur der reduzierte Bildausschnitt RBA notwendig ist, wodurch die Rechenzeit erheblich reduziert und erstmals ein Echtzeittracking in-ovo erlaubt wird.

Weitere Vorteile dieses Verfahrens bestehen darin, dass

- ein einfacher Aufbau mit nur einer Kamera,
- konfokale und nichtkonfokale Messungen,
- eine Messung an Blutgefäßen aller Größen mit der Voraussetzung sich im Blutgefäß bewegender Erythrozyten

erfolgen können.

Weiterbildungen und weitere Ausgestaltungen der Erfindung werden in weiteren Unteransprüchen angegeben.

Die Erfindung wird mittels eines Ausführungsbeispieles anhand mehrerer Zeichnungen näher erläutert. Es zeigt:

Fig. 1 eine schematische Darstellung der Vorrichtung zur Bestimmung des Geschlechtes von befruchteten und bebrüteten Vogeleiern nach dem Stand der Technik,

Fig. 2 eine schematischen Darstellung der Aufbau einer Vorrichtung zur Einstellung des Laserfokus eines Anregungslasers in Blut durchflossenen Gefäßen für optische Messungen, vorzugsweise für Ramanspektroskopische Messungen in geöffneten Vogeleiern zur Bestimmung des Geschlechts von Vogeleiern, wobei Fig. 2a eine vergrößerte Darstellung der optischen Vorrichtung zur Einbringung von Laserstrahlung, zur Erfassung von Rückstreustrahlung und Analysepositionsbildflächen $SAF_1$, $SAF_2$ zeigt,

Fig. 3 eine schematische Darstellung eines Teils eines Eies mit dem Fokus der Kamera im selektierten Blut durchflossenen Gefäß im geöffneten Ei, wobei Fig. 3a die Nachführung des Fokus der Kamera bei einer ersten Absenkung der Eiweißschicht einschließlich des Blutgefäßgeflechts in zAchsenrichtung, Fig. 3b die Nachführung des Fokus der Kamera bei einer weiteren Absenkung der Eiweißschicht einschließlich des Blutgefäßgeflechts in z-Achsenrichtung und Fig. 3c eine vergrößerte Darstellung gemäß Fig. 3 des Fokus des Objektivs der Kamera und des Laserfokus des Anregungslasers zeigen,

Fig. 4 eine schematische Darstellung einer Analysepositionsfläche $SAF_1$ des Blutgefäßgeflechts einschließlich des Embryos für die Nutzung des kontrastbasierten Auswerteverfahrens einschließlich der selektierten Analysepositionsbildfläche $SAF_2$,

Fig. 5 eine schematische Darstellung der vergrößerten, auf die selektierte Analysepositionsbildfläche $SAF_2$ und der auf die auf das Blut durchflossene Gefäß reduzierten Bildfläche RBF (ROI) für die Nutzung des erythrozytbewegungsbasierten Differenzbild-Verfahrens,

Fig. 6 eine Darstellung des normierten Schärfewertes in Abhängigkeit der Relativbewegung der z-Achse für ein kontrastbasiertes Auswerteverfahren und für ein erythrozytbewegungsbasiertes Differenzbild-Verfahren,

Fig. 7 eine vergrößerte, der Fig. 6 angepasste Darstellung des Laserfokus und des Fokus des Objektivs der Kamera mit Bezug auf Fig. 3c,

Fig. 8   eine schematische Darstellung der durchzuführenden Verfahrensschritte in dem Selektions- und Positionsrechner SPR,

Fig. 9   eine schematische Darstellung der durchzuführenden Verfahrensschritte zur Selektion eines Blut durchflossenen Gefäßes,

Fig. 10   eine schematische Darstellung von Detailschritten zur Selektion des Blut durchflossenen Gefäßes,

Fig. 11   eine schematische Darstellung der Verfahrensschritte zur Positionierung und Haltung des selektierten Blut durchflossenen Gefäßes bezüglich der z-Achse,

Fig. 12   eine schematische Darstellung der Verfahrensschritte zur Positionierung und Haltung des selektierten Blut durchflossenen Gefäßes in Bezug auf die Fokussierung auf die Erythrozyten für den Ausgleich in der x-, y- Horizontalebene bei Bewegung des Blut durchflossenen Gefäßes bezüglich der x-, y- Achsen,

Fig. 13   Kurvendarstellungen des Schärfewertes bei einem kontrastbasierten Auswerteverfahren und der ersten Schärfewert-Ableitung zur Eingrenzung des $Z_{fokus}$-Bereiches in Bezug auf die Distanz der z-Achse mit den eingestellten Zweipunkten $Z_{min}$ und $Z_{max}$ in Bezug auf den Fokus $Z_{fo\text{-}kus}$ und

Fig. 14   Kurvendarstellungen des Schärfewertes bei einem erythrozytbewegungsbasierten Differenzbild-Verfahren in Bezug auf die Distanz der z-Achse mit den eingestellten Zweipunkten $Z_{min\_exakt}$ und $Z_{max\_exakt}$ und dem eingestellten Punkt $Z_{versatz}$ und der Versatz-Differenz $\Delta Z_{max}$

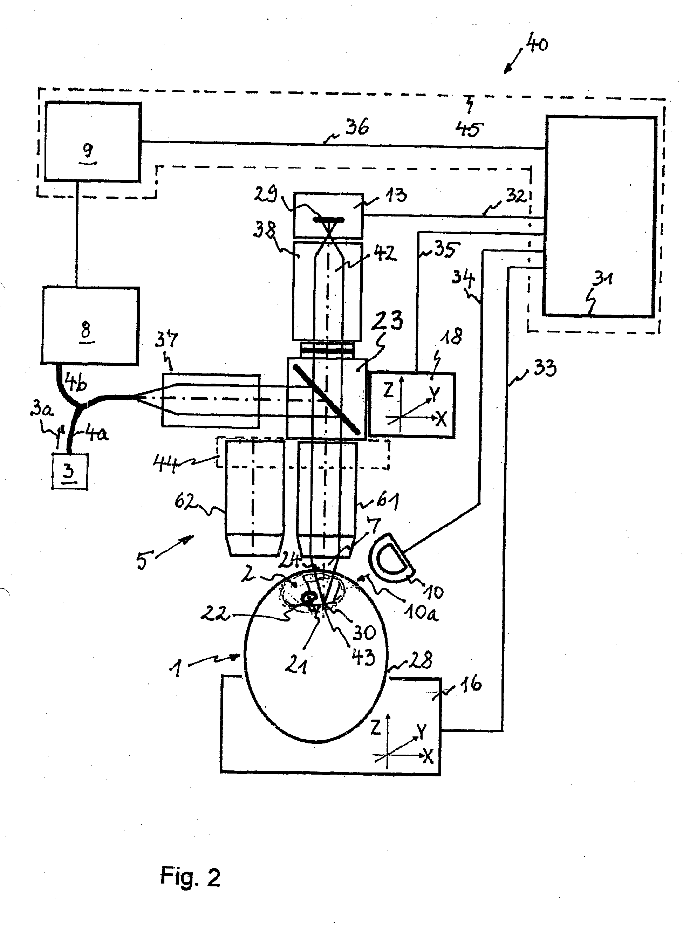

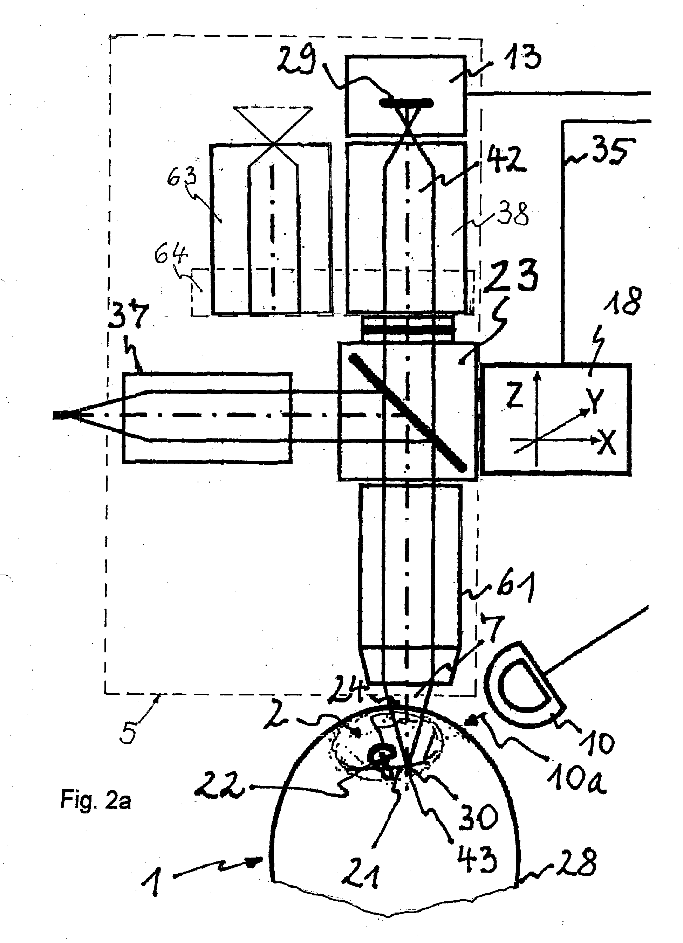

Im Folgenden werden die Fig. 2 und Fig. 2a gemeinsam betrachtet.

In Fig. 2 ist in einer schematischen Darstellung der Aufbau einer Vorrichtung 40 zur Einstellung des Laserfokus 30 eines Anregungslasers 3 in Blut durchflossenen Gefäßen 27, 21 für optische Messungen zur Bestimmung des Geschlechts von Vogeleiern 1 gezeigt, wobei im Folgenden als Ausführungsbeispiel für die optischen Messungen Ramanspektroskopische Messungen erläutert werden.

In Fig. 2a ist eine vergrößerte Darstellung gemäß Fig. 2 der optischen Vorrichtung 5 zur Einbringung von Laserstrahlung 3a, zur Erfassung von Rückstreustrahlung 7 und Analysepositionsbildflächen $SAF_1$, $SAF_2$ gezeigt.

Der Embryo 22 einschließlich der Blut durchflossenen Gefäße 27, 21 ist beweglich im Ei 1 und ist noch nicht zum Zeitpunkt einer optischen Messung der Ramanstreustrahlung an der Kalkschale 28 fixiert.

Die Vorrichtung 40 umfasst in Fig. 2 zumindest

- eine Halterungseinrichtung 16 für das mit einem Loch 2 versehene Ei 1, wobei die Eier 1 jeweils mit dem spitzen Pol 24 nach oben gelagert sind,
- eine Beleuchtungseinrichtung 10 für die Beleuchtung der im Ei 1 befindlichen Fläche mit Eiweißschicht 26 und mit Blutgefäßgeflecht 27,
- ein erstes Mess-Objektiv 61 und ein zweites in den Strahlengang 42 einbringbares Mess-Objektiv 62,
- ein Kamera-Objektiv 38,
- einen Strahlteiler 23, der zwischen dem Mess-Objektiv 61 und dem Kamera-Objektiv 38 angeordnet ist,
- eine Kamera 13,
- eine CCD-Matrix 29 in der Kamera 13,

wobei das erste Mess-Objektiv 61 zur Durchführung eines kontrastbasierten Auswerteverfahrens und das zweite Mess-Objektiv 62 zur Durchführung eines erythrozytbewegungsbasierten Differenzbild-Verfahrens vorgesehen sind, wobei sich die Mess-Objektive 61, 62 abwechselnd in den Strahlengang 42 der

Kamera 13 einschieben und das zweite Mess-Objektiv 62 eine kürzere Brennweite und damit eine Vergrößerung gegenüber dem ersten Mess-Objektiv 61 aufweist,

- eine Auswerteeinrichtung 9 für Ramanspektroskopische Messungen,
- eine Auswerteeinheit 31, die zumindest umfasst

  - eine Verbindungsleitung 32 zur Kamera 13 mit CCD-Matrix 29,
  - eine Verbindungsleitung 33 zur Halterungseinrichtung 16,
  - eine Verbindungsleitung 34 zur Strahlungsquelle 10,

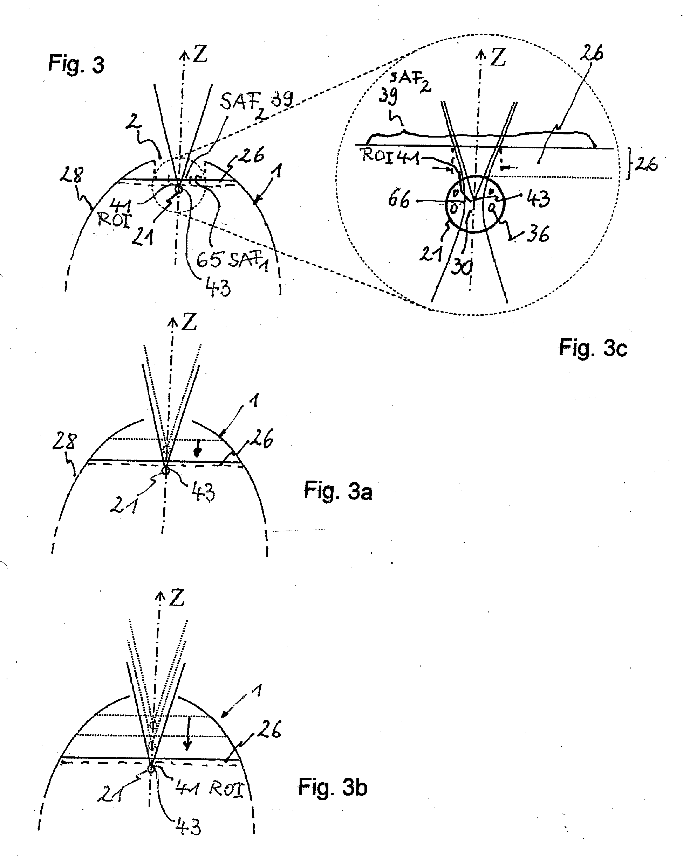

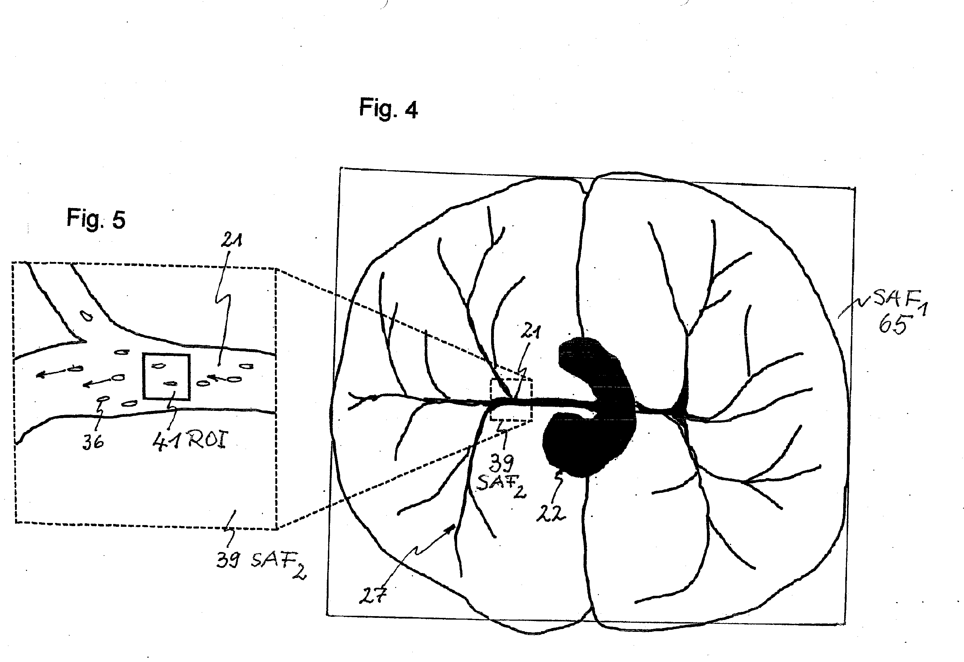

- eine Verbindungsleitung 35 zur koordinativen x-,y-,z-Positioniereinheit 18,
- eine Verbindungsleitung 36 zur Auswerteeinrichtung 9 für Ramanspektroskopische Messungen,

wobei die Auswerteeinheit 31 programmtechnische Mittel zur Durchführung eines kontrastbasierten Verfahrens, das sich gemäß Fig. 3 und Fig. 4 auf eine gesamte Analysepositionsbildfläche $SAF_1$ 65 mit dem Blutgefäßgeflecht 27 bezieht, und zur Durchführung eines erythrozytbewegungsbasierten Differenzbild-Verfahrens, das sich gemäß Fig. 3 und Fig. 5 auf eine reduzierte Bildfläche RBF 41 der selektierten Analysepositionsbildfläche $SAF_2$ 39 auf einem selektierten Blut durchflossenen Gefäß 21 des Blutgefäßgeflechtes 27 bezieht.

Die optische Vorrichtung 5 zur Einbringung von Laserstrahlung 3a, zur Erfassung von Rückstreustrahlung 7 und Analysepositionsbildflächen $SAF_1$, $SAF_2$ kann zumindest in Fig. 2 umfassen

- die beiden Mess-Objektive 61, 62,
- den Strahlteiler 23,
- das Kamera-Objektiv 38,
- die Kamera 13 mit einer CCD-Matrix 29,
- den Kollimator 37 und
- den Revolver 44 als Objektivwechsler für den Wechsel der beiden eine unterschiedliche Brennweite aufweisenden Mess-Objektive 61, 62,

wobei die optische Vorrichtung 5 fest mit der koordinativen x-,y-,z-Positioniereinheit 18 verbunden ist.

In Fig. 2a kann eine optische Vorrichtung 5 zur Einbringung von Laserstrahlung 3a, zur Erfassung von Rückstreustrahlung 7 und Analysepositionsbildflächen $SAF_1$, $SAF_2$ aufweisen, die zumindest in Fig. 2 umfassen kann

- das Mess-Objektiv 61,
- den Strahlteiler 23,
- das erste Kamera-Objektiv 38 und ein zweites in den Strahlengang 42 einbringbares Kamera-Objektiv 63,
- die Kamera 13 mit einer CCD-Marix 29,
- den Kollimator 37 und
- den Revolver 64 als Objektivwechsler für den Wechsel der beiden eine unterschiedliche Brennweite aufweisenden Kamera-Objektive 38, 63,

wobei die optische Vorrichtung 5 fest mit der koordinativen x-,y-,z-Positioniereinheit 18 verbunden ist.

Da für die Durchführung des Verfahrens eine Brennweitenänderung des Mess-Objektivs 61 oder des Kamera-Objektivs 38 erforderlich ist, können anstelle der eingebrachten, wechselnden Objektive 62 und 63 auch die Objektive 61 und 38 mit veränderlichen Brennweiten ausgebildet sein. Wesentlich ist, dass beim Wechsel vom kontrastbasierten Auswerteverfahren zum erythrozytbewegungsbasierten Differenzbild-Verfahren brennweitengeänderte Objektive eingesetzt werden.

Anstelle der beiden Mess-Objektive 61, 62 kann ein Objektiv mit variabler Vergrößerung in den Strahlengang 42 der Kamera 13 eingebracht sein, so dass kein zweites Messobjektiv und keine Verschiebung von Mess-Objektiven erforderlich sind.

Anstelle der beiden Kamera-Objektive 38, 63 kann ein Objektiv mit variabler Vergrößerung in den Strahlengang 42 der Kamera 13 eingebracht sein, so dass kein zweites Kamera-Objektiv und keine Verschiebung von Kamera-Objektiven erforderlich sind.

In Fig. 3 sind mehrere schematische Darstellungen des mit dem Laserfokus 30 gleichbeabstandeten Fokus 43 der Kamera 13 im selektierten Blut durchflossenen Gefäß 21 des geöffneten Eies 1 gezeigt, wobei Fig. 3a die Nachführung des Fokus 43 und somit des Laserfokus 30 bei einer ersten Absenkung der Eiweißschicht 26 einschließlich des Blutgefäßgeflechts 27 in z-Achsenrichtung,

Fig. 3b die Nachführung des Fokus 43 und somit des Laserfokus 30 bei einer weiteren Absenkung der Eiweißschicht 26 einschließlich des Blutgefäßgeflechts 27 in z-Achsenrichtung und Fig. 3c eine vergrößerte Darstellung gemäß Fig. 3 des Fokus 43 und des gleich zugeordneten Laserfokus 30 zeigen.

Dabei sind dem Bezugszeichen 39 die reduzierte Analysepositionsbildfläche $SAF_2$ und dem Bezugszeichen 65 die Analysepositionsbildfläche $SAF_1$ zugeordnet.

Das Verfahren zur Einstellung des Laserfokus 30 eines Anregungslasers 3 in Blut durchflossene Gefäße 27, 21 für Ramanspekroskopische Messungen oder für Fluoreszenzmessungen zur Bestimmung des Geschlechts von Vogeleiern 1, wobei der Embryo 22 einschließlich des Blutgefäßgeflechts 27 beweglich im Ei 1 ist und noch nicht zum Zeitpunkt einer Messung der Ramanstreustrahlung oder Fluoreszenzstrahlung an der Kalkschale 28 fixiert ist, durchgeführt mit der Vorrichtung 40 weist folgende Schritte auf:

- Selektion eines Blut durchflossenen Gefäßes 21 in dem lochgeöffneten und bebrüteten Ei 1 mit einem kontrastbasierten Auswerteverfahren unter zumindest grüner Beleuchtung eines Blutgefäßgeflechts 27 ,
- Herstellen eines Fokus 43 für eine Kamera 13 in dem selektierten Blut durchflossenen Gefäß 21 durch das dem Blutgefäß 21 zugewandte Loch 2 in der Kalkschale 28 hindurch,
- Herstellen eines Laserfokus 30 mit dem ersten Mess-Objektiv 61 für einen Anregungslaser 3 in dem selektierten Blut durchflossenen Gefäß 21 durch das dem Blutgefäß 21 zugewandte Loch 2 in der Kalkschale 28 hindurch, wobei das Herstellen der beiden Fokusse 43 und 30 mit einer optischen Vorrichtung 5 zur Einbringung von Laserstrahlung 3a, zur Erfassung von Rückstreustrahlung 7 und Analysepositionsbildflächen $SAF_1$, $SAF_2$ durchgeführt wird,
- Halten des Blut durchflossenen Gefäßes 21 im Laserfokus 30 eines gewechselten zweiten Objektivs 62 innerhalb des selektierten Blutgefäßes 21 und Durchführung eines erythrozytbewegungsbasierten Differenzbild-Verfahrens aufgrund der Intensitätsänderungen der Pixel zumindest zweier nachfolgender Bilder infolge der Bewegung der Erythrozyten innerhalb des selektierten Blutgefäßes 21 und Nachführen des Laserfokus 30 und des Fokus 43 bei Absenken der selektierten Analysepositionsbildfläche $SAF_2$ 39 des Blutgefäßgeflechtes 27,
- Ausgleich von auftretenden x-,y-Horizontalbewegungen mittels der optischen Vorrichtung 5 zur Einbringung von Laserstrahlung 3a, zur Erfassung von Rückstreustrahlung 7 und Analysepositionsbildflächen $SAF_1$, $SAF_2$,
- Durchführung von optischen Messungen, insbesondere von Ramanspektroskopischen Messungen und Fluoreszenzmessungen.

Das Verfahren umfasst im Detail folgende Schritte:

- Einbringung eines Lochs 2 in der Kalkschale 28 mittels einer Locherzeugungseinheit und Positionierung der Blutgefäße 21 im Bereich des Loches 2,
- Suchen der sich im Ei 1 ausbildenden Blutgefäße 21 mittels eines zumindest aus einer Strahlungseinrichtung 10 und aus einem Detektor/Kamera 13 bestehenden Visions-Systems 10, 13 und einer koaxialen oder lateralen Beleuchtung mit Licht 10a des sichtbaren Wellenlängenbereiches,
- Positionieren zumindest des Blut durchflossenen Gefäßes 21 in den Laserfokus 30 des Anregungslasers 3 entweder durch Bewegen des Eies 1 mittels einer gesteuerten Positioniereinheit 16 oder durch ein gesamtheitliches Bewegen der optischen Vorrichtung 5 zur Einbringung von Laserstrahlung 3a, zur Erfassung von Rückstreustrahlung 7 und Analysepositionsbildflächen $SAF_1$, $SAF_2$ oder durch einen Wechsel mindestens eines Objektivs 61, 62 oder gemäß Fig. 2a mindestens eines Kamera-Objektivs 38, 63 in der optischen Vorrichtung 5 zur Einbringung von Laserstrahlung 3a, zur Erfassung von Rückstreustrahlung 7 und Analysepositionsbildflächen $SAF_1$, $SAF_2$,
- Registrieren der Rückstreustrahlung 7 des bestrahlten Blut durchflossenen Gefäßes 21 mittels der Vorrichtung 5 zur Einbringung von Laserstrahlung 3a und zur Erfassung von Rückstreustrahlung 7 und Analysepositionsbildflächen $SAF_1$, $SAF_2$, wobei während der Registrierung der Rückstreustrahlung 7 eine Bewegung des Blutgefäßes 21 aus dem Laserfokus 30 und dem Fokus 43 der Kamera 13 heraus durch Nachführung des Eies 1 mittels einer gesteuerten Positioniereinheit 16 und/oder durch ein gesamtheitliches Bewegen der optischen Vorrichtung 5 zur Einbringung von Laserstrahlung 3a, zur Erfassung von Rückstreustrahlung 7 und Analysepositionsbildflächen $SAF_1$, $SAF_2$ oder durch einen Wechsel mindestens eines Objektivs 61, 62 oder 38, 63 in der optischen Vorrichtung 5 zur Einbringung von Laserstrahlung 3a, zur Erfassung von Rückstreustrahlung 7 und Analysepositionsbildflächen $SAF_1$, $SAF_2$ vermieden werden kann,
- Auswertung der Rückstreustrahlung 7 und Bestimmung des Geschlechtes des Embryos in einer Auswerteeinrichtung 9,
- Anzeige des Geschlechtes des Embryos im Vogelei 1,

dadurch gekennzeichnet,
dass in der Auswerteeinrichtung 9 mittels programmtechnischen Mitteln in einem ersten Algorithmus ein kontrastbasiertes Verfahren zur Festlegung einer gesamten Bildfläche der Analysepositionsbildflächen $SAF_1$ 65 mit dem Blut durchflossenen Blutgefäßgeflecht 27 durchgeführt wird, und
in der Auswerteeinheit 9 mittels programmtechnischer Mittel in einem zweiten Algorithmus ein erythrozytbewegungsbasiertes Differenzbild-Verfahren zur Einstellung des Laserfokus 30 mittels der optischen Vorrichtung 5 zur Einbringung von Laserstrahlung 3a, zur Erfassung von Rückstreustrahlung 7 und Analysepositionsbildflächen $SAF_1$, $SAF_2$ für eine reduzierte Bildfläche RBF 41 der selektierten Analysepositionsbildfläche $SAF_2$ 39 mit dem selektierten Blut durchflossenen Gefäß 21 durchgeführt wird, wobei beim erythrozytbewegungsbasierten Differenzbild-Verfahren die Intensitätsinhalte $I_{t-1}(x)$, $I_t(x)$ von mindestens zwei aufeinanderfolgenden Bildern einer auf das selektierte Blut durchflossenen Gefäß 21 reduzierten Bildfläche RBF mit der - ROI - 41 der $SAF_2$ 39 verglichen werden, wobei die Intensitätsinhalte $I_{t-1}(x)$, $I_t(x)$ von den sich jeweils im Bild widerspiegelnden Bewegungen der Erythrozyten 36 unterschiedlich ausgebildet werden, und wobei der Laserfokus 30 des Anregungslasers 3 und der Fokus 43 der Kamera 13 mittels der optischen Vorrichtung 5 zur Einbringung von Laserstrahlung 3a, zur Erfassung von Rückstreustrahlung 7 und Analysepositions-

bildflächen SAF$_1$, SAF$_2$ auf eine dem selektierten Blut durchflossenen Gefäß 21 zugeordnete reduzierte Bildfläche RBF der selektierten Analysepositionsbildfläche SAF$_2$ 39 eingestellt wird, wobei sich der Laserfokus 30 und der Fokus 43 im finalen Schritt immer innerhalb des selektierten Blut durchflossenen Gefäßes 21 befinden.

Der Gesamtablauf der Einstellung des mit dem Fokus 43 der Kamera 13 konform bewegbaren Laserfokus 30 des Anregungslasers 3 besteht aus folgenden Schritten:

Schritt 1: Positionieren eines Eies 1 in einer Halterungseinrichtung 16,

Schritt 2: Einbringen eines Loches/einer Öffnung 2 in die Kalkschale 28 entweder direkt über dem Blutgefäßgeflecht 27 oder in einem Bereich außerhalb des Blutgefäßgeflechtes 27, wobei sich der Embryo 22 mit dem Blutgefäßgeflecht 27 aufgrund der Dichteunterschiede im Ei 1 ab dem dritten Bruttag immer am höchsten Punkt des Ei-Inneren befindet,

Schritt 3: Positionieren des Blutgefäßgeflechts 27 im Bereich der Öffnung 2 durch Bewegung/Drehung des Eies 1 für den Fall der Locherzeugung in der Kalkschale 28 außerhalb des Blutgefäßgeflechtes 27, bis die erzeugte Öffnung den höchsten Punkt des Eies 1 bildet:

Schritt 4: Beleuchtung des Embryos 22 und des Blutgefäßgeflechts 27 mittels Strahlung 10a im sichtbaren Wellenlängenbereich oder im grünen Wellenlängenbereich unter Verwendung der Strahlungseinrichtung 10.

Schritt 5: Bestimmung der Fokusebene:

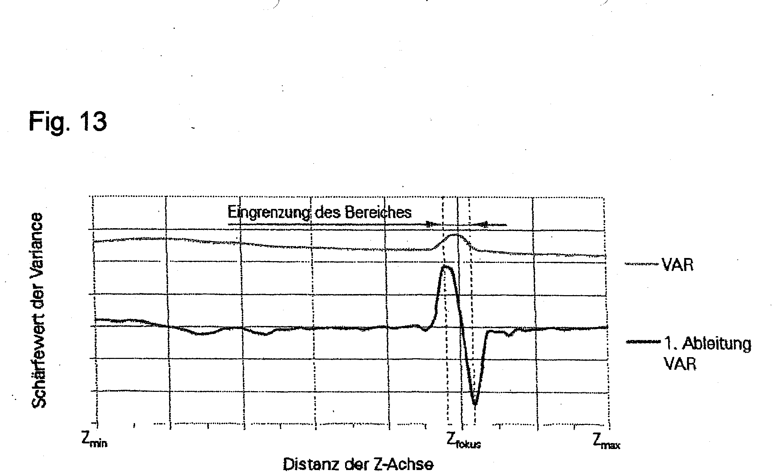

- durch Nutzung der angepassten Objektivvergrößerung, wobei eine Adaption an den Lochdurchmesser erfolgt,
- damit die schärfste Ebene $z_{Fokus}$ innerhalb des Blutgefäßes 21 ermittelt werden kann, wird mittels Schrittmotoren einer koordinativen x-,y-,z-Positioniereinheit/ Steuereinheit 18 gemäß Fig. 13 ein definierter Bereich von $z_{max}$ < $z_{Fokus}$ < bis $z_{min}$, wobei $z_{min}$ den minimaler Abstand und $z_{max}$ den maximalen Abstand zwischen Objektiv 61 und Blutgefäß 21 sind, abgefahren,
- Ermittlung des Maximums gemäß Fig. 13 mittels eines kontrastbasierten Schärfeindikators mit Hilfe eines kontrastbasierten Verfahrens, z.B. mittels eine Varianz-Darstellung "Variance" mit
Schärfewert der Varianz:

$$S_{VAR} = \frac{1}{H\ddot{o}he \cdot Breite} \sum_{(x,y)\in I} \sum_{(x,y)\in I} (I(x,y) - \mu)^2$$

wobei, wenn notwendig, eine Glättung des Verlaufes des Schärfeindikators zur Minimierung der durch Rauschen bedingten Störeinflüsse, z.B. mit einem Savitzky-Golay-Filter, und eine Einschränkung des definierten Bereiches für das lokale Maximum $z_{Fokus}$ durch Bildung der in Fig. 13 dargestellten ersten Ableitung der Funktion des Schärfewertes der Varianz erzeugt wird:

- Positionierung der z-Achse über eine absolute Bewegung zum ermittelten Punkt $z_{Fokus}$ und
- Bildaufnahme des gesamten Blutgefäßgeflechtes 27 des definierten Bereiches mittels einer Kamera 13 mit einem vorgegebenen ersten Objektiv 61.

Schritt 6: Blutgefäßselektion durch Auswertung der Bildmatrix:

• Homogenisierung / Entfernung des Hintergrundes (alle Bestandteile des Eies, die nicht zum extraembryonalen Gefäßgeflecht gehören, z.B. Dottersack, Embryo mit Herz) mittels mathematischer Algorithmen, z.B. mittels eines Rolling-Ball-Algorithmus,
• Maximierung des Kontrastes, z.B. durch lineare Transformation der Grauwertfunktion,
• Segmentierung der Blutgefäße 27, z.B. bestehend aus den Schritten

- Schwellwertbestimmung,
- Partikelentfernung mittels Erosion und
- morphologischem Schließen,

• Extraktion des Blutgefäßes 21 und Bestimmung der selektierten Analysepositionsbildfläche SAF$_2$ 39 für die anschließende optische Messung:

Ermittlung der selektierten Analysepositionsbildfläche $SAF_2$ 39 durch Bildung des geometrischen Schwerpunktes aus dem Objekt mit der größten Fläche A, welches nach mehrfacher Erosion bis zum Iterationsschritt i-1 erhalten wird (wenn $A_{max}$= 0 bei Laufvariable i), wobei unter Erosion die morphologische Operation in der Bildverarbeitung, bei dem Pixel durch ein Strukturelement abgetragen werden, verstanden wird.

Schritt 7: Adaption der Objektivvergrößerung an die Autofokussierung und das Tracking des Laserfokus 30 in der selektierten Analysepositionsbildfläche $SAF_2$ 39, beispielsweise durch einen Objektivwechsel von Objektiv 61 zu Objektiv 62 gemäß Fig. 2.

Schritt 8: Autofokussierung und Tracking der selektierten Analysepositionsbildfläche $SAF_2$ 39 mit im Blut durchflossenen Gefäß 21:

Unterschritt 8.1: Erneute Bestimmung der Fokusebene entsprechend Schritt 5.) nur dann, wenn durch ein starkes Absinken der Blutgefäße 27 einschließlich dem Blut durchflossenen Gefäß 21 während des Objektivwechsels der Laserfokus 30 des ersten Objektivs 61 des Anregungslasers 3 für die Ramanstreustrahlungsmessung nicht mit dem Fokus 43 der Kamera 13 für die Blutgefäßselektion übereinstimmt.

Unterschritt 8.2: Exakte Positionierung der z-Achse im Blutgefäß 21 durch Anwendung des erythrozytbewegungsbasierten Differenzbild-Verfahrens, wobei das erythrozytbewegungsbasierte Differenzbild-Verfahren als Differenz der Intensitäten der Pixel der beiden nacheinander registrierten Bilder des Blut durchflossenen Gefäßes 21 definiert wird.
Dabei erfolgt eine

- Auswahl eines reduzierten Bildausschnitts RBF 41 gemäß Fig. 5 in Form einer ROI (engl. region of interest) mit z.B. 400 x 400 Pixel (entspricht 100 $\mu$m x 100 $\mu$m) aus der selektierten Analysepositionsbildfläche $SAF_2$ 39.

Eine exakte Positionierung des reduzierten Bildausschnitts RBF 41 in das Bild des Blutgefäßes 21 mittels erythrozytbewegungsbasierten Differenzbild-Verfahrens ist notwendig, da gemäß Fig. 13 das kontrastbasierte Verfahren das Maximum des Schärfewertes für das gesamte Bild mit der $SAF_1$ 65 erstellt, jedoch nicht den Fokus 30 bzw. Fokus 43 in das Blutgefäß 21 hinein selbst erstellen kann.

Die Ermittlung des exakten Laserfokus-Punktes $z_{Fokus\_exakt}$ 30 funktioniert folgendermaßen ähnlich der Bestimmung der Fokusebene $SAF_2$ 39:
wobei

- sich der abgefahrene Bereich des ROI 41 aus dem ermittelten Punkt $z_{Fokus}$ und den erwarteten Änderungen $\Delta z_{max}$ und $\Delta z_{min}$ ermittelt, die durch das Absenken des Embryos 22 innerhalb des Eies 1 und der unpräzisen kontrastbasierten Fokussierung auf das Blutgefäß 21 entstehen:

$$z_{max\_exakt} = z_{Fokus} + \Delta z_{max} \text{ und } z_{min\_exakt} = z_{Fokus} + \Delta z_{min}$$

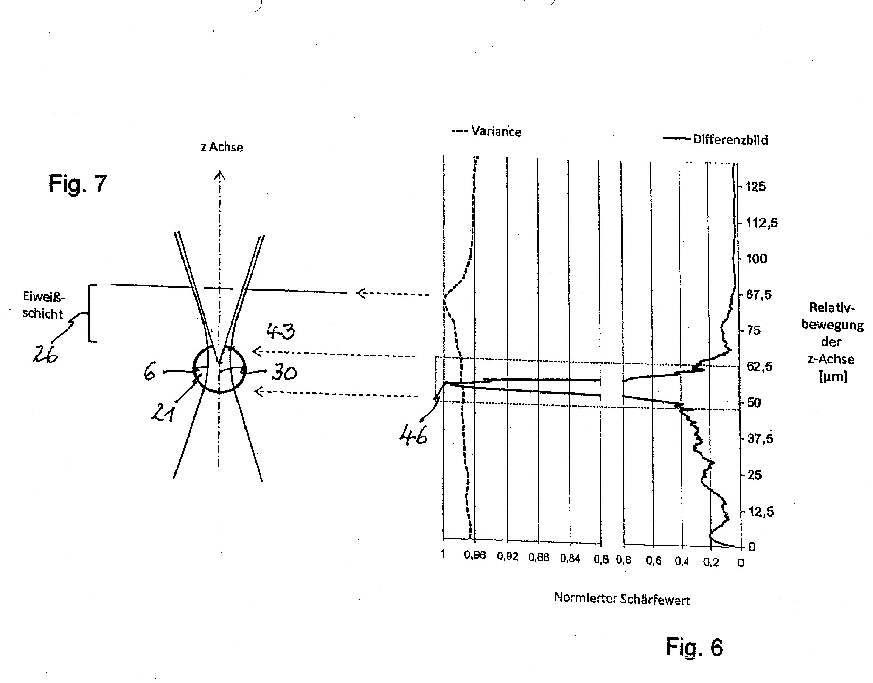

- Glättung des Schärfewertes vom erythrozytbewegungsbasierten Differenzbild bevorzugt durch einen kausalen gleitenden Mittelwert, um die Pulsation der Erythrozyten 36 in dem Blutgefäß 21 auszugleichen,
- Ermittlung des Maximums des Schärfewertes gemäß Fig. 6 mittels der jeweiligen erythrozytbewegungsbasierten Differenzbilder,
- exakte Positionierung der z-Achse in das Blutgefäß 21 gemäß Fig. 7.

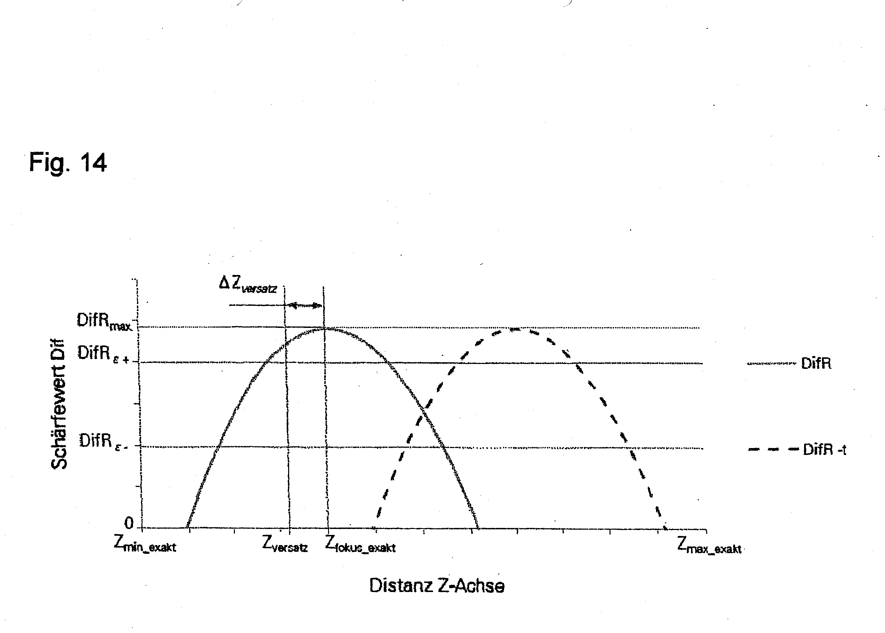

Zusätzlich wird gemäß Fig. 14 vom ermittelten Maximum $z_{Fokus\_exakt}$ ein Versatz benötigt, damit gewährleistet ist, dass der Zweipunktregler der Positioniereinheit 18 immer kontinuierlich hinterher eilt: $z_{Versatz} = z_{Fokus\_exakt} - \Delta z_{Versatz}$, wobei für das erythrozytbewegungsbasierte Differenzbild-Verfahren vom Zeitpunkt t=0 die durchgängig verlaufende Kurve DifR in Fig. 14 gezeigt wird.
Mittels einer Absolutbewegung wird der Ausgangspunkt $z_{Versatz}$ für die Regelung angefahren.
Aufgrund des Embryo-Absinkens im Ei 1 verschiebt sich die in Fig. 14 gestrichelte Kurve DifR-t mit größer werdender Zeit t, wodurch ein Halten der Position im Blutgefäß 21 mittels dieser Zweipunktregelung erfolgt.
Unterschritt 8.3: Ausgleich der Bewegungen in der x-,y-Ebene z.B. durch Templatevergleich.

Im Folgenden wird der Einsatz des erythrozytbewegungsbasierten Differenzbild-Verfahrens näher erläutert: Dabei wird die Bildgebung der fließenden Erythrozyten 36 eines Blutgefäßes 21 innerhalb der reduzierten Bildfläche RBA - ROI ("region of interest") - 41 und deren Auswertung über das erythrozytbewegungsbasierte Differenzbild-Verfahren zweier aufeinanderfolgender, durch den Blutfluss, d.h. die Bewegung der Erythrozyten, in der Intensität $I_t$ und $I_{t-1}$ veränderten Bilder zum Zeitpunkt $t$ und $t-1$ genutzt, um den Laserfokus 30 des Anregungslasers 2 und den Fokus 43 der Kamera 13, konform nachgeführt, in dem sich seine Lage verändernden Blutgefäß 21 zu halten. Damit ist die Differenz der beiden in der Auswertung berücksichtigten nachfolgenden Bilder durch die Bewegung der Erythrozyten 36 selbst Teil des in Fig. 6 dargestellten Schärfeindikators für das lageveränderte Blutgefäß 21 gegeben, weil sich die Bewegung der Erythrozyten 36 im Blutgefäß 21 in veränderten Pixelintensitäten $I_t$ und $I_{t-1}$ der berücksichtigten, zu verarbeitenden Bilder niederschlägt.

Der Schärfeindikator 46 in Fig. 6 auf der Basis des erythrozytbewegungsbasierten Differenzbild-Verfahrens bezüglich der Bewegung der Erythrozyten 36 unterstützt das Positionieren des Laserfokus 30 des Anregungslasers 3 in das Blutgefäß 21 eines embryonalen Vogeleies 1 hinein mit hoher Genauigkeit und wesentlich geringerer Standardabweichung im Vergleich zu kontrastbasierten Auswertungsalgorithmen.

Das Verfahren zeichnet sich weiterhin dadurch aus, dass nur der reduzierte Bildausschnitt RBF 41 (ROI) notwendig ist, wodurch die Rechenzeit erheblich reduziert und erstmals ein Echtzeittracking in-ovo erlaubt wird.

Weitere Vorteile dieses Verfahrens bestehen darin, dass

- ein einfacher Aufbau mit nur einer Kamera 13,
- konfokale/nichtkonfokale Messungen,
- eine Messung an Blutgefäßen 21 aller Größen mit der Voraussetzung sich im Blutgefäß 21 bewegender Erythrozyten 36

erfolgen können.

Das Loch 2 am nach oben gerichteten, spitzen Pol 24 des Eies 1 kann mit einem Durchmesser bis zu 18 mm, vorzugsweise zwischen 8 mm und 12 mm mittels der Locherzeugungseinheit ausgebildet werden.

Als Licht 10a des sichtbaren Wellenlängenbereiches kann weißes und/oder grünes Licht aus der Lichtquelle 10 des Visions-Systems 10, 13 zur Überwachung des Zeitverlaufs der Vorgänge beim Bebrüten des Vogeleies 1 eingesetzt werden.

Das Verfahren mit den optischen Messungen zur in-ovo Geschlechtsbestimmung von befruchteten und bebrüteten Vogeleiern 1 kann ab dem dritten Bruttag durchgeführt werden.

Folgende Parameter können zum Beispiel für die Ramanspektroskopie gemäß Fig. 2 eingesetzt werden:

- Anregungswellenlängen des Laserlichts 3a der Laserstrahlungsquelle 3: > 600 nm (z.B. HeNe Laser 633 nm, Festkörperlaser (Nd basiert z.B. Nd:YAG Laser 1064 nm, VIS NIR Diodenlaser z.B. 785 nm),
- Einkopplung des Laserlichts 3a direkt mit Spiegeln und/oder mit optischen Fasern 4a,
- Ramanstreustrahlungsmessungen werden unter Nutzung einer optischen Vorrichtung 5 mit großer numerischer Apertur wie Mikroskop-Objektiven 61 oder einer Raman-Fasersonde 4b durchgeführt,
- Direkte Auskopplung der gesammelten Ramanstreustrahlung 7 mit Spiegeln zu einem Spektrometer 8 oder mittels Transports mit optischen Fasern 4b,
- Einsatz eines Spektrometers 8 in Form von dispersivem Ramanspektrometer und Fourier Transform Ramanspektrometer.

Während des Positionierungsvorgangs des Vogeleies 1 können permanent rückgeführte Ramanspektren aufgezeichnet und einer Auswertung zugeführt werden, wobei eine automatische Klassifizierung der Ramanstreustrahlung 7 anhand des spektralen Fingerabdruckes des Blutes erfolgt.

Folgende Parameter für die optischen Messungen der vom Blut durchflossenen Gefäß 21 rückgestreuten Fluoreszenzstrahlung können eingesetzt werden:

- Anregungswellenlängen der Laserstrahlung 3a der Laserstrahlenquelle (3): > 400 nm (z.B. HeNe Laser 633 nm, Festkörperlaser (Nd basiert z.B. Nd:YAG Laser 532 nm, VIS NIR Diodenlaser, z.B. 785 nm),
- Einkopplung der Laserstrahlung 3a direkt mit Spiegeln und/oder mit optischen Fasern 4a,
- Fluoreszenzstrahlungsmessungen werden unter Nutzung von optischen Vorrichtungen 5 wie Mikroskop-Objektiven 61 oder einer Fasersonde 4b durchgeführt,
- Direkte Auskopplung der gesammelten Fluoreszenzstrahlung 7 mittels mindestens eines Spiegels oder mittels Transports über optische Fasern,
- Einsatz mindestens eines Detektors 8.

Das Vogelei 1 wird pollastig gestellt und ein Loch 2 kann z.B. im Bereich des nach oben gerichteten spitzen Pols 24 bei einer Lochgröße von 10 mm ab drei Bruttagen eingebracht werden.

Der erste Schritt stellt das Perforieren der Kalkschale 28 durch einen Laser einer Locherzeugungseinheit 67 gemäß Fig. 1 dar. Die Bebrütungszeit, nachfolgend Inkubation genannt, des Vogeleies 1 beträgt zu diesem Zeitpunkt drei Tage. Im zweiten Schritt kann die im Vorfeld bearbeitete Kalkschale 28 geöffnet werden. Im dritten Schritt wird mit dem Raman-Spektroskop 8 ein Blutgefäß 21 analysiert und das Geschlecht festgestellt. Im letzten Schritt wird die im zweiten Schritt geöffnete Kalkschale 28 wieder verschlossen. Die Inkubation des Embryos 22 kann anschließend weiter fortgesetzt werden.

Der Blutkreislauf entsteht bereits in den ersten 36 bis 48 Stunden der Inkubation. Das Blut beginnt durch die Kontraktion des Herzens zu zirkulieren. In dieser Phase liegt ein Blutgefäßgeflecht 27 in einer Ebene vor.

Die Blutgefäße 21 besitzen zur Inkubationszeit von 72 bis 84 Stunden einen maximalen Durchmesser von circa 150 bis 350 $\mu$m.

Über dem Blutgefäßgeflecht 27 befindet sich eine Eiweißschicht 26, wie in den Fig. 3, 3a, 3b, 3c dargestellt ist. Demnach besteht ein Höhenunterschied zwischen der Eiweißschicht 26 und dem eingebetteten Blut durchflossenen Gefäß 21.

Der Anregungslaser 3 ist ein zentrales Element für die optischen Messungen, insbesondere bei der Raman-Spektroskopie. Damit der Laserfokus 30 des Anregungslasers 3 immer im Zentrum des Blutgefäßes 21 gehalten werden kann, wird jeweils die Sollposition mit der aktuellen Position abgeglichen.

Der Laserfokus 30 des Anregungslasers 3 und der Fokus 43 der Kamera 13 befinden sich in derselben Fokusebene, wie in den Fig. 3, Fig. 3c gezeigt ist. Dabei weist der in Fig. 3c dargestellte Laserfokus 30 eine Taille 66 auf, in deren engster Stelle sich in fester Konformität der Fokus 43 befindet.

Zur Inkubationszeit bis fünf Tage ist es möglich, durch eine vertikale Ausrichtung des Vogeleies 1, mit spitzem Pol 24 nach oben, Zugang zum Blutgefäßgeflecht 27 zu erhalten. Durch diese charakteristische Eigenschaft ist eine Analyse mit dem Raman-Spektroskop 8 möglich.

Im Folgenden wird ein Ausführungsbeispiel für den Aufbau des Raman-Messkopfes in einer erfindungsgemäßen Vorrichtung 40 zur Einstellung des Laserfokus 30 für die Ramanspektroskopischen Geschlechtsbestimmung beschrieben.

Der Aufbau des Raman-Messkopfes ist als Schema in Fig. 2 dargestellt und besteht aus mehreren Bestandteilen, deren Funktionen nachfolgend kurz erläutert werden.

Das zu untersuchende Vogelei 1 befindet sich auf einer Halterungseinrichtung 16, welche sich in einem begrenzten Raum in x-, y- und z-Richtung variieren lässt. Die variable Haltungseinrichtung 16 ist dabei notwendig, um die inneren Bewegungen des Vogeleies 1 auszugleichen. Zum Einsatz können z.B. drei Linear-Bühnen (nicht eingezeichnet) kommen, die jeweils von einem Schrittmotor angetrieben werden.

Die drei Linear-Bühnen sind an eine Motorsteuerung angeschlossen und können über die Auswerteeinheit 31 in Form eines Computers parametriert werden.

Im Allgemeinen ist die Beleuchtung 10a nur für den Einsatz der Kamera 13 erforderlich. Die Beleuchtung 10a muss bei einer Wellenlänge erfolgen, die das Messsignal nicht beeinflusst.

Wie in der Fig. 2 ersichtlich ist, wird ein Anregungslaser 3 mit einer emittierten Wellenlänge von 785 nm für eine Ramanspektroskopie genutzt und die vom Blutgefäß 21 bzw. Blutgefäßgeflecht 27 gestreuten Strahlen treffen auf den Strahlteiler 23 und werden unterhalb von 670 nm zur Kamera 13 durchgelassen. Alle Wellenlängen, die größer als 670 nm sind, werden zum Raman-Spektroskop 8 geleitet.

Des Weiteren besteht eine räumliche Trennung zwischen dem Messkopf 37, 23, 61 und dem Raman-Spektroskop 8, so dass die Lichtsignale über eine Faseroptik 4b gesendet werden können. Simultan zu den Lichtsignalen wird der Anregungslaser 3 über dieselbe Faseroptik 4a in den Messkopf 37, 23, 61 eingeführt, wobei ein Kollimator 37 über den Strahlteiler 23 vor dem Mess-Objektiv 61 eingebracht ist.

Das erste Mess-Objektiv 61 wird für die Auswahl der Messposition verwendet. Dazu ist eine hohe Vergrößerung des Bildbereiches von Vorteil, damit möglichst exakt in ein großes Blutgefäß 21 positioniert werden kann. Die lineare Vergrößerung ohne Strahlteiler kann z.B. 18-fach zwischen Blutgefäß 21 und KameraSensor 29 betragen.

Für die Realisierung wird das erste Mess-Objektiv 61 verwendet, das eine lineare Vergrößerung von 5 mit Strahlteiler zwischen Blutgefäß 21 und KameraSensor 29 aufweist.

Für die Aufnahme der Bilder kommt bei der Analyse die Kamera 13 zum Einsatz. Durch die in der Kamera 13 enthaltene CCD-Matrix 29 werden in der Analyse die Bilder ermittelt, die zur Bestimmung der Schärfeindikatoren notwendig sind. Die Bildrate von 20 fps ist für Analysezwecke ausreichend.

Eine höhere Bildrate erlaubt es, mehr Messpunkte pro Zeiteinheit zu erhalten. Dies ist insbesondere für die Ermittlung von Schärfeindikatoren von Vorteil. Gleichzeitig werden die Bilddaten auch für den x-,y-Horizontalausgleich genutzt.

In den Fig. 4 und Fig. 5 ist der Unterschied zwischen gesamter Bildfläche SAF$_1$ 65 und selektierter Bildfläche SAF$_2$ 39 und minimierter, reduzierter Bildfläche RBF (ROI) 41 angegeben.

Zur Reduzierung der Rechenzeit wird nur die ROI (engl. Region of Interest) 41 für diese Indikatoren genutzt. Die ROI 41 beschreibt in diesem Fall einen Ausschnitt, der sich nur im selektierten Blutgefäß 21 befindet.

Aufgrund von Rauschen der Schärfeindikatoren kann z.B. ein Savitzky-Golay Filter mit für alle Verfahren genutzt werden.

Einen wesentlichen Einfluss auf den Kontrast und damit auch auf das Gesamtergebnis hat die Beleuchtung 10a. Das grün-emittierte LED-Licht 10a wird besonders stark vom untersuchten Hämoglobin der Erythrozyten 36 absorbiert. Dadurch setzen sich die Erythrozyten 36 deutlich vom Dottersack ab.

Daraus lässt sich die Schlussfolgerung ziehen, dass ein grüner Kanal einer RGB-Kamera 13 die Erythrozyten 36 am besten sichtbar macht und gleichzeitig einen hohen Kontrast zum Dottersack liefert. Des Weiteren wird deutlich, dass weiße Beleuchtung mit einem grünen Anteil ausreichend ist, um die roten Blutkörper 36 sichtbar zu machen. Einen noch besseren Kontrast erzielt man bei Verwendung von grünem LED-Licht.

Die reduzierte Bildfläche RFB 41 kann in ein Gitter von Pixeln (x, y) eingeteilt werden. Die Intensität des Grauwertes wird durch die Größe I(x,y) von einem bestimmten Pixel (x,y) beschrieben.

Als Beispiel zur Durchführung eines kontrastbasierten Verfahren wird das bekannte Verfahren "Variance VAR" unter Verwendung der Größe I(x,y) mit der Formel benutzt:

$$S_{VAR} = \frac{1}{H\ddot{o}he \cdot Breite} \sum_{(x,y) \in I} \sum_{(x,y) \in I} (I(x,y) - \mu)^2 .$$

Der Mittelwert der Intensitäten I(x,y) entspricht der Variablen $\mu$ (Mittelwert) und die "Höhe" und "Breite" beziehen sich auf Größe des 2D-Bildes.

Das "Differenzbild" (Dif)-Verfahren stellt den Unterschied zwischen von Intensitäten $I_A(x,y)$ und $I_B(x,y)$ zweier Bildern dar und der Unterschied $U_{Dif}$ wird durch die Formel

$$U_{Dif}(x,y) = |I_A(x,y) - I_B(x,y)| .$$

wobei A und B zeitbezogen sind,
beschrieben.

Im speziellen Fall des Hühnerembryos 22 wird die Eigenschaft der Bewegung von Erythrozyten 36 im Blutgefäß 21 genutzt, um einen Unterschied in der Bildmatrix zu erhalten. $I_A(x,y)$ und $I_B(x,y)$ beschreiben dabei zwei zeitlich direkt aufeinander folgende aufgenommene Bilder. Damit die Differenz aufgrund von Rauschen vermieden wird, werden alle $U_{Dif}(x,y)$, die kleiner sind als ein vorgegebener Schwellwert $SW$, nicht berücksichtigt, damit gilt für ein Binärbild b(x,y)

$$b(x,y) = \begin{cases} 1 \ wenn \ U_{Dif}(x,y) \geq SW \\ 0 \ sonstige \end{cases} .$$

,

wobei SW einen vorgegebenen Schwellwert darstellt.

Ein Tiefpassfilter $TP(b(x,y))$ sorgt dafür, dass einzelne Pixel, die sich stark von der Umgebung unterscheiden, der Umgebung angepasst werden. Der resultierende Schärfewert $S_{Dif}$ des Differenzbildes ermittelt sich aus

$$S_{Dif} = \sum_{(x,y) \in I} TP(b(x,y)) .$$

Bei allen kontrastbasierten Schärfeindikatoren wird die volle Größe der Flächen $SAF_1$ 65 der Bilder verwendet.

Beim erythrozytbewegungsbasierten Differenzbild-Verfahren ist jedoch nur die Bewegung der roten Blutkörper (Erythrozyten) 36 innerhalb des Blutgefäßes 21, wie in Fig. 5 gezeigt ist, von Interesse, da der statische Dottersack keine Intensitätsänderung in zwei aufeinander folgenden Bildern bewirkt. Die Intensitätsänderung wird durch die Erythrozyten 36 erzeugt, welche in zwei aufeinanderfolgenden Bildern eine Änderung generieren. Zudem existiert, wie bereits beschrieben, innerhalb des Eies 1 ein Höhenunterschied zwischen Blutgefäß 21 und Eiweißschicht 26. Daraus resultierend befindet sich die ROI 41 direkt im Blutgefäß 21.

Das Maximum des Schärfewertes $S_{Max}$ soll sich im Idealfall im Zentrum des Blutgefäßes 21 befinden. In diesem Mittelpunkt ist davon auszugehen, dass das Raman-Signal des Blutes den höchsten Wert liefert.

Das erythrozytbewegungsbasierte Differenzbild-Verfahren kann den Laserfokus 30 im Blut durchflossenen Gefäß 21 am sichersten ermitteln. Die Kontrastverfahren unterliegen jedoch starken Schwankungen und das Maximum kann sowohl in der überlagerten Eiweißschicht 26 als auch anderen Ebenen liegen.

Das VAR-kontrastbasierte Verfahren bildet bei der Nutzung des gesamten Bildbereiches der Analysepositionsfläche SAF$_1$ 65 die geringste Standardabweichung.

Bei den kontrastbasierten Verfahren spielt die Bewegung der Erythrozyten 36 und damit die Veränderung der Grauwerte der Intensitäten keine Rolle.

Die Rechenzeit ist eine wichtige Größe, insbesondere bei erhöhter Bildgröße oder Bildfrequenz. Das kontrastbasierte Verfahren VAR ist ein sehr schneller Algorithmus, der auch für sehr hohe Bildraten genutzt wird.

Des Weiteren kann das erythrozytbewegungsbasierte Differenzbild-Verfahren aufgrund der Nutzung seiner eingeschränkten Bildgröße eine sehr kurze Rechenzeit erreichen.

Die kamerabasierte Vorrichtung 5 in Fig. 2 für die Selektierung und Positionierung eines Blutgefäßes 21 benötigt den Einsatz von Bildverarbeitungsalgorithmen. Im weiteren Verlauf werden diese detailliert formal und mathematisch beschrieben.

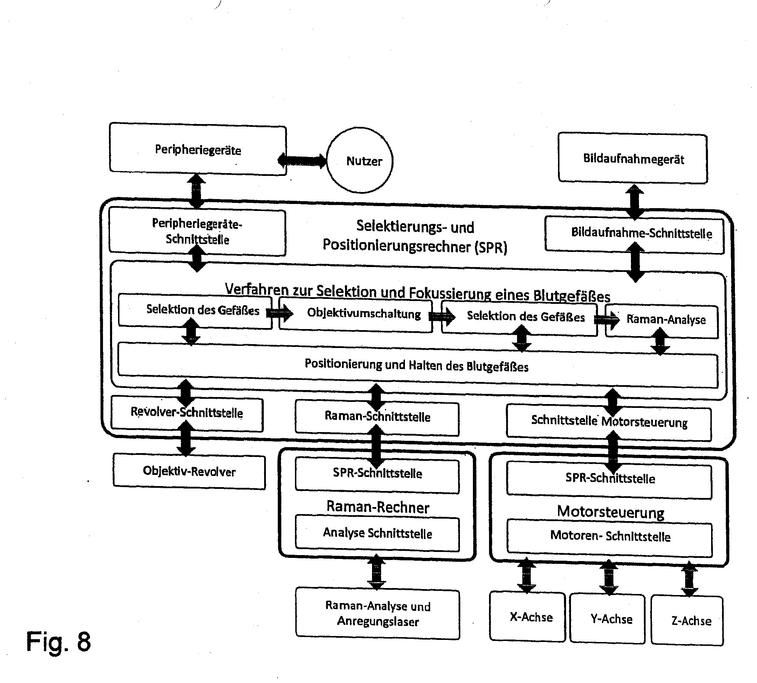

Das Herzstück der Bildverarbeitungsaufgabe stellt gemäß Fig. 2 und Fig. 8 der Selektierungs- und Positionierungsrechner (SPR) 31 dar. Neben der Bildverarbeitung finden hierüber auch die Steuerung der Schrittmotoren der koordinativen Positioniereinheit 18 und die automatische Umschaltung der Mess-Objektive 61, 62 z.B. über einen Revolver 44 oder gemäß Fig. 2a die automatische Umschaltung der Objektive 38, 63 der Kamera 13 z.B. über einen anderen Revolver 64 statt.

Zudem ist es dem Nutzer möglich, über Peripherien mit dem SPR 31 zu interagieren. Die Bilddaten selbst werden durch die RGB-Kamera 13 erzeugt. Mit Hilfe der Motorsteuerung können die Schrittmotoren parametriert werden und die X-, Y- und Z-Achse über eine Spindel antreiben. Der Raman-Rechner 9 in Fig. 2 ist für die Aufnahme des Spektrums verantwortlich, woraus das Geschlecht bestimmt werden kann.

Sowohl der Raman-Rechner 9 als auch der SPR 31 können auch in einem einzigen Rechner 45 vereinigt enthalten sein.

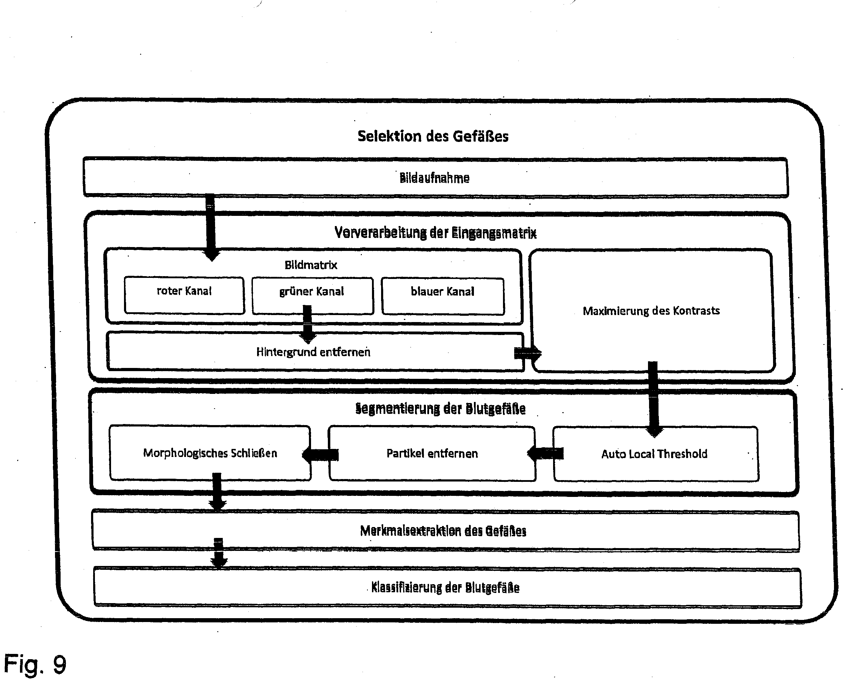

Zu Beginn der Analyse ist die Aufnahme eines Bildes erforderlich. Im weiteren Schritt werden die Vorverarbeitung und die anschließende Segmentierung durchgeführt. Diese Schritte sind notwendig, um das gesamte Bild der Analysepositionsbildfläche SAF$_1$ 65 in Objektbereiche und Hintergrund zu trennen. In Fig. 9 sind die Teilschritte für die Vorverarbeitung und Segmentierung als Bestandteile für die "Selektion eines Gefäßes" dargestellt.

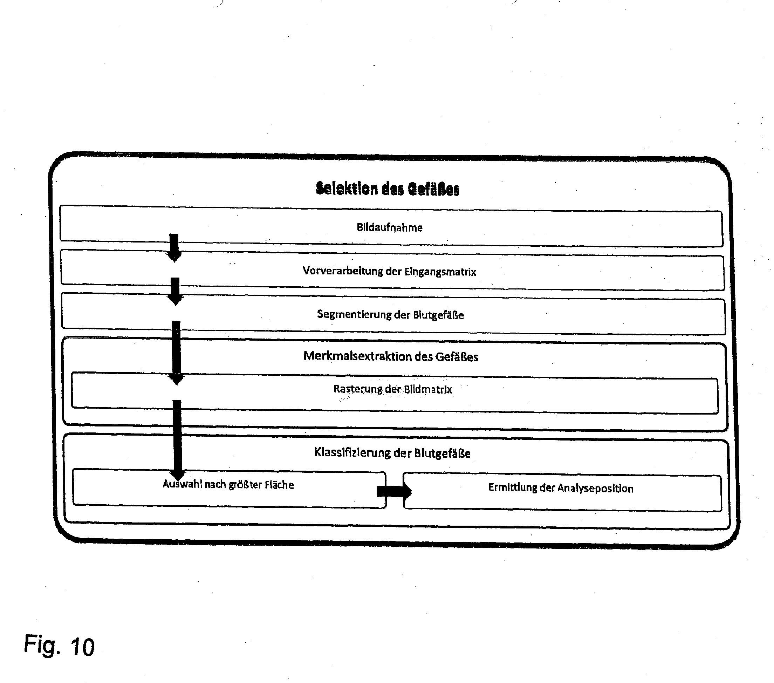

Die ausgeklappten Blöcke in der Grafik veranschaulichen die Schritte, die in diesem Abschnitt detailliert betrachtet werden. Des Weiteren wird kurz beschrieben, welche Punkte bei der Aufnahme des Bildes beachtet werden. Die Merkmalsextraktion und Klassifizierung der Blutgefäße 21 gemäß Fig. 10 sind Bestandteil weiterer Schritte. Im "Verfahren zur Selektion und Fokussierung eines Blutgefäßes" gemäß Fig. 8 ist die "Selektion des Gefäßes" zweifach vorhanden. Das Verfahren wird als erstes genutzt, um ein Blutgefäß 21 aus dem gesamten Blutgefäßgeflecht 27 zu selektieren. Im zweiten Schritt kann das Verfahren genutzt werden, um die Position im Blutgefäß 21 erneut zu bestimmen. Dies ist notwendig, falls durch die Objektivumschaltung von Objektiv 61 zu Objektiv 62 in der Kamera 13 die Position des Laserfokus 30 verloren gegangen ist. Nachfolgend wird der erste Aufruf der Funktion "Selektion des Gefäßes" beschrieben.

Zunächst ist es jedoch notwendig, dass das gesamte Blutgefäßgeflecht 27 gemäß Fig. 4 auf dem Kamerasensor 29 dargestellt wird. Dazu ist ein Objektiv 61 zu wählen, das das gesamte Blutgefäßgeflecht 27 einschließlich des Embryos 22 erfassen kann. Damit ist garantiert, dass alle Gefäße 21, die als Analysepunkt dienen könnten, sichtbar und damit auswertbar sind. In Fig. 4 ist solch ein Blutgefäßgefecht 27 mit Embryo 22 dargestellt.

Der Vergrößerungsfaktor des ersten Objektivs 61 der Kamera 13 hat großen Einfluss darauf, ob die Schale 28 des Eies 1 sichtbar ist. Deshalb sollte die Vergrößerung des Objektivs auf den Durchmesser der gewünschten Laserperforation adaptiert werden.

Damit eine Segmentierung des Blutgefäßgeflechtes 27 durchgeführt werden kann, ist es von Vorteil, einen homogenen Hintergrund zu besitzen. Als Hintergrund sind alle Bestandteile zu verstehen, welche nicht zum Blutgefäßgeflecht 27 selbst gehören. Dazu gehören vor allem der Dottersack, das Herz und der Embryo selbst.

Der letzte Schritt der Vorverarbeitung dient zur Maximierung des Kontrasts und stellt ein Standardverfahren dar. Dabei wird die Grauwertfunktion des Histogramms stückweise linear transformiert. Diese Skalierung lässt sich beschreiben durch

$$T(g_{RB}) = \begin{cases} 0, & \text{falls } (g_{RB} + a_t)b_t < 0 \\ (g_{RB} + a_t)b_t, & \text{falls } 0 \le (g_{RB} + a_t)b_t \le 255 \\ 255, & \text{falls } (g_{RB} + a_t)b_t > 255 \end{cases}.$$

zur linearen Transformation des Histogramms.

Die Parameter $a_t$ und $b_t$ für die lineare Transformation ermitteln sich aus der Ausgangsfunktion nach einem Rolling-Ball-Algorithmus $g_{RB}$. Daraus ergeben sich die Parameter

$$a_t = -\min(g_{RB})$$

und

$$b_t = \frac{255}{\max(g_{RB}) - \min(g_{RB})}$$

mit jeweils dem größten und kleinsten vorkommenden Grauwert im Ursprungsbild. Dadurch entsteht durch Einsetzen der Formeln eine Treppenfunktion, woraus eine Spreizung des Grauwertbereiches von $\min(g_{RB})$ auf 0 und von $\max(g_{RB})$ auf 255 entsteht.

Nachdem die Vorverarbeitung abgeschlossen ist, kann gemäß Fig. 10 die Segmentierung durchgeführt werden. Darunter versteht man das Trennen von Hintergrund und Blutgefäß 21 als Zielobjekt in binäre Werte.

Wird diese Logik auf ein Grauwertbild g(x,y) bezogen, so entspricht die segmentierte Bildmatrix

$$o(x,y) = \begin{cases} 0, & falls\ g(x,y) \le SW(x,y) \\ 255, & sonstige \end{cases} .$$

zur Segmentierung eines Grauwertbildes, wobei SW den Schwellwert darstellt. Das Ziel bei einem Auto Local Threshold gemäß Fig. 9 ist es, einen Schwellwert $SW(x,y)$ für jeden Pixel $(x,y)$ zu bestimmen. In dem in der Druckschrift von Sauvola J., Pietikanen, Adaptive document image binarization, Pergamon Vol. 33 (2000), beschriebene Verfahren wird dafür der Mittelwert $\mu(x,y)$ und die Standardabweichung $\sigma(x,y)$ innerhalb einer Fenstergröße verwendet. Das Fenster besitzt eine Größe von $h_m \cdot x \cdot v_m$ Pixel und ist um das Zentrum des zu analysierenden Pixels aufgespannt, wobei $h_m$ die Breite und $v_m$ die Höhe sind. Damit ergibt sich die Berechnung des Schwellwertes aus

$$SW(x,y) = \mu(x,y)\left[1 + q\left(\frac{\sigma(x,y)}{R} - 1\right)\right].$$

Dabei stellen $R$ den Maximalwert der Standardabweichung und q einen Faktor dar.

Mit der Segmentierung können nahezu alle definierten Hintergründe entfernt werden. Gleichzeitig sind die größten Strukturen der Gefäße 21 eindeutig lokalisierbar. Gefäße, die klein sind oder sehr tief liegen, werden teilweise entfernt. Für die Analyse ist jedoch nur das Blutgefäß 21 mit dem größten Querschnitt gemäß Fig. 5 entscheidend.

Damit gemäß Fig. 9 kleine Partikel, die nicht zum Gefäß 21 gehören, entfernt werden, ist es möglich, diese mittels einer Erosion zu beseitigen. Im Vorfeld wird dafür das gesamte Bild invertiert, da die meisten Algorithmen und Quellen weiße Objekte als Zielobjekt und schwarze Objekte als Hintergrund definieren. Die Erosion kann in Abhängigkeit von der Gefäßgröße beliebig oft wiederholt werden. Die Strukturen, die nach der Erosion erhalten bleiben, sind exakt die gleichen wie im Ursprungsbild. Es wird demnach keine Veränderung der Gefäße vorgenommen, sondern ausschließlich Partikel entfernt.

Strukturen, die aufgrund der Segmentierung nicht vollständig gefüllt wurden, sollen mit dem Schritt: Morphologisches Schließen gemäß Fig. 9 wieder eine homogene Fläche bilden. Dadurch werden alle Pixel, die zum Hintergrund gehören, durch Vordergrund ersetzt, wenn diese vollständig von Vordergrund umschlossen sind.

Nachdem die Segmentierung der Blutgefäße 27 erfolgreich abschlossen ist, gilt es, das Blutgefäß 21 zu extrahieren und die Analyseposition für die Raman-Spektroskopie gemäß Fig. 9 zu ermitteln.

Im Folgenden wird ein Beispiel der Ermittlung eine Analyseposition mit einer Block-Klassifizierung angegeben: Das segmentierte Bild wird durch eine Rasterung geteilt. Anschließend wird jeder einzelne Block nach Blockfläche klassifiziert. Von der größten Fläche kann nachfolgend die Position für eine Analyse bestimmt werden.

Damit ein Blutgefäß 21 mit hohem Durchmesser innerhalb der großen Bildfläche ermittelt werden kann, wird zunächst das notwendige Merkmal extrahiert. Wenn die Annahme getroffen wird, dass die vorkommenden Blutgefäße die größten Strukturen sind, ergeben diese Strukturen auch die größte Fläche. Aus dieser Annahme heraus können die segmentierte Bildmatrix gerastert werden und für jeden Block kann der Flächeninhalt des Gefäßes 21 bestimmt werden.

Die Anzahl der Blöcke ist abhängig von der Bildgröße $h_m$ x $v_m$ des Kamerasensors 29 und der Blockgröße B. Damit immer die gleiche Größe der Blöcke abgebildet wird, werden die Bedingungen

$$K_1 = \frac{h_m}{B} \text{ , } K_1 \in \mathbb{N}^*$$

zur Berechnung der Anzahl der horizontalen Blöcke
und

$$K_2 = \frac{v_m}{B} \text{ , } K_2 \in \mathbb{N}^*$$

zur Berechnung der Anzahl der vertikalen Blöcke
erfüllt. Falls die größte Struktur zwischen zwei Blöcken liegt, kann diese nicht sicher ermittelt werden. Deshalb muss ein zweites Raster erstellt werden, welches in x- und y- Richtung um $B/2$ verschoben ist und $K_1$ - 1 sowie $K_2$ - 1
Blöcke besitzt. Damit Objekte, die nicht zum Gefäßgeflecht 27 gehören, ausgeschlossen werden, sollte $B$ ausreichend groß gewählt werden. In der Klassifizierung kann so vermieden werden, dass Teile des Herzens eingeschlossen werden. Die Fläche von mehreren Gefäßen ist damit größer als die Gesamtfläche des Herzens für eine ausreichend große Blockgröße.
Zur Berechnung und Auswahl der größten Fläche wird iterativ von allen Blöcken der Flächeninhalt $A_{ij}$ der Objektpixel $g_{ij}(x,y)$ bestimmt. Dieser Zusammenhang lässt sich beschreiben durch

$$A_{ij}\left(g_{ij}\right) = \sum_{x=1}^{B} \sum_{y=1}^{B} g_{ij}(x,y)$$

zur Berechnung der Blockfläche segmentierter Objektpixel und

$$A_{max} = max\left[\sum_{i=1}^{K_1} \sum_{j=1}^{K_2} A_{ij}\right].$$

 zur Ermittlung der maximalen Fläche segmentierter Objektpixel.
Der Block mit der größten resultierenden Fläche wird verwendet, um die Analyseposition zu bestimmen. Die Laufvariablen i und j beschreiben die Position des Blocks in der Bildmatrix.
Mit der Bestimmung des geometrischen Schwerpunktes erfolgt eine Ermittlung der Analyseposition. Dieser Punkt kann in x-Richtung ermittelt werden durch

$$x_c = \frac{\sum_{x=1}^{B}\sum_{y=1}^{B} x \cdot g_{ij}(x,y)}{A_{max}(g_{ij})}$$

zur Ermittlung des geometrischen Schwerpunktes in x-Richtung
und in y-Richtung durch

$$y_c = \frac{\sum_{x=1}^{B}\sum_{y=1}^{B} y \cdot g_{ij}(x,y)}{A_{max}(g_{ij})} .$$

zur Ermittlung des geometrischen Schwerpunktes in y-Richtung.
Der ermittelte Punkt liegt gemäß Fig. 5 im Zentrum des breitesten Blutgefäßes 21 und ist deshalb für die Raman- Spektroskopie gut geeignet.
Nachfolgend werden Bestandteile der Objektivumschaltung von einem ersten Objektiv 61 zum zweiten Objektiv 62 mit kürzerer Brennweite und jeweils wieder zurück und der Raman-Analyse erläutert:

Die Umschaltung des Raman-Objektivs 61 ist zweckmäßig, da für das Selektieren des zu analysierenden Blutgefäßes 21 eine möglichst große Auswahl an Strukturen vorliegen sollte. Im Gegensatz dazu kommt es bei der Raman-Analyse selbst auf eine genaue Positionierung des Laserfokus 30 in dem Blutgefäß 21 an. Wird nur ein Objektiv 61 oder 38 verwendet, muss für die Auswahl des Blutgefäßes 21 ein Bereich gerastert und schrittweise abgesucht werden. Innerhalb dieses Vorgangs kann sich jedoch die horizontale Position des Gefäßes 21 verändern, wodurch eine erneute Suche des zuvor bestimmten Blutgefäßes 21 notwendig ist, was sehr zeitaufwändig ist.

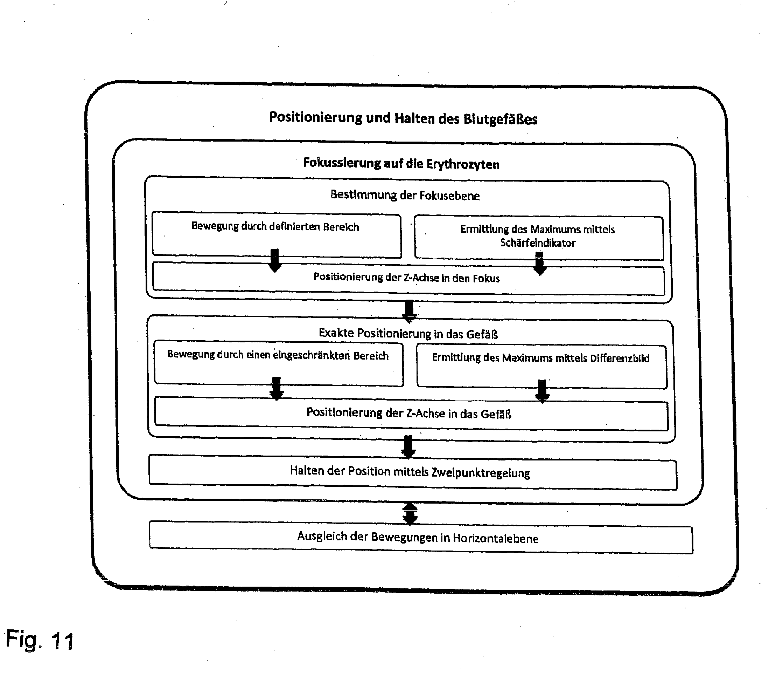

Das Positionieren und Halten des Blutgefäßes 21 im Laserfokus 30 des Anregungsstrahls ist Bestandteil beim Selektieren des Gefäßes 21 und der Raman-Analyse selbst. Im Idealfall hat die Umschaltung der Objektive 61 zu 62 keinen Einfluss, sodass die Bestimmung der Fokusebene gemäß Fig. 11 beim Selektieren des Gefäßes 21 einmalig ausgeführt wird. Nach der Umschaltung zum Raman-Objektiv 62 liegt der Fokus 30 exakt in derselben Ebene wie mit dem Objektiv 61 zum Selektieren des Gefäßes, sodass direkt die exakte Positionierung des Laserfokus 30 in das Gefäß 21 stattfinden kann. Im Anschluss wird der Laserfokus 30 während der Raman-Analyse gehalten. Findet ein starker Abfall während der Selektierung und Umschaltung der Objektive von 61 auf 62 statt, so muss von vorn mit der Bestimmung der Fokusebene begonnen werden. Eine Dekomposition für diese Positionierung in den Fokus, ist in Fig. 11 mit einer Dekomposition der Fokussierung auf die Erythrozyten 36 dargestellt.

Den ersten Schritt gemäß Fig. 11 stellt die Bestimmung der Fokusebene mittels Kontrastverfahren dar. Anschließend wird die Z-Achse in die ermittelte Ebene gefahren. Im zweiten Teil wird die Z-Achse durch Anwendung des Differenzbild-Verfahrens in der Auswerteeinheit 31 exakt in das Blutgefäß 21 positioniert. Im letzten Schritt wird dieser Punkt durch eine Zweipunktregelung der koordinativen x-,y-,z-Positioniereinheit 18 im Fokus gehalten.

Damit die schärfste Ebene innerhalb des Blutgefäßes 21 ermittelt werden kann, wird gemäß Fig. 13 ein definierter Bereich längs der z-Achse abgefahren. Innerhalb dieses definierten Bereiches $Z_{max}$ und $Z_{min}$ liegt der Punkt $Z_{fokus}$. Der Wert $Z_{max}$ stellt den minimalen Abstand von Objektiv zum Blutgefäß 21 dar und bildet den Startpunkt für die Analyse. Der Parameter $Z_{min}$ hingegen ergibt sich aus der tiefsten Ebene des Blutgefäßgeflechtes 27, welches innerhalb des Blutgefäßes 21 erreicht werden kann.

Parallel zur Durchfahrt des Bereiches zwischen $Z_{max}$ und $Z_{min}$ wird der Schärfeindikator durch die Kamera 13 ermittelt.

Damit Störeinflüsse durch Rauschen minimiert werden, wird der Verlauf des Schärfeindikators durch einen Savitzky-Golay-Filter geglättet. Der geglättete Wert $g_{sg}$ ergibt sich dabei aus dem Messpunkt $f_i$ mit einer gewichteten Mittelung über die Nachbarwerte $n_R$ bis $-n_L$.

Dabei stellt $c_l$ einen geeigneten Koeffizientensatz für den Savitzky-Golay-Filter dar. Es werden vergangene und zukünftige Werte in den Glättungsalgorithmus mit einbezogen. Insgesamt bietet das Filter gute Eigenschaften in der Darstellung von Maxima, wie sie bei der Ermittlung der Variance-Fokuswerte vorkommen, da auch höher frequentierte Signale mit in den Verlauf einbezogen werden.

Werden die Schärfewerte bei der Durchfahrt durch den Bereich von $Z_{max}$ zu $Z_{min}$ aufgetragen, entsteht der Graph gemäß Fig. 13 als Verlauf des Schärfeindikators VAR und VAR' in Abhängigkeit von der Distanz, der ein Maximum bei $Z_{fokus}$ ausbildet.

Gleichzeitig ist aus Fig. 13 ersichtlich, dass der Fokuswert $Z_{fokus}$ mit höherer Distanz sinkt und mit geringerer Distanz steigt. Eine mögliche Ursache für dieses Verhalten ist der Einfluss der Beleuchtung 10a auf die Kalkschale 28 des Eies 1. Der steigende Wert Z verursacht eine Verdunklung des Gefäßgeflechtes 27, da der Kegel partiell von der Kalkschale 28 verdeckt wird und somit nicht auf die Gefäße treffen kann. Wohingegen eine Verringerung der Z-Achse zu einer größeren Beleuchtungsfläche führt und über die Kalkschale 28 zurück reflektiert wird. Diese Steigung kann dazu führen, dass das globale Maximum größer ist als das lokale Maximum bei $Z_{fokus}$. Deshalb ist es zweckmäßig, den Bereich für das lokale Maximum $Z_{fokus}$ einzuschränken. Eine geeignete Charakteristik ist der starke Anstieg und Abfall der Gauß-Form. Werden von der Funktion VAR die erste Ableitung, wie in Fig. 13 gezeigt ist, gebildet, so ergibt sich ein globales Maximum und Minimum, die als Eingrenzung für den Bereich genutzt werden können. Die Positionierung der Z-Achse erfolgt über eine absolute Bewegung zum ermittelten Punkt $Z_{fokus}$.

Die exakte Positionierung in das Blut durchflossene Gefäß 21 mittels erythrozytebewegungsbasierten Differenzbild-Verfahrens gemäß Fig. 11 ist notwendig, da das beispielsweise eingesetzte Kontrastverfahren "Variance" den größten Fokuswert für das gesamte Bild der Analysepositionsbildfläche $SAF_1$ 65 herstellt, jedoch nicht den Fokus 30 in das Gefäß 21 selbst. Die Ermittlung des Punktes $Z_{fokus\_exakt}$ funktioniert prinzipiell ähnlich den Verfahrensschritten "Bestimmung der Fokusebene". Der Bereich, der abgefahren wird, ermittelt sich aus dem bekannten Punkt $Z_{fokus}$ und der erwarteten Änderung $\Delta Z_{max}$ und $\Delta Z_{min}$.

Diese Änderungen entstehen durch das Absenken des Embryos 22 innerhalb des Eies 1 und der unpräzisen Fokussierung auf das Blutgefäß 21, bedingt durch das Kontrastverfahren. Die Größen $Z_{max\_exakt}$ und $Z_{min\_exakt}$ werden beschrieben durch

$$Z_{\mathrm{max\_exakt}} = Z_{fokus} + \Delta Z_{max}$$

zur Bestimmung der exakten Maximalposition für den Durchfahrbereich und

$$Z_{\mathrm{min\_exakt}} = Z_{fokus} - \Delta Z_{min} \cdot$$

zur Bestimmung der exakten Minimalposition für den Durchfahrbereich.

Des Weiteren ist zu beachten, dass die Geschwindigkeit $v_z \gg v_{z\_exakt}$ ist.

Für die Glättung des Schärfewertes vom erythrozytebewegungsbasierten Differenzbild-Verfahren wird ein kausaler gleitender Mittelwert verwendet. Grund dafür ist die Pulsation des Flusses der Erythrozyten 36 in den Blutgefäßen 21, welche unerwünscht ist. Der gleitende Mittelwert gm(k) kann dieses Verhalten ausgleichen und wird bestimmt durch

$$g_{gm}(k) = \frac{1}{w} \sum_{i=0}^{w-1} S(k-i) \cdot$$

Der Schärfewert S an der Position k beschreibt den aktuellen Zeitpunkt. Die Variable i beschreibt die zurückliegenden Werte. Die Fensterbreite, die über den der Mittelwert berechnet werden soll, wird durch die Variable w zum Ausdruck gebracht.

Eine Bestimmung der Ableitung vom Differenzwert ist nicht notwendig, da das in Fig. 6 gezeigte, ermittelte Maximum 46 eindeutig ist. Zum anderen wird gemäß Fig. 14 vom ermittelten Maximum $Z_{fokus\_exakt}$ ein Versatz benötigt, damit gewährleistet wird, dass ein Zweipunktregler immer hinterhereilt. Der Punkt $Z_{versatz}$ ermittelt sich aus

$$Z_{versatz} = Z_{fokus\_exakt} - \Delta Z_{versatz} \cdot$$

zur Bestimmung der Z-Position mit Versatz.

In der Fig. 14 sind die wichtigsten Parameter veranschaulicht.

In der Grafik der Fig. 14 ist eine Kurve "DifR" ersichtlich, die zum Zeitpunkt t=0 mit dem erythrozytbewegungsbasierten Differenzbild-Verfahren aufgenommen wurde. Mittels einer Absolutbewegung wird der Ausgangspunkt $Z_{versatz}$ für die Regelung angefahren. Aufgrund eines Absenkens des Embryos 22 verschiebt sich die Kurve "DifR -t" mit größer werdender Zeit t.

Um das Verhalten des Absenkens auszugleichen, ist eine Vergrößerung des Z-Wertes mittels Regler - z.B. eines Zweipunktreglers - in der Positioniereinheit 18 erforderlich. Die obere Schaltschwelle und die untere Schaltschwelle des Zweipunktreglers mit Hysterese-Verhalten ermittelt sich aus

$$w_{\varepsilon+}(t) = DifR_{\varepsilon+} = DifR_{max} \cdot p_{\varepsilon+} \quad mit\, p_{\varepsilon+}[0,1]\, und\, p_{\varepsilon+} > p_{\varepsilon-}$$

zur Bestimmung der oberen Schaltschwelle für die Zweipunktreglung und

$$w_{\varepsilon-}(t) = DifR_{\varepsilon-} = DifR_{max} \cdot p_{\varepsilon-} \quad mit\, p_{\varepsilon-}[0,1]\, und\, p_{\varepsilon+} > p_{\varepsilon-} \cdot$$

zur Bestimmung der oberen Schaltschwelle für die Zweipunktreglung,

wobei $p_{\varepsilon+}$ und $p_{\varepsilon-}$ den prozentualen Wert zur Ermittlung der Schaltschwelle darstellen.

Zu Beginn entsteht eine Totzeit $t_{tot}$, die auf den Abfall des Embryos 22 zurückzuführen ist. Der Regler versucht diesen Zustand auszugleichen, indem die Stellgröße $u_{ein}(t)$ zugeschaltet wird. Dadurch beginnt der Schrittmotor mit konstanter Geschwindigkeit $v_z$ die Z-Achse anzutreiben und die Variable Z zu vergrößern. Dieser Zustand wird beibehalten bis die Schaltschwelle $w_{\varepsilon+}(t)$ erreicht wird. Folglich wechselt die Stellgröße zu $u_{aus}(t)$ und der Schrittmotor stoppt. Wird $w_{\varepsilon+}(t)$ nochmals passiert, bleibt der Motor ausgeschaltet. Erst beim Unterschreiten der Schaltschwelle $w_{\varepsilon-}(t)$ beginnt der Motor erneut die Variable Z zu vergrößern, bis $w_{\varepsilon+}(t)$ überschritten ist. Die Regelung stoppt, sobald die Raman-Analyse erfolgreich abgeschlossen ist.

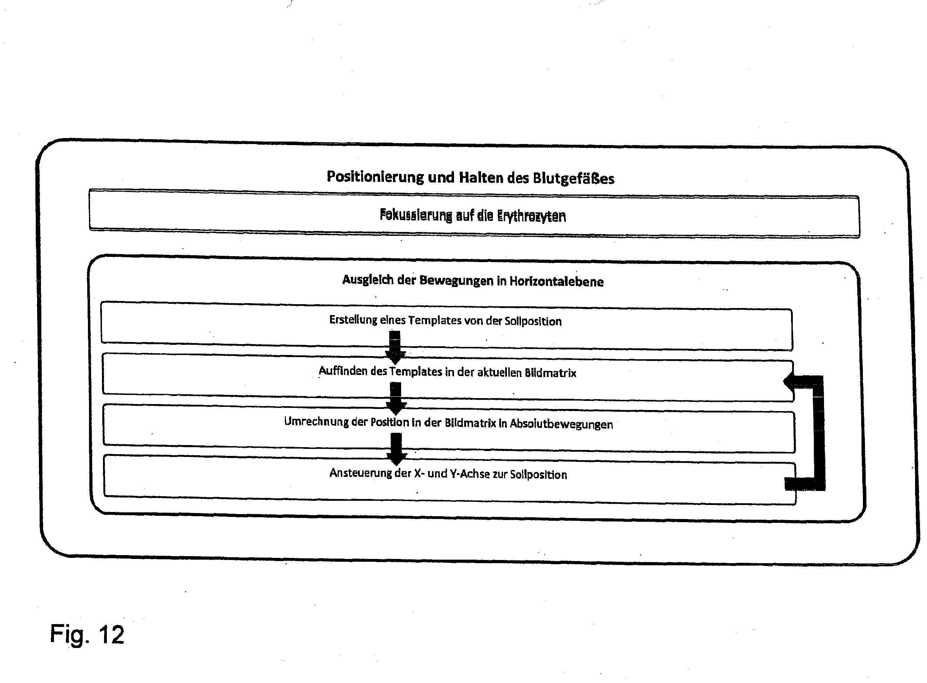

Der Ausgleich der Bewegung in x-, y-Horizontalebene gemäß dem Unterschritt 8.3 ist immer aktiv, wenn der Laserfokus 30 in der z-Achse hergestellt ist. Die notwendigen Schritte als Beispiel für den Ausgleich mittels "Template Matching" sind in Fig. 12 dargestellt.

Der erste Schritt ist das Erstellen eines Templates in der horizontalen Sollposition mit hergestelltem Laserfokus 30 in der Z-Ebene. Im Anschluss wird versucht, dieses Template erneut in der aktuellen Bildmatrix aufzufinden. Korreliert ein Ausschnitt der aktuellen Bildmatrix mit dem erzeugten Template, wird das Zentrum des "Match" ermittelt. Die Differenz zwischen Match und Sollposition kann nun durch einen Maßstab in Absolutbewegungen umgerechnet werden. Die Absolutbewegungen werden folglich von den Schrittmotoren in X- und Y-Richtung ausgeführt.

Das Template t(i,j) stellt eine Grauwertmatrix dar, welche eine Breite $h_m$ und eine Höhe $v_m$ besitzt. Der Ursprung des Templates selbst liegt im Originalbild. Es stellt einen Ausschnitt dar, indem sich das Gefäß 21 zentral als Zielobjekt befindet.

Nachdem das Template erfolgreich auf der aktuellen Bildmatrix ermittelt wurde, wird daraus das Zentrum z(x,y) mit

$$z(x,y) = g\left( x + \left( \frac{h_m - 1}{2} \right), y + \left( \frac{v_m - 1}{2} \right) \right)$$

für das Zentrum des Templates auf der aktuellen Bildmatrix bestimmt. Die Sollposition $p_s(x,y)$ kann frei auf der Bildmatrix gewählt werden, wenn garantiert ist, dass das Template vollständig auf der Bildmatrix abgebildet werden kann. Den Betrag der Differenz zwischen der Istposition z(x,y) und der Sollposition $p_s(x,y)$ ergeben die notwendige Änderung $\Delta s_{px}$ und $\Delta s_{py}$.

Die verwendeten Schrittmotoren für die X- und Y-Achse besitzen dieselbe Fahrdynamik wie die Z-Achse. Die Motoren in der X-Y-Ebene besitzen die Aufgabe die reale Streckendifferenz $s_{rx}$ und $s_{ry}$ je nach Situation auszugleichen. Die parametrierten Geschwindigkeiten $v_x$ und $v_y$ sollten nur maximal so groß gewählt werden, dass keine erheblichen Änderungen innerhalb des Differenzbildes durch die Bewegung der Schrittmotoren entsteht.

Die Analyse des Geschlechtes wird in dem Blut durchflossenen Gefäß 21 durchgeführt. Ausgehend von der maximalen Erythrozytengröße entsteht ein maximaler Grad an Änderung bis zu einer Bildrate von circa 70 fps. Damit diese Erythrozyten 36 scharf abgebildet werden können, sollte die Änderung auf der Kamera 13 weniger als 1 Pixel betragen, da sonst Bewegungsunschärfe durch den Blutfluss entsteht.

Folgende zusammengefasste Vorteile der Erfindung sind vorhanden:

Das gewählte erythrozytebewegungsbasierten Differenzbild-Verfahren zeichnet sich dadurch aus, dass es im Gegensatz zu kontrastbasierten Verfahren möglich ist, mit einer geringen Standardabweichung den Schärfewert 46 direkt im Blutgefäß 21 zu ermitteln. Folglich ist nur ein kleiner Bildausschnitt RBF 41 in der selektierten Analysepositionsbildfläche $SAF_2$ 39 erforderlich, wodurch die Rechenzeit erheblich geringer ausfällt als bei anderen Verfahren mit voller Bildgröße gemäß Fig. 4.

Damit eine optimale Selektion des Blutgefäßes 21 stattfinden kann, ist die Wahl einer anderen Vergrößerung zu einem Objektiv 62 gemäß Fig. 2 oder Objektiv 63 gemäß Fig. 2a zweckmäßig.

Der erste Schritt zur Fokussierung wird durch Nutzung beispielsweise des kontrastbasierten Verfahrens "Variance" realisiert, welches unabhängig von Blutgefäßen 21, 27 die schärfste Ebene ermittelt. Die hohe Durchfahrgeschwindigkeit garantiert, dass aus einem großen Bereich die Fokusebene schnell ermittelt werden kann. Die Nutzung der Ableitung der Fokusfunktion zur Begrenzung des Bereiches erhöht zudem die Robustheit des Systems.

Dem parallel zum Fokus arbeitenden Horizontalausgleich ist es möglich, bei homogener Helligkeitsverteilung einen Ausgleich, auch bei starker Änderung der Z-Achse, durchzuführen.

Insbesondere durch Anwendung des erythrozytebewegungsbasierten Differenzbild-Verfahrens ist eine exakte Positionierung des Laserfokus 30 in die Blutbahn 21 möglich.

Mit dem erfindungsgemäßen Verfahren und den erfindungsgemäßen Vorrichtungen 40 können mit zeitlich bedingten Vorteilen in einer automatisierten Durchführung und Ausstattung zumindest eine der erfindungsgemäßen Vorrichtungen 40 mehrfach in paralleler Anordnung nebeneinander ausgeführt sein, um den Durchlauf unterhalb der optischen Vorrichtung 5 der vielen parallel durchlaufenden Eier 1 zu deren Geschlechtsbestimmung zu beschleunigen. Das kann auch durch den Einsatz einer zentralen Steuereinheit erfolgen, die über Verbindungsleitungen oder kabellos mit den einzelnen Steuereinheiten 45 bzw. Auswerteeinheiten der Vorrichtungen 40 in energieversorgungstechnischer und signaltechnischer Verbindung stehen kann.

Bezugszeichenliste

| 1 | Ei |
|---|---|
| 2 | Loch in der Kalkschale |
| 3 | Anregungslaser |
| 3a | in das Loch der Kalkschale eingebrachte Laserstrahlung |
| 4a | Laserstrahlung zum Ei führende optische Glasfaser |
| 4b | Rückstreustrahlung/Ramanstreustrahlung zum Spektrometer/Detektor führende optische Glasfaser |
| 5 | Optische Vorrichtung zur Einbringung von Laserstrahlung 3a, zur Erfassung von Rückstreustrahlung 7 und Analysepositionsbildflächen $SAF_1$, $SAF_2$ |
| 6 | Objektiv |
| 7 | Rückstreustrahlung/Ramanstreustrahlung/Fluoreszenzstrahlung |
| 8 | Spektrometer mit Detektor |
| 9 | Auswerteeinrichtung |
| 10 | Lichtquelle/Beleuchtungseinrichtung |
| 10a | Licht, mit dem das Vogelei bestrahlt wird |
| 10b | transmittiertes bzw. gestreutes sichtbares Licht |
| 11 | Filter grün |
| 12 | Linse |
| 13 | Kamera zur Detektion von Licht 10b/Detektor |
| 14 | Blutgefäß-Positions-Auswerteeinrichtung |
| 15 | Steuerleitung |
| 16 | xyz-Positioniereinheit zur Ei-Lagesteuerung |
| 17 | Steuerleitungen |
| 18 | koordinative x-,y-,z-Positioniereinheit/Steuereinheit |
| 19 | Strahlungseinrichtung |
| 20 | Vorrichtung des Standes der Technik |
| 21 | Blut durchflossenes Gefäß |
| 22 | Embryo |
| 23 | Strahlteiler |
| 24 | Spitzer Pol |
| 25 | Fläche |
| 26 | Eiweißschicht |
| 27 | Blutgefäßgeflecht |
| 28 | Kalkschale |
| 29 | CCD-Matrix |
| 30 | Laserfokus |
| 31 | Auswerteeinheit |
| 32 | Verbindungsleitung zur Kamera, |
| 33 | Verbindungsleitung zur Halterungseinrichtung, |
| 34 | Verbindungsleitung zur Strahlungsquelle, |
| 35 | Verbindungsleitung zur koordinativen x-,y-,z-Positioniereinheit, |
| 36 | Verbindungsleitung zur Auswerteeinrichtung für optische Messungen, |
| 37 | Kollimator |
| 38 | Erstes Objektiv der Kamera |
| 39 | Analysepositionsbildfläche $SAF_2$ |
| 40 | Erfindungsgemäße Vorrichtung |
| 41 | Reduzierte Bildfläche RBF - ROI - |
| 42 | Strahlengang |
| 43 | Fokus des Objektivs der Kamera |
| 44 | Erster Revolver/Objektivwechsler |
| 45 | Rechner |
| 46 | Maximum des normierten Schärfewertes in Z-Achsenrichtung |
| 61 | erstes Mess-Objektiv |
| 62 | zweites Mess-Objektiv |
| 63 | zweites Kamera-Objektiv |
| 64 | zweiter Revolver/Objektivwechsler |
| 65 | Analysepositionsbildfläche $SAF_1$ |
| 66 | Taille des Laserfokus |
| 67 | Locherzeugungseinheit |

| 68 | Elektrische Leitung |
|---|---|
| $a_t$ | Parameter für die lineare Transformation |
| $A_{ij}$ | Flächeninhalt eines Blocks an der Position i, j |
| $A_{max}$ | maximaler Flächeninhalt |
| $b_t$ | Parameter für die lineare Transformation |
| b | Binärbild |
| B | Blockgröße |
| $c_l$ | Koeffizientensatz für Savitzky-Golay Filter |
| $f_i$ | Messpunkt am Punkt i |
| g | Grauwertfunktion |
| $g_{gm}$ | Glättungsfunktion durch gleitenden Mittelwert |
| $g_{ij}$ | Objektpixel |
| $g_{RB}$ | Ausgangsfunktion nach Rolling-Ball-Algorithmus |
| $g_{sg}$ | Glättungsfunktion nach Savitzky-Golay |
| $h_m$ | Breite einer Bildmatrix |
| i | Laufvariable |
| I | Intensität der Grauwertmatrix |
| j | Laufvariable |
| k | Position |
| $K_1, K_2$ | Anzahl Blöcke |
| l | Laufvariable |
| n | Anzahl |
| $n_R, n_L$ | Nachbarwert rechts und links |
| o | segmentierte Bildmatrix |
| $p_s$ | Sollposition |
| $p_{\varepsilon+}$ | Prozentwert für obere Schaltschwelle |
| $p_{\varepsilon-}$ | Prozentwert für untere Schaltschwelle |
| q | Faktor zur Bestimmung der Tragweite der Standardabweichung |
| R | maximaler Schwellwert |
| $S_{px1}, S_{px2}$ | Strecke in Pixel |
| $S_{rx}$ | reale Strecke in x- Richtung |
| $S_{rz}$ | reale Strecke in z-Richtung |
| S | Schärfewert |
| SW | Schwellwert |
| t | Zeit; Template |
| T | lineare Transformation |
| TP | Tiefpass |
| u | Stellgröße |
| $U_{aus}, U_{ein}$ | Stellgröße aus und ein |
| $U_{Dif}$ | Unterschied Differenzbild |
| $v_z$ | Geschwindigkeit in z-Richtung |
| $v_{z\_exakt}$ | Geschwindigkeit in z-Richtung für exakte Positionierung |
| w | Führungsgröße; Fensterbreite |
| $w_{\varepsilon+}$ | Führungsgröße obere Schaltschwelle |
| $w_{\varepsilon-}$ | Führungsgröße untere Schaltschwelle |
| x | Position des Pixels in der Bildmatrix in x-Richtung |
| $x_c$ | Zentroid in x-Richtung |
| X | Position der Achse in x-Richtung |
| y | Position des Pixels in der Bildmatrix in y-Richtung |
| $y_c$ | Zentroid in y-Richtung |
| $v_m$ | Höhe einer Bildmatrix |
| Y | Position der Achse in y-Richtung |
| z | Zentrum |
| Z | Position der Achse in z-Richtung |
| $\Delta$ | allg. Änderung |
| $\mu$ | Mittelwert |
| $\sigma$ | Standardabweichung |

**Patentansprüche**

1. Verfahren zur Einstellung des Laserfokus (30) eines Anregungslasers (3) in einem Blut durchflossenen Gefäß (21) für optische Messungen zur Bestimmung des Geschlechts von Vogeleiern (1), wobei die Blut durchflossenen Gefäße (21) einschließlich des Embryos (22) beweglich im Vogelei (1) sind und die Blut durchflossenen Gefäße (21) einschließlich des Embryos (22) noch nicht zum Zeitpunkt einer optischen Messung der Rückstreustrahlung (7) an der Kalkschale (28) fixiert sind, **gekennzeichnet durch** folgende Schritte:

   - Selektion eines Blut durchflossenen Gefäßes (21) in dem lochgeöffneten und bebrüteten Vogelei (1) mittels eines kontrastbasierten Auswerteverfahrens unter zumindest einer Beleuchtung eines Blut durchflossenen Gefäßgeflechts (27) mit Strahlung im grünen Wellenlängenbereich mittels einer optischen Vorrichtung (5) zur Einbringung von Laserstrahlung (3a), zur Erfassung von Rückstreustrahlung (7) und Analysepositionsbildflächen SAF$_1$, SAF$_2$,
   - Herstellen des Laserfokus (30) für den Anregungslaser (3) und eines Fokus (43) für eine Kamera (13) in dem selektierten Blut durchflossenen Gefäß (21) durch das dem Gefäß (21) zugewandten Loch (2) hindurch mittels der optischen Vorrichtung (5) zur Einbringung von Laserstrahlung (3a), zur Erfassung von Rückstreustrahlung (7) und Analysepositionsbildflächen SAF$_1$, SAF$_2$,
   - Einstellung des Laserfokus (30) innerhalb des selektierten Blut durchflossenen Gefäßes (21) mittels der optischen Vorrichtung (5) zur Einbringung von Laserstrahlung (3a), zur Erfassung von Rückstreustrahlung (7) und Analysepositionsbildflächen SAF$_1$, SAF$_2$ mittels eines erythrozytbewegungsbasierten Differenzbild-Verfahrens aufgrund der in jeweils zwei nacheinander aufgenommenen Bildern mit infolge der Bewegung der Erythrozyten (36) innerhalb des selektierten Blut durchflossenen Gefäßes (21) auftretenden Intensitätsänderungen als Intensitätswerte $I_{t-1}(x)$, $I_t(x)$ an der Pixelposition $x$ zur Zeit $t-1$ und $t$ und Nachführen des Laserfokus (30) und des Fokus (43) der Kamera (13) bei Absenken der Fläche des Blutgefäßgeflechtes (27) und einer über dem Blutgefäßgeflecht (27) befindlichen Eiweißschicht (26),
   - Ausgleich von auftretenden x-,y-Horizontalbewegungen des selektierten Blut durchflossenen Gefäßes (21) mittels der optischen Vorrichtung (5) zur Einbringung von Laserstrahlung (3a), zur Erfassung von Rückstreustrahlung (7) und Analysepositionsbildflächen SAF$_1$, SAF$_2$,
   - Durchführung der optischen Messungen mittels der optischen Vorrichtung (5) zur Einbringung von Laserstrahlung (3a), zur Erfassung von Rückstreustrahlung (7) und Analysepositionsbildflächen SAF$_1$, SAF$_2$.

2. Verfahren nach Anspruch 1, **dadurch gekennzeichnet,**
   **dass** als die zu messende, aus dem Blut durchflossenen Gefäß (21) rückgestrahlte Rückstreustrahlung (7) eine Ramanstreustrahlung und/oder eine rückgestreute Fluoreszenzstrahlung für die optischen Messungen ausgewählt werden.

3. Verfahren nach Anspruch 1 oder 2, **gekennzeichnet durch** folgende Schritte:

   - Einbringung eines Lochs (2) in der Kalkschale (28) mittels einer Locherzeugungseinheit und Positionierung der Blutgefäße (21) im Bereich des Loches (2),
   - Suchen der sich im Vogelei (1) ausbildenden Blutgefäße (21) mittels eines zumindest aus einer Strahlungseinrichtung (10) und aus einem Detektor (13) bestehenden Visions-Systems (10, 13) und einer koaxialen oder lateralen Beleuchtung mit Licht (10a) des sichtbaren Wellenlängenbereiches,
   - Positionieren zumindest des Blut durchflossenen Gefäßes (21) in den Laserfokus (30) des Anregungslasers (3) entweder durch Bewegen des Vogeleies (1) mittels einer gesteuerten Positioniereinheit (16) oder durch ein gesamtheitliches Bewegen der optischen Vorrichtung (5) zur Einbringung von Laserstrahlung (3a), zur Erfassung von Rückstreustrahlung (7) und Analysepositionsbildflächen SAF$_1$, SAF$_2$ oder durch einen Wechsel mindestens eines Objektivs (61, 62; 38, 63) oder durch ein Objektiv (61; 38) mit variabler Vergrößerung in der optischen Vorrichtung (5) zur Einbringung von Laserstrahlung (3a), zur Erfassung von Rückstreustrahlung (7) und Analysepositionsbildflächen SAF$_1$, SAF$_2$,
   - Registrieren der Rückstreustrahlung (7) des bestrahlten Blut durchflossenen Gefäßes (21) mittels der optischen Vorrichtung (5) zur Einbringung von Laserstrahlung, zur Erfassung von Rückstreustrahlung (7) und Analysepositionsbildflächen SAF$_1$, SAF$_2$, wobei während der Registrierung der Rückstreustrahlung (7) eine Bewegung des Blutgefäßes (21) aus dem Laserfokus (30) und dem Fokus (43) der Kamera (13) heraus durch Nachführung des Vogeleies (1) mittels einer gesteuerten Positioniereinheit (16) und/oder durch ein gesamtheitliches Bewegen der optischen Vorrichtung (5) zur Einbringung von Laserstrahlung (3a), zur Erfassung von Rückstreustrahlung (7) und Analysepositionsbildflächen (SAF$_1$, SAF$_2$) oder nach einem Wechsel mindestens eines Objektivs (61, 62; 38, 63) oder nach einem Wechsel der Vergrößerung jeweils eines der Objektive (61; 38) in der optischen Vorrichtung (5) zur Einbringung von Laserstrahlung (3a), zur Erfassung von Rückstreustrahlung (7) und Analysepositionsbildflächen SAF$_1$, SAF$_2$ vermieden werden kann,
- Auswertung der Rückstreustrahlung (7) und Bestimmung des Geschlechtes des Embryos in einer Auswerteeinheit (9),
- Anzeige des Geschlechtes des Embryos im Vogelei (1),

**dadurch gekennzeichnet, dass** in der Auswerteeinheit (9) mittels programmtechnischen Mitteln in einem ersten Algorithmus ein kontrastbasiertes Verfahren zur Festlegung einer gesamten Analysepositionsbildfläche SAF$_1$ (65) mit dem Blut durchflossenen Blutgefäßgeflecht (27) durchgeführt wird, und
in der Auswerteeinheit (9) mittels programmtechnischer Mittel in einem zweiten Algorithmus ein erythrozytbewegungsbasiertes Differenzbild-Verfahren zur Einstellung des Laserfokus (30) mittels der optischen Vorrichtung (5) zur Einbringung von Laserstrahlung (3a), zur Erfassung von Rückstreustrahlung (7) und Analysepositionsbildflächen SAF$_1$, SAF$_2$ auf eine reduzierte Bildfläche RBF (41) in der selektierten Analysepositionsbildfläche SAF$_2$ (39) mit dem selektierten Blut durchflossenen Gefäß (21) durchgeführt wird, wobei beim erythrozytbewegungsbasierten Differenzbild-Verfahren die Intensitätsinhalte $I_{t-1}(x)$, $I_t(x)$ von mindestens zwei aufeinanderfolgenden Bildern der auf das selektierte Blut durchflossenen Gefäß (21) reduzierten Bildfläche RBF - ROI - (41) innerhalb der selektierten Analysepositionsbildfläche SAF$_2$ (39) verglichen werden, wobei die Intensitätswerte $I_{t-1}(x)$, $I_t(x)$ von den sich jeweils in der reduzierten Bildfläche RBF (41) widerspiegelnden Bewegungen der Erythrozyten unterschiedlich ausgebildet werden, und wobei der Laserfokus (30) des Anregungslasers (3) und der Fokus (43) der Kamera (13) mittels der optischen Vorrichtung (5) zur Einbringung von Laserstrahlung (3a), zur Erfassung von Rückstreustrahlung (7) und Analysepositionsbildflächen SAF$_1$, SAF$_2$ auf eine dem selektierten Blut durchflossenen Gefäß (21) zugeordnete reduzierte Bildfläche RBF (41) eingestellt wird,
wobei sich der Laserfokus (30) und der Fokus (43) immer innerhalb des selektierten Blut durchflossenen Gefäßes (21) befinden.

4. Verfahren nach Anspruch 3, **dadurch gekennzeichnet,**
**dass** als Licht (10a) eine Strahlung des sichtbaren Wellenlängenbereiches oder des grünes Wellenlängenbereiches aus der Strahlungsquelle (10) des Visions-Systems (10, 13) zur Überwachung des Zeitverlaufs der Vorgänge beim Bebrüten des Vogeleies (1) eingesetzt wird.

5. Verfahren nach Anspruch 2 bis 4, **dadurch gekennzeichnet,**
**dass** das optische Messverfahren zur in-ovo Geschlechtsbestimmung von befruchteten und bebrüteten Vogeleiern (1) zumindest nach 48 Stunden Bebrütung durchgeführt wird.

6. Verfahren nach den Ansprüchen 2 bis 5, **dadurch gekennzeichnet,**
**dass** folgende Parameter für die optischen Messungen der vom Blut durchflossenen Gefäß (21) rückgestreuten Ramanstreustrahlung eingesetzt werden:

- Anregungswellenlängen der Laserstrahlung (3a) der Anregungslaser (3): > 600 nm (z.B. HeNe Laser 633 nm, Festkörperlaser (Nd basiert z.B. Nd:YAG Laser 1064 nm, VIS NIR Diodenlaser, z.B. 785 nm),
- Einkopplung der Laserstrahlung (3a) direkt mit Spiegeln und/oder mit optischen Fasern (4a),
- Ramanstreustrahlungsmessungen werden unter Nutzung einer optischen Vorrichtung (5) mit großer numerischer Apertur wie Mikroskop-Objektiven (61) oder einer Fasersonde (4b) durchgeführt,
- Direkte Auskopplung der gesammelten Ramanstreustrahlung (7) mittels Spiegeln zu einem Spektrometer (8) oder mittels Transports über optische Fasern,
- Einsatz eines Spektrometers (8) in Form von dispersivem Spektrometer und Fourier Transform Spektrometer.

7. Verfahren nach den Ansprüchen 2 bis 5, **dadurch gekennzeichnet,**
**dass** folgende Parameter für die optischen Messungen der vom Blut durchflossenen Gefäß (21) rückgestreuten Fluoreszenzstrahlung eingesetzt werden:

- Anregungswellenlängen der Laserstrahlung (3a) der Anregungslaser (3): > 400 nm (z.B. HeNe Laser 633 nm, Festkörperlaser (Nd basiert z.B. Nd:YAG Laser 532 nm, VIS NIR Diodenlaser, z.B. 785 nm),
- Einkopplung der Laserstrahlung (3a) direkt mit Spiegeln und/oder mit optischen Fasern (4a),
- Fluoreszenzstrahlungsmessungen werden unter Nutzung von optischen Vorrichtungen (5) wie Mikroskop-Objektiven (61) oder einer Fasersonde (4b) durchgeführt,
- Direkte Auskopplung der gesammelten Fluoreszenzstrahlung (7) mittels mindestens eines Spiegels oder mittels Transports über optische Fasern,

- Einsatz eines Detektors (8).

8. Verfahren nach den Ansprüchen 1 bis 7, **dadurch gekennzeichnet,**
**dass** während eines Positionierungsvorgangs des Vogeleies (1) permanent Messwerte von optischen Messungen aufgezeichnet und einer Auswertung zugeführt werden, wobei eine automatische Klassifizierung der Rückstreustrahlung (7) anhand des spektralen Fingerabdruckes des Blutes erfolgt.

9. Verfahren nach Anspruch 1 oder 2, **dadurch gekennzeichnet,**
**dass** das Vogelei (1) pollastig gestellt wird.

10. Verfahren nach Anspruch 9, **dadurch gekennzeichnet,**
**dass** ein Loch (2) im Bereich des nach oben gerichteten spitzen Pols (24) bei einer Lochgröße bis zu 18 mm zumindest nach 48 Stunden Bebrütung mittels der Locherzeugungseinheit ausgebildet wird.

11. Verfahren nach Anspruch 9, **dadurch gekennzeichnet,**
**dass** ein Loch (2) am nach oben gerichteten, stumpfen Pol des Vogeleies (1) einen Durchmesser bis zu 18 mm, vorzugsweise zwischen 8 mm und 12 mm mittels der Locherzeugungseinheit ausgebildet wird.

12. Verfahren nach Anspruch 1 bis 6 und 8 bis 11, **dadurch gekennzeichnet, dass** das optische Messverfahren als Ramanspektroskopisches in-ovo Mess-Verfahren zur Geschlechtsbestimmung von befruchteten und bebrüteten Vogeleiern (1) zumindest nach 48 Stunden Bebrütung durchgeführt wird.

13. Vorrichtung (40) zur Einstellung des Laserfokus (30) eines Anregungslasers (3) in einem Blut durchflossenen Gefäß (21) für optische Messungen zur Bestimmung des Geschlechts von Vogeleiern (1), wobei die Blut durchflossenen Gefäße (21) einschließlich des Embryos (22) beweglich im Vogelei (1) sind und noch nicht zum Zeitpunkt einer optischen Messung der Rückstreustrahlung (7) an der Kalkschale (28) fixiert ist, wobei die Vorrichtung derart ausgestattet ist ein Verfahren nach den Ansprüchen 1 bis 12 durchzuführen, und wobei die Vorrichtung (40) zumindest umfasst

- eine Halterungseinrichtung (16) für das mit einem Loch (2) versehene Vogelei (1), wobei die Vogeleier (1) jeweils mit einem Pol (24) nach oben gelagert sind,
- eine Strahlungsquelle (10) für die Beleuchtung der im Vogelei (1) befindlichen Fläche mit Eiweißschicht (26) und mit Blutgefäßgeflecht (27),
- eine koordinative x-,y-,z-Positioniereinheit (18) für eine optische Vorrichtung (5) zur Einbringung von Laserstrahlung (3a), zur Erfassung von Rückstreustrahlung (7) und Analysepositionsbildflächen SAF$_1$, SAF$_2$,
- die optische Vorrichtung (5) zur Einbringung von Laserstrahlung (3a), zur Erfassung von Rückstreustrahlung (7) und Analysepositionsbildflächen SAF$_1$, SAF$_2$, zumindest enthaltend

- mindestens ein Mess-Objektiv (61, 62),
- einen Strahlteiler (23),
- eine Kamera (13) mit mindestens einem Kamera-Objektiv (38, 63), wobei die optische Vorrichtung (5) zur Abbildungsänderung zwischen einem Bild der SAF$_1$ (65) und einem Bild der RBF (41) dient, wobei das Bild der SAF$_1$ (65) die gesamte Bildfläche des Blutgefäßgeflechtes (27) und das Bild der RBF (41) die reduzierte Bildfläche des selektierten Blut durchflossenen Gefäßes (21) betreffen,

- eine Auswerteeinrichtung (9) für optische Spektren,
- eine Auswerteeinheit (31), die zumindest umfasst

- eine Verbindungsleitung (32) zur Kamera (13),
- eine Verbindungsleitung (33) zur Halterungseinrichtung (16),
- eine Verbindungsleitung (34) zur Strahlungsquelle (10),
- eine Verbindungsleitung (35) zur koordinativen x-,y-,z-Positioniereinheit (18),
- eine Verbindungsleitung (36) zur Auswerteeinrichtung (9) für optische Messungen,

wobei die Auswerteeinheit (31) programmtechnische Mittel zur Durchführung eines kontrastbasierten Verfahrens, das sich auf eine gesamte Bildfläche SAF$_1$ (65) mit dem Blutgefäßgeflecht (27) bezieht, und eines erythrozytbewegungsbasierten Differenzbild-Verfahrens, das sich auf die reduzierte Bildfläche RBF (41) der selektierten Analysepositionsbildfläche SAF$_2$ (39) mit einem selektierten Blut durchflossenen Gefäß (21) des Blutgefäßgeflechtes (27) bezieht.

14. Vorrichtung nach Anspruch 13, **dadurch gekennzeichnet,**
**dass** in der optischen Vorrichtung (5) zur Einbringung von Laserstrahlung, zur Erfassung von Rückstreustrahlung (7) und Analysepositionsbildflächen $SAF_1$, $SAF_2$,
ein erstes Mess-Objektiv (61) zur Durchführung eines kontrastbasierten Auswerteverfahrens und
ein zweites Mess-Objektiv (62) zur Durchführung eines erythrozytbewegungsbasierten Differenzbild-Verfahrens enthalten sind, wobei sich die Mess-Objektive (61, 62) jeweils zur Durchführung der beiden Verfahren abwechselnd in den Strahlengang (42) der Kamera (13) einschieben.

15. Vorrichtung nach Anspruch 14, **dadurch gekennzeichnet,**
**dass** anstelle der beiden Mess-Objektive (61, 62) ein Mess-Objektiv mit variabler Vergrößerung in den Strahlengang (42) der Kamera (13) eingebracht ist, so dass kein zweites Mess-Objektiv und keine Verschiebung von Objektiven erforderlich sind.

16. Vorrichtung nach Anspruch 13, **dadurch gekennzeichnet,**
**dass** in der optischen Vorrichtung (5) zur Einbringung von Laserstrahlung (3a), zur Erfassung von Rückstreustrahlung (7) und Analysepositionsbildflächen $SAF_1$, $SAF_2$,
ein erstes Kamera-Objektiv (38) zur Durchführung eines kontrastbasierten Auswerteverfahrens und
ein zweites Kamera-Objektiv (63) zur Durchführung eines erythrozytbewegungsbasierten Differenzbild-Verfahrens enthalten sind, wobei sich die Kamera-Objektive (38, 63) jeweils zur Durchführung der beiden Verfahren abwechselnd in den Strahlengang (42) der Kamera (13) einschieben.

17. Vorrichtung nach Anspruch 16, **dadurch gekennzeichnet,**
**dass** anstelle der beiden Kamera-Objektive (38, 63) ein Kamera-Objektiv mit variabler Vergrößerung in den Strahlengang (42) der Kamera (13) eingebracht werden, so dass kein zweites Kamera-Objektiv und keine Verschiebung von Objektiven erforderlich sind.

18. Vorrichtung nach den Ansprüchen 13 bis 17, **dadurch gekennzeichnet,**
**dass** die optische Vorrichtung (5) zur Einbringung von Laserstrahlung, zur Erfassung von Rückstreustrahlung (7) und Analysepositionsbildflächen $SAF_1$, $SAF_2$ mit der koordinativen x-,y-,z-Positioniereinheit (18) in Verbindung steht, wobei die koordinative x-,y-,z-Positioniereinheit (18) die optische Vorrichtung (5) in x-,y-,z-Richtung steuernd bewegen kann.

19. Vorrichtung nach den Ansprüchen 13 bis 18, **dadurch gekennzeichnet,**
**dass** die zu messende, aus dem Blut durchflossenen Gefäß (21) rückgestrahlte Rückstreustrahlung (7) eine Ramanstreustrahlung und/oder eine rückgestreute Fluoreszenzstrahlung ist.

## Claims

1. Method for setting the laser focus (30) of an excitation laser (3) in a vessel (21) through which blood flows, for the purposes of optical measurements for determining the sex of bird's eggs (1), wherein the vessels (21) through which blood flows, including the embryo (22), are movable in the bird's egg (1) and the vessels (21) through which blood flows, including the embryo (22), are not yet fixated at the time of an optical measurement of the backscattered radiation (7) at the shell (28), **characterized by** the following steps:

- selecting a vessel (21) through which blood flows in the hole-opened and incubated bird's egg (1) by means of a contrast-based evaluation method while at least illuminating a vascular plexus (27) through which blood flows with radiation in the green wavelength range by means of an optical apparatus (5) for introducing laser radiation (3a), for the purposes of detecting backscattered radiation (7) and analysis position image areas $SAF_1$, $SAF_2$,
- producing the laser focus (30) for the excitation laser (3) and a focus (43) for a camera (13) in the selected vessel (21) through which blood flows, through the hole (2) facing the vessel (21), by means of the optical apparatus (5) for introducing laser radiation (3a), for the purposes of detecting backscattered radiation (7) and analysis position image areas SAF1, SAF2,
- setting the laser focus (30) within the selected vessel (21) through which blood flows, by means of the optical apparatus (5) for introducing laser radiation (3a) and for the purposes of capturing backscattered radiation (7) and analysis position image areas $SAF_1$, $SAF_2$ by means of an erythrocyte movement-based difference image method on the basis of the intensity changes, occurring in respectively two successively recorded images on account of the movement of the erythrocytes (36) within the selected vessel (21) through which blood flows, as intensity values $I_{t-1}(x)$, $I_t(x)$ at the pixel position $x$ at times $t-1$ and $t$ and tracking the laser focus (30) and the focus (43) of the camera (13) when lowering the area of the blood vascular plexus (27) and an albumen layer (26) situated above the blood vascular plexus (27),

- compensating occurring x-, y-horizontal movements of the selected vessel (21) through which blood flows, by means of the optical apparatus (5) for introducing laser radiation (3a) and for the purposes of capturing backscattered radiation (7) and analysis position image areas $SAF_1$, $SAF_2$,
- carrying out the optical measurements by means of the optical apparatus (5) for introducing laser radiation (3a) and for the purposes of capturing backscattered radiation (7) and analysis position image areas $SAF_1$, $SAF_2$.

2. Method according to Claim 1, **characterized in that** Raman scattering radiation and/or backscattered fluorescence radiation is selected for the optical measurements as the backscattered radiation (7) to be measured, which was scattered back from the vessel (21) through which blood flows.

3. Method according to Claim 1 or 2, **characterized by** the following steps:

- introducing a hole (2) in the shell (28) by means of a hole generating unit and positioning the blood vessels (21) in the region of the hole (2),
- searching for the blood vessels (21) forming in the bird's egg (1) by means of a vision system (10, 13), which at least consists of a radiation device (10) and a detector (13), and coaxial or lateral illumination with light (10a) in the visible wavelength range,
- positioning at least the vessel (21) through which blood flows in the laser focus (30) of the excitation laser (3), either by moving the bird's egg (1) by means of a controlled positioning unit (16) or by means of an overall movement of the optical apparatus (5) for introducing laser radiation (3a), and for the purposes of capturing backscattered radiation (7) and analysis position image areas $SAF_1$, $SAF_2$, or by means of changing at least one objective (61, 62; 38, 63) or by means of an objective (61; 38) with a variable magnification in the optical apparatus (5) for introducing laser radiation (3a), and for the purposes of capturing backscattered radiation (7) and analysis position image areas $SAF_1$, $SAF_2$,
- registering the backscattered radiation (7) of the irradiated vessel (21) through which blood flows, by means of the optical apparatus (5) for introducing laser radiation and for the purposes of capturing backscattered radiation (7) and analysis position image areas $SAF_1$, $SAF_2$, wherein, during registration of the backscattered radiation (7), a movement of the blood vessel (21) from the laser focus (30) and the focus (43) of the camera (13) can be avoided by tracking the bird's egg (1) by means of a controlled positioning unit (16) and/or by means of an overall movement of the optical apparatus (5) for introducing laser radiation (3a), and for the purposes of capturing backscattered radiation (7) and analysis position image areas ($SAF_1$, $SAF_2$), or after a change of at least one objective (61, 62; 38, 63) or after a change of the magnification of respectively one of the objectives (61; 38) in the optical apparatus (5) for introducing laser radiation (3a), and for the purposes of capturing backscattered radiation (7) and analysis position image areas $SAF_1$, $SAF_2$,
- evaluating the backscattered radiation (7) and determining the sex of the embryo in an evaluation unit (9),
- displaying the sex of the embryo in the bird's egg (1),

**characterized in that** a contrast-based method for defining an overall analysis position image area $SAF_1$ (65) with the blood vascular plexus (27) through which blood flows is carried out in a first algorithm in the evaluation unit (9) by means of programming means, and an erythrocyte movement-based difference image method for setting the laser focus (30), by means of the optical apparatus (5) for introducing laser radiation (3a) and for the purposes of capturing backscattered radiation (7) and analysis position image areas $SAF_1$, $SAF_2$, to a reduced image area RBF (41) in the selected analysis position image area $SAF_2$ (39) with the selected vessel (21) through which blood flows is carried out in a second algorithm in the evaluation unit (9) by means of programming means, wherein the intensity contents $I_{t-1}(x)$, $I_t(x)$ of at least two successive images of the reduced image area RBF - ROI - (41) within the selected analysis position image area $SAF_2$ (39) are compared in the erythrocyte movement-based difference image method, wherein the intensity values $I_{t-1}(x)$, $I_t(x)$ of the movements of the erythrocytes, which are respectively reflected in the reduced image area RBF (41), are embodied differently, and wherein the laser focus (30) of the excitation laser (3) and the focus (43) of the camera (13) are set to a reduced image area RBF (41) assigned to the selected vessel (21) through which blood flows, by means of the optical apparatus (5) for introducing laser radiation (3a) and for the purposes of capturing backscattered radiation (7) and analysis position image areas $SAF_1$, $SAF_2$, wherein the laser focus (30) and the focus (43) are always situated within the selected vessel (21) through which blood flows.

4. Method according to Claim 3, **characterized in that** radiation of the visible wavelength range or of the green wavelength range is used as light (10a) from the radiation source (10) of the vision system (10, 13) for monitoring the progress over time of the processes when incubating the bird's egg (1).

5. Method according to Claims 2 to 4, **characterized in that** the optical measuring method for in-ovo sexing of fertilized and incubated bird's eggs (1) is carried out at least after 48 hours of incubation.

6. Method according to Claims 2 to 5, **characterized in that** the following parameters are used for optical measurements of the Raman scattering radiation backscattered from the vessel (21) through which blood flows:

- excitation wavelengths of the laser radiation (3a) of the excitation lasers (3): >600 nm (e.g., HeNe laser 633 nm, solid-state laser (Nd-based, e.g., Nd:YAG laser 1064 nm, VIS NIR diode laser, e.g., 785 nm),
- direct input coupling of the laser radiation (3a) using mirrors and/or using optical fibres (4a),
- Raman scattering radiation measurements are performed using an optical apparatus (5) with a large numerical aperture, such as microscope objectives (61) or a fibre probe (4b),
- direct output coupling of the collected Raman scattering radiation (7) by means of mirrors to a spectrometer (8) or by means of transportation via optical fibres,
- use of a spectrometer (8) in the form of a dispersive spectrometer and Fourier transform spectrometer.

7. Method according to Claims 2 to 5, **characterized in that** the following parameters are used for the optical measurements of the fluorescence radiation backscattered from the vessel (21) through which blood flows:

- excitation wavelengths of the laser radiation (3a) of the excitation lasers (3): >400 nm (e.g., HeNe laser 633 nm, solid-state laser (Nd-based, e.g., Nd:YAG laser 532 nm, VIS NIR diode laser, e.g., 785 nm),
- direct input coupling of the laser radiation (3a) using mirrors and/or using optical fibres (4a),
- fluorescence radiation measurements are performed using optical apparatuses (5) such as microscope objectives (61) or a fibre probe (4b),
- direct output coupling of the collected fluorescence radiation (7) by means of at least one mirror or by means of transportation via optical fibres,
- use of a detector (8).

8. Method according to Claims 1 to 7, **characterized in that** measurement values of optical measurements are recorded on an ongoing basis during a positioning procedure for the bird's egg (1) and said optical measurements are fed to an evaluation, wherein there is an automatic classification of the backscattered radiation (7) on the basis of the spectral fingerprint of the blood.

9. Method according to Claim 1 or 2, **characterized in that** the bird's egg (1) is placed in a pole-heavy fashion.

10. Method according to Claim 9, **characterized in that** a hole (2) in the region of the upwardly directed pointed pole (24) is formed at least after 48 hours of incubation by means of the hole generating device in the case of a hole dimension up to 18 mm.

11. Method according to Claim 9, **characterized in that** a hole (2) in the upwardly directed blunt pole of the bird's egg (1) is formed by means of the hole generating device, having a diameter up to 18 mm, preferably between 8 mm and 12 mm.

12. Method according to Claims 1 to 6 and 8 to 11, **characterized in that** the optical measuring method is performed as Raman spectroscopic in-ovo measuring method for sexing fertilized and incubated bird's eggs (1) at least after 48 hours of incubation.

13. Apparatus (40) for setting the laser focus (30) of an excitation laser (3) in a vessel (21) through which blood flows, for the purposes of optical measurements for determining the sex of bird's eggs (1), wherein the vessels (21) through which blood flows, including the embryo (22), are movable in the bird's egg (1) and not yet fixated at the time of an optical measurement of the backscattered radiation (7) at the shell (28), wherein the apparatus is equipped to carry out a method according to Claims 1 to 12 and wherein the apparatus (40) at least comprises:

- a holding device (16) for the bird's egg (1) which has been provided with a hole (2), wherein the bird's eggs (1) are each stored with one pole (24) at the top,
- a radiation source (10) for illuminating the area with albumin layer (26) and with the blood vascular plexus (27), which area is situated in the bird's egg (1),
- a coordinative x-, y-, z-positioning unit (18) for an optical apparatus (5) for introducing laser radiation (3a), and for the purposes of capturing backscattered radiation (7) and analysis position image areas $SAF_1$, $SAF_2$,
- the optical apparatus (5) for introducing laser radiation (3a), and for the purposes of capturing backscattered radiation (7) and analysis position image areas $SAF_1$, $SAF_2$, at least containing
- at least one measurement objective (61, 62),
- a beam splitter (23),
- a camera (13) with at least one camera objective (38, 63),

wherein the optical apparatus (5) serves for changing the image representation between an image of the $SAF_1$ (65) and an image of the RBF (41), wherein the image of the $SAF_1$ (65) relates to the entire image area of the blood vascular plexus (27) and the image of the RBF (41) relates to the reduced image area of the selected vessel (21) through which blood flows,

- an evaluation device (9) for optical spectra,
- an evaluation unit (31), which at least comprises
- a connecting cable (32) to the camera (13),
- a connecting cable (33) to the holding device (16),
- a connecting cable (34) to the radiation source (10),
- a connecting cable (35) to the coordinative x-, y-, z-positioning unit (18),
- a connecting cable (36) to the evaluation device (9) for optical measurements,

wherein the evaluation unit (31) has programming means for carrying out a contrast-based method, which relates to an overall image area $SAF_1$ (65) with the blood vascular plexus (27), and an erythrocyte movement-based difference image method, which relates to the reduced image area RBF (41) of the selected analysis position image area $SAF_2$ (39) with a selected vessel (21) through which blood flows of the blood vascular plexus (27).

14. Apparatus according to Claim 13, **characterized in that** the optical apparatus (5) for introducing laser radiation, and for the purposes of capturing backscattered radiation (7) and analysis position image areas $SAF_1$, $SAF_2$, contains
a first measurement objective (61) for performing a contrast-based evaluation method and
a second measurement objective (62) for performing an erythrocyte movement-based difference image method, wherein the measurement objectives (61, 62) are alternately pushed into the beam path (42) of the camera (13), in each case to carry out the two methods.

15. Apparatus according to Claim 14, **characterized in that** a measurement objective with a variable magnification is introduced into the beam path (42) of the camera (13) in place of the two measurement objectives (61, 62), and so there is no need for a second measurement objective and no need for a displacement of objectives.

16. Apparatus according to Claim 13, **characterized in that** the optical apparatus (5) for introducing laser radiation (3a), and for the purposes of capturing backscattered radiation (7) and analysis position image areas $SAF_1$, $SAF_2$, contains
a first camera objective (38) for performing a contrast-based evaluation method and
a second camera objective (63) for performing an erythrocyte movement-based difference image method, wherein the camera objectives (38, 63) are alternately pushed into the beam path (42) of the camera (13), in each case to carry out the two methods.

17. Apparatus according to Claim 16, **characterized in that** a camera objective with a variable magnification is introduced into the beam path (42) of the camera (13) in place of the two camera objectives (38, 63), and so there is no need for a second camera objective and no need for a displacement of objectives.

18. Apparatus according to Claims 13 to 17, **characterized in that** the optical apparatus (5) for introducing laser radiation, and for the purposes of capturing backscattered radiation (7) and analysis position image areas $SAF_1$, $SAF_2$, is connected to the coordinative x-, y-, z-positioning unit (18), wherein the coordinative x-, y-, z-positioning unit (18) can move the optical apparatus (5) in the x-, y-, z-direction in controlling fashion.

**19.** Apparatus according to Claims 13 to 18, **characterized in that** the backscattered radiation (7) to be measured, which was scattered back from the vessel (21) through which blood flows, is Raman scattering radiation and/or backscattered fluorescence radiation.

**Revendications**

**1.** Procédé de réglage du foyer laser (30) d'un laser d'excitation (3) dans un vaisseau (21) traversé par un flux de sang pour des mesures optiques en vue de déterminer le sexe d'œufs d'oiseau (1), les vaisseaux (21) traversés par un flux de sang ainsi que l'embryon (22) étant mobiles dans l'œuf d'oiseau (1) et les vaisseaux (21) traversés par un flux de sang ainsi que l'embryon (22), au moment d'une mesure optique du rayonnement rétrodiffusé (7), n'étant pas encore attachés à la coquille calcaire (28), **caractérisé par** les étapes suivantes :

- sélection d'un vaisseau (21) traversé par un flux de sang dans l'œuf d'oiseau (1) perforé et incubé au moyen d'un procédé d'interprétation basé sur le contraste sous au moins un éclairage d'un réseau de vaisseaux (27) traversés par un flux de sang avec un rayonnement dans la plage de longueurs d'onde verte au moyen d'un arrangement optique (5) destiné à introduire le rayonnement laser (3a), en vue de détecter un rayonnement rétrodiffusé (7) et des surfaces d'image de position d'analyse SAF$_1$, SAF$_2$,
- création du foyer laser (30) pour le laser d'excitation (3) et d'un foyer (43) pour une caméra (13) dans le vaisseau (21) traversé par un flux de sang sélectionné à travers le trou (2) qui fait face au vaisseau (21) au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$,
- réglage du foyer laser (30) à l'intérieur du vaisseau (21) traversé par un flux de sang sélectionné au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$ au moyen d'un procédé d'image différentielle basé sur un mouvement des érythrocytes en s'appuyant sur les variations d'intensité qui se produisent respectivement dans deux images enregistrées l'une après l'autre en conséquence du mouvement des érythrocytes (36) à l'intérieur du vaisseau (21) traversé par un flux de sang sélectionné, sous la forme de valeurs d'intensité $I_{t-1}(x)$, $I_t(x)$, à la position de pixel x à l'instant t-1 et t, et asservissement du foyer laser (30) et du foyer (43) de la caméra (13) lors de l'abaissement de la surface du réseau de vaisseaux sanguins (27) et d'une couche de blanc d'œuf (26) qui se trouve au-dessus du réseau de vaisseaux sanguins (27),
- compensation des mouvements horizontaux x, y qui se produisent du vaisseau (21) traversé par un flux de sang sélectionné au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$,
- réalisation des mesures optiques au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$.

**2.** Procédé selon la revendication 1, **caractérisé en ce que** le rayonnement rétrodiffusé (7) à mesurer sélectionné pour les mesures optiques, provenant du vaisseau (21) traversé par un flux de sang, est un rayonnement Raman et/ou un rayonnement fluorescent rétrodiffusé.

**3.** Procédé selon la revendication 1 ou 2, **caractérisé par** les étapes suivantes :

- mise en place d'un trou (2) dans la coquille calcaire (28) au moyen d'une unité de création de trou et positionnement des vaisseaux sanguins (21) dans la zone du trou (2),
- recherche des vaisseaux sanguins (21) qui se forment dans l'œuf d'oiseau (1) au moyen d'un système de vision (10, 13) qui se compose au moins d'un arrangement de rayonnement (10) et d'un détecteur (13) et d'un éclairage coaxial ou latéral avec de la lumière (10a) dans la plage de longueurs d'onde visible,
- positionnement d'au moins le vaisseau (21) traversé par un flux de sang dans le foyer laser (30) du laser d'excitation (3) soit par déplacement de l'œuf d'oiseau (1) au moyen d'une unité de positionnement commandée (16), soit par un déplacement commun de l'arrangement optique (5) destiné à introduire le rayonnement laser (3a), en vue de détecter le rayonnement rétrodiffusé (7) et les surfaces d'image de position d'analyse SAF$_1$, SAF$_2$, soit par un changement d'au moins un objectif (61, 62 ; 38, 63), soit par un objectif (61 ; 38) à grossissement variable dans l'arrangement optique (5) destiné à introduire le rayonnement laser (3a), en vue de détecter le rayonnement rétrodiffusé (7) et les surfaces d'image de position d'analyse SAF$_1$, SAF$_2$,
- enregistrement du rayonnement rétrodiffusé (7) du vaisseau (21) traversé par un flux de sang irradié au moyen de l'arrangement optique (5) destiné à introduire le rayonnement laser, en vue de détecter le rayonnement rétrodiffusé (7) et les surfaces d'image de position d'analyse SAF$_1$, SAF$_2$, un déplacement du vaisseau sanguin (21) hors du foyer laser (30) et du foyer (43) de la caméra (13) pouvant être évité pendant l'enregistrement du rayonnement rétrodiffusé (7) par un asservissement de l'œuf d'oiseau (1) au moyen d'une unité de positionnement (16) commandée et/ou par un déplacement commun de l'arrangement optique (5) destiné à introduire le rayonnement laser (3a), en vue de détecter le rayonnement rétrodiffusé (7) et les surfaces d'image de position d'analyse (SAF$_1$, SAF$_2$) ou après un changement d'au moins un objectif (61, 62 ; 38, 63) ou après un changement du grossissement respectivement d'au moins un objectif (61 ; 38) dans l'arrangement optique (5) destiné à introduire le rayonnement laser (3a), en vue de détecter le rayonnement rétrodiffusé (7) et les surfaces d'image de position d'analyse SAF$_1$, SAF$_2$,

- interprétation du rayonnement rétrodiffusé (7) et détermination du sexe de l'embryon dans une unité d'interprétation (9),
- affichage du sexe de l'embryon dans l'œuf d'oiseau (1),

**caractérisé en ce qu'**un procédé basé sur le contraste est mis en œuvre dans l'unité d'interprétation (9) avec le réseau de vaisseaux sanguins (27) traversé par un flux de sang, au moyen de moyens techniques de programme dans un premier algorithme, en vue de définir une surface d'image de position d'analyse totale SAF$_1$ (65), et un procédé d'image différentielle basé sur un mouvement des érythrocytes est mis en œuvre dans l'unité d'interprétation (9) au moyen de moyens techniques de programme dans un deuxième algorithme en vue de régler le foyer laser (30) au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$ sur une surface d'image réduite RBF (41) dans la surface d'image de position d'analyse sélectionnée SAF$_2$ (39) avec le vaisseau (21) traversé par un flux de sang sélectionné, lors du procédé d'image différentielle basé sur un mouvement des érythrocytes, les teneurs en intensité $I_{t-1}(x)$, $I_t(x)$ d'au moins deux images successives de la surface d'image RBF - ROI (41) réduite au vaisseau (21) traversé par un flux de sang sélectionné étant comparées à l'intérieur de la surface d'image de position d'analyse sélectionnée SAF$_2$ (39), les valeurs d'intensité $I_{t-1}(x)$, $I_t(x)$ des mouvements des érythrocytes qui se reflètent respectivement dans la surface d'image réduite RBF (41) étant représentées différemment, et le foyer laser (30) du laser d'excitation (3) ainsi que le foyer (43) de la caméra (13) étant réglés au moyen de l'arrangement optique (5) destiné à introduire le rayonnement laser (3a), en vue de détecter le rayonnement rétrodiffusé (7) et les surfaces d'image de position d'analyse SAF$_1$, SAF$_2$ sur une surface d'image réduite RBF (41) associée au vaisseau (21) traversé par un flux de sang sélectionné, le foyer laser (30) et le foyer (43) se trouvant toujours à l'intérieur du vaisseau (21) traversé par un flux de sang sélectionné.

4. Procédé selon la revendication 3, **caractérisé en ce que** la lumière (10a) utilisée est un rayonnement de la plage de longueurs d'onde visible ou de la plage de longueurs d'onde verte issu de la source de rayonnement (10) du système de vision (10, 13) servant à surveiller l'évolution dans le temps des opérations lors de l'incubation de l'œuf d'oiseau (1).

5. Procédé selon les revendications 2 à 4, **caractérisé en ce que** le procédé de mesure optique destiné à la détermination in-ovo du sexe d'œufs d'oiseau (1) fécondés et incubés est mis en œuvre au moins après 48 heures d'incubation.

6. Procédé selon les revendications 2 à 5, **caractérisé en ce que** les paramètres suivants sont utilisés pour les mesures optiques du rayonnement Raman rétrodiffusé par le vaisseau (21) traversé par un flux de sang sélectionné :

- longueurs d'onde d'excitation du rayonnement laser (3a) du laser d'excitation (3) : > 600 nm (par exemple laser HeNe 633 nm, laser à corps solide (basé sur Nd, par exemple laser Nd:YAG 1064 nm, laser à diode VIS NIR, par exemple 785 nm)),
- injection du rayonnement laser (3a) directement avec des miroirs et/ou avec des fibres optiques (4a),
- les mesures du rayonnement Raman rétrodiffusé sont réalisées en utilisant un arrangement optique (5) ayant une grande ouverture numérique comme des objectifs de microscope (61) ou une sonde à fibre (4b),
- extraction directe du rayonnement Raman rétrodiffusé (7) collecté au moyen de miroirs vers un spectromètre (8) ou au moyen du transport par des fibres optiques,
- utilisation d'un spectromètre (8) sous la forme d'un spectromètre dispersif et d'un spectromètre à transformée de Fourier.

7. Procédé selon les revendications 2 à 5, **caractérisé en ce que** les paramètres suivants sont utilisés pour les mesures optiques du rayonnement fluorescent rétrodiffusé par le vaisseau (21) traversé par un flux de sang sélectionné :

- longueurs d'onde d'excitation du rayonnement laser (3a) du laser d'excitation (3) : > 400 nm (par exemple laser HeNe 633 nm, laser à corps solide (basé sur Nd, par exemple laser Nd:YAG 532 nm, laser à diode VIS NIR, par exemple 785 nm)),
- injection du rayonnement laser (3a) directement avec des miroirs et/ou avec des fibres optiques (4a),
- les mesures du rayonnement fluorescent sont réalisées en utilisant des arrangements optiques (5) comme des objectifs de microscope (61) ou une sonde à fibre (4b),
- extraction directe du rayonnement fluorescent (7) collecté au moyen d'au moins un miroir ou au moyen du transport par des fibres optiques,
- utilisation d'un détecteur (8).

8. Procédé selon les revendications 1 à 7, **caractérisé en ce que** pendant une opération de positionnement de l'œuf d'oiseau (1) les valeurs mesurées de mesures optiques sont enregistrées en permanence et sont acheminées à une interprétation, une classification automatique du rayonnement rétrodiffusé (7) étant effectuée à l'aide de l'empreinte numérique spectrale du sang.

9. Procédé selon la revendication 1 ou 2, **caractérisé en ce que** l'œuf d'oiseau (1) est posé en reposant sur un pôle.

10. Procédé selon la revendication 9, **caractérisé en ce qu'**un trou (2) est formé dans la zone du pôle pointu (24) dirigé vers le haut, avec une taille de trou maximale de 18 mm au moins après 48 heures d'incubation au moyen de l'unité de création de trou.

11. Procédé selon la revendication 9, **caractérisé en ce qu'**un trou (2) est formé dans la zone du pôle tronqué dirigé vers le haut de l'œuf d'oiseau (1), avec un diamètre maximal de 18 mm, de préférence entre 8 mm et 12 mm, au moyen de l'unité de création de trou.

12. Procédé selon les revendications 1 à 6 et 8 à 11, **caractérisé en ce que** le procédé de mesure optique est mis en œuvre sous la forme d'un procédé de mesure in-ovo à spectroscopie Raman destiné à déterminer le sexe d'œufs d'oiseau (1) fécondés et incubés au moins après 48 heures d'incubation

13. Arrangement (40) de réglage du foyer laser (30) d'un laser d'excitation (3) dans un vaisseau (21) traversé par un flux de sang pour des mesures optiques en vue de déterminer le sexe d'œufs d'oiseau (1), les vaisseaux (21) traversés par un flux de sang ainsi que l'embryon (22) étant mobiles dans l'œuf d'oiseau (1) et n'étant pas encore attachés à la coquille calcaire (28) au moment d'une mesure optique du rayonnement rétrodiffusé (7), le dispositif étant configuré pour mettre en œuvre un procédé selon l'une des revendications 1 à 12, et l'arrangement (40) comprenant au moins

- un dispositif de maintien (16) pour l'œuf d'oiseau (1) doté d'un trou (2), les œufs d'oiseau (1) étant respectivement stockés avec un pôle (24) vers le haut,
- une source de rayonnement (10) pour l'éclairage de la surface se trouvant dans l'œuf d'oiseau (1) avec la couche de blanc d'œuf (26) et avec un réseau de vaisseaux sanguins (27),
- une unité de positionnement x, y, z coordonnée (18) pour un dispositif optique (5) destiné à introduire le rayonnement laser (3a), en vue de détecter un rayonnement rétrodiffusé (7) et des surfaces d'image de position d'analyse $SAF_1$, $SAF_2$,
- le dispositif optique (5) destiné à introduire le rayonnement laser (3a), en vue de détecter un rayonnement rétrodiffusé (7) et des surfaces d'image de position d'analyse $SAF_1$, $SAF_2$ contenant au moins
- au moins un objectif de mesure (61, 62),
- un séparateur de faisceaux (23),
- une caméra (13) comportant au moins un objectif de caméra (38, 63), le dispositif optique (5) servant à modifier la représentation entre une image de la $SAF_1$ (65) et une image de la RBF (41), l'image de la $SAF_1$ (65) concernant la surface d'image totale du réseau de vaisseaux sanguins (27) et l'image de la RBF (41) la surface d'image réduite du vaisseau (21) traversé par un flux de sang sélectionné,
- un dispositif d'interprétation (9) pour les spectres optiques,
- une unité d'interprétation (31), qui comprend au moins
- un câble de liaison (32) vers la caméra (13),
- un câble de liaison (33) vers le dispositif de maintien (16),
- un câble de liaison (34) vers la source de rayonnement (10),
- un câble de liaison (35) vers l'unité de positionnement x, y, z coordonnée (18),
- un câble de liaison (36) vers le dispositif d'interprétation (9) pour les mesures optiques,

l'unité d'interprétation (31) possède des moyens techniques de programme pour mettre en œuvre un procédé basé sur le contraste, lequel se rapporte à une surface d'image de position d'analyse totale $SAF_1$ (65) avec le réseau de vaisseaux sanguins (27), et un procédé d'image différentielle basé sur un mouvement des érythrocytes, lequel se rapporte à la surface d'image réduite RBF (41) de la surface d'image de position d'analyse $SAF_2$ (39) sélectionnée avec un vaisseau (21) traversé par un flux de sang sélectionné du réseau de vaisseaux sanguins (27).

**14.** Dispositif selon la revendication 13, **caractérisé en ce que**
un premier objectif de mesure (61) destiné à mettre en œuvre un procédé d'interprétation basé sur le contraste et un deuxième objectif de mesure (62) destiné à mettre en œuvre un procédé d'image différentielle basé sur un mouvement des érythrocytes,
sont inclus dans l'arrangement optique (5) destiné à introduire le rayonnement laser, en vue de détecter un rayonnement rétrodiffusé (7) et des surfaces d'image de position d'analyse $SAF_1$, $SAF_2$, les objectifs de mesure (61, 62) s'insérant en alternance dans le trajet de rayon (42) de la caméra (13) respectivement en vue de mettre en œuvre les deux procédés.

**15.** Dispositif selon la revendication 14, **caractérisé en ce que**
un objectif de mesure ayant un grossissement variable est introduit dans le trajet de rayon (42) de la caméra (13) à la place des deux objectifs de mesure (61, 62), de sorte qu'aucun deuxième objectif de mesure et aucun déplacement des objectifs ne sont nécessaires.

**16.** Dispositif selon la revendication 13, **caractérisé en ce que**
un premier objectif de caméra (38) destiné à mettre en œuvre un procédé d'interprétation basé sur le contraste et un deuxième objectif de caméra (63) destiné à mettre en œuvre un procédé d'image différentielle basé sur un mouvement des érythrocytes,
sont inclus dans l'arrangement optique (5) destiné à introduire le rayonnement laser (3a), en vue de détecter un rayonnement rétrodiffusé (7) et des surfaces d'image de position d'analyse $SAF_1$, $SAF_2$, les objectifs de caméra (38, 63) s'insérant en alternance dans le trajet de rayon (42) de la caméra (13) respectivement en vue de mettre en œuvre les deux procédés.

**17.** Dispositif selon la revendication 16, **caractérisé en ce que**
un objectif de caméra ayant un grossissement variable est introduit dans le trajet de rayon (42) de la caméra (13) à la place des deux objectifs de caméra (38, 63), de sorte qu'aucun deuxième objectif de caméra et aucun déplacement des objectifs ne sont nécessaires.

**18.** Dispositif selon l'une des revendications 13 à 17, **caractérisé en ce que** l'arrangement optique (5) destiné à introduire le rayonnement laser, en vue de détecter un rayonnement rétrodiffusé (7) et des surfaces d'image de position d'analyse $SAF_1$, $SAF_2$ est en liaison avec l'unité de positionnement x, y, z coordonnée (18), l'unité de positionnement x, y, z coordonnée (18) pouvant déplacer l'arrangement optique (5) de manière commandée dans la direction x, y, z.

**19.** Dispositif selon l'une des revendications 13 à 18, **caractérisé en ce que** le rayonnement rétrodiffusé (7) à mesurer provenant du vaisseau (21) traversé par un flux de sang, est un rayonnement Raman et/ou un rayonnement fluorescent rétrodiffusé.

Fig. 1

Stand der Technik

EP 3 244 197 B1

Fig. 2

Fig. 2a

Fig. 3

Fig. 3c

Fig. 3a

Fig. 3b

Fig. 4

Fig. 5

**Fig. 7**

z Achse

Eiweißschicht

26

43

6

30

21

46

---- Variance

Differenzbild

125

112,5

100

87,5

75

Relativbewegung
der
z-Achse
[µm]

62,5

50

37,5

25

12,5

0

1   0,96  0,92  0,88  0,84   0,8  0,8   0,6   0,4   0,2   0

Normierter Schärfewert

**Fig. 6**

Fig. 8

**Selektion des Gefäßes**

Bildaufnahme

**Vorverarbeitung der Eingangsmatrix**

Bildmatrix

| roter Kanal | grüner Kanal | blauer Kanal |

Maximierung des Kontrasts

Hintergrund entfernen

**Segmentierung der Blutgefäße**

| Morphologisches Schließen | Partikel entfernen | Auto Local Threshold |

Merkmalsextraktion des Gefäßes

Klassifizierung der Blutgefäße

Fig. 9

**Selektion des Gefäßes**

Bildaufnahme

Vorverarbeitung der Eingangsmatrix

Segmentierung der Blutgefäße

Merkmalsextraktion des Gefäßes

Rasterung der Bildmatrix

Klassifizierung der Blutgefäße

Auswahl nach größter Fläche → Ermittlung der Analyseposition

Fig. 10

**Positionierung und Halten des Blutgefäßes**

**Fokussierung auf die Erythrozyten**

Bestimmung der Fokusebene

| Bewegung durch definierten Bereich | Ermittlung des Maximums mittels Schärfeindikator |

Positionierung der Z-Achse in den Fokus

Exakte Positionierung in das Gefäß

| Bewegung durch einen eingeschränkten Bereich | Ermittlung des Maximums mittels Differenzbild |

Positionierung der Z-Achse in das Gefäß

Halten der Position mittels Zweipunktregelung

Ausgleich der Bewegungen in Horizontalebene

Fig. 11

**Positionierung und Halten des Blutgefäßes**

Fokussierung auf die Erythrozyten

Ausgleich der Bewegungen in Horizontalebene

Erstellung eines Templates von der Sollposition

Auffinden des Templates in der aktuellen Bildmatrix

Umrechnung der Position in der Bildmatrix in Absolutbewegungen

Ansteuerung der X- und Y-Achse zur Sollposition

Fig. 12

Fig. 13

Fig. 14

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20140273068 A
- US 7583380 B2
- US 7663748 B2
- US 6681133 B2
- US 8159662 B2
- US 8306780 B2
- WO 2016000678 A1
- DE 102010006161 B3
- DE 102007013107 A1


**In der Beschreibung aufgeführte Nicht-Patentliteratur**

- **MARTINEZ-MARTIN.** del Pobil: Robust Motion Detection in Real-Life Scenarios. Springer-Verlag, 2012
- **LEE, JI, LEE.** Micro-PIV measurements of blood flow in extraembryonic blood vessels of chicken embryos. *Physiol. Meas.,* 2007, vol. 28, 1149-1162
- **KLOOSTERMAN ; HIERCK ; WESTERWEEL ; POELMA.** Quantification of Blood Flow and Topology in Developing Vascular Networks. *PlosONE,* 2014, vol. 9 (5), e96856
- *Applied Optics,* 2010, vol. 49 (13
- **GOERTZ, YU ; KERBEL, BURNS ; FOSTER.** High-frequency 3-D color-flow imaging of the microcirculation. *Ultrasound Med Biol.,* Januar 2003, vol. 29 (1), 39-51
- **DRUCKSCHRIFT AOUDI ; LIEBGOTT ; NEEDLES ; YANG ; FOSTER ; VRAY.** Estimation methods for flow imaging with high frequency ultrasound. *Ultrasonics,* 2006, vol. 44, 135-140
- **DRUCKSCHRIFTEN NAKAMACHI.** Development of Three Dimensional Blood Vessel Search System by Using on Stereo and Autofocus Hybrid Method. *33rd Annual International Conference of the IEEE EMBS,* 2011
- **NAKAMACHI ; MORITA, MIZUNO.** Development of Automatic 3D Blood Vessel Search and Automatic Blood Sampling System by Using Hybrid Stereo-Autofocus Method. *International Journal of Optics,* vol. 2012
- **SAUVOLA J. ; PIETIKANEN.** Adaptive document image binarization. Pergamon, 2000, vol. 33